# EUROPEAN PATENT APPLICATION

(11) **EP 4 821 942 A1**
(43) Date of publication of application: **14.10.2026**
(21) Application number: 24899929.4
(22) Date of filing: 05.12.2024
(51) Int. Cl.: C07K 14/32, C12N 9/22, C12N 9/78, C12N 15/00, C12N 5/00

(54) **DEAMINASE, FUSION PROTEIN, NUCLEIC ACID, PHARMACEUTICAL COMPOSITION AND USE THEREOF**

(30) Priority: 05.12.2023 CN 202311657105; 03.04.2024 CN 202410403512; 04.07.2024 CN 202410893197
(71) Applicant: Guangzhou Reforgene Medicine Co., Ltd., Guangzhou, Guangdong 510535 (CN)
(72) Inventor: LIANG, Junbin, Guangzhou, Guangdong 510535 (CN); HUANG, Liancheng, Guangzhou, Guangdong 510535 (CN); LIANG, Xingxiang, Guangzhou, Guangdong 510535 (CN); XU, Hui, Guangzhou, Guangdong 510535 (CN); YANG, Zhengxia, Guangzhou, Guangdong 510535 (CN); OU, Zheyuan, Guangzhou, Guangdong 510535 (CN); CAI, Jinxiu, Guangzhou, Guangdong 510535 (CN); HUANGFU, Desheng, Guangzhou, Guangdong 510535 (CN)
(74) Representative: Dai, Simin
(86) International application number: PCT/CN2024/137235
(87) International publication number: WO 2025/119295

(57) **Abstract**

A deaminase, a fusion protein and the use thereof. The deaminase contains an amino acid sequence having at least 50% sequence identity to any one of SEQ ID NOs: 1-6, 39-164 and 297-312, and the deaminase can deaminize at least one nucleotide in a target polynucleotide. The deaminase can be fused with a nucleic acid-binding polypeptide to obtain various base editors for the sequence-specific base conversion of the target polynucleotide. The performance of the editors can be further improved by means of fusing one or two UGls, fusing a Gam protein and other strategies.

## Description

### TECHNICAL FIELD

The present disclosure generally relates to the field of gene editing, and in particular to deaminase, fusion protein, nucleic acid, pharmaceutical composition, and use thereof.

### BACKGROUND

A CRISPR-Cas system is an adaptive immune defense formed by bacteria and archaea during long-term evolution and can be used to combat invading viruses and exogenous DNA. In 2016, David Liu's laboratory developed cytosine base editors (CBEs). Currently, discovering more suitable cytosine deaminases for constructing base editors and expanding a gene editing toolbox have become important research directions in gene editing.

### SUMMARY

The present disclosure provides a deaminase, a fusion protein, a nucleic acid, a pharmaceutical composition, and use thereof. The deaminase provided in the present disclosure has high deamination editing efficiency and a wide editing window, and the constructed fusion protein also has high editing efficiency and a wide editing window. Some deaminase fusion proteins provided in the present disclosure have editing window positions different from those of existing fusion proteins.

One aspect of the present disclosure provides a deaminase, comprising an amino acid sequence having at least 50% sequence identity to a sequence set forth in any one of SEQ ID NO: 1-6, 39-164, and 297-312.

In some embodiments of the present disclosure, the deaminase is capable of deaminating at least one nucleotide in a polynucleotide.

In some embodiments of the present disclosure, the deaminase is capable of deaminating at least one nucleotide in a target polynucleotide.

In some embodiments of the present disclosure, the deaminase deaminates the at least one nucleotide in the polynucleotide.

In some embodiments of the present disclosure, the deaminase deaminates the at least one nucleotide in the target polynucleotide.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in any one of SEQ ID NO: 1-6, 39-164, and 297-312.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in any one of SEQ ID NO: 1, 3-6, 43, 46, 48, 51, 63, 114, 126, 160, 163, 164, 297, 299, and 301.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in any one of SEQ ID NO: 3, 5, 6, 126, 160, 163, 164, 297, 299, and 301.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 3.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 5.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 6.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 126.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 160.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 163.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 164.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 297.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 299.

In some embodiments of the present disclosure, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in SEQ ID NO: 301.

In some embodiments of the present disclosure, the deaminase comprises a mutation at a position corresponding to amino acid residue 122, 114, 176, 2, and/or 68 of a reference sequence set forth in SEQ ID NO: 301.

In some embodiments of the present disclosure, the deaminase comprises a mutation at a position corresponding to any one, two, three, four, or five amino acid residues selected from position 122, position 114, position 176, position 2, and position 68 of a reference sequence set forth in SEQ ID NO: 301.

In some embodiments of the present disclosure, the deaminase comprises a mutation at a position corresponding to any one, two, three, four, or five amino acid residues selected from position 122, position 191, position 114, position 2, and position 89 of the reference sequence set forth in SEQ ID NO: 301.

Non-limitingly, the mutation may be a substitution, insertion, and/or deletion of an amino acid residue; further, the mutation may be a substitution of an amino acid residue. Non-limitingly, the corresponding position may be identified by performing sequence alignment of the amino acid sequence of the deaminase with the reference sequence set forth in SEQ ID NO: 301. In some embodiments of the present disclosure, the deaminase comprises a mutation at a position corresponding to amino acid residue 122 of the reference sequence set forth in SEQ ID NO: 301. In some embodiments of the present disclosure, the deaminase comprises a mutation at a position corresponding to amino acid residue 191 of the reference sequence set forth in SEQ ID NO: 301. In some embodiments of the present disclosure, the deaminase comprises a mutation at a position corresponding to amino acid residue 114 of the reference sequence set forth in SEQ ID NO: 301. In some embodiments of the present disclosure, the deaminase comprises a mutation at a position corresponding to amino acid residue 2 of the reference sequence set forth in SEQ ID NO: 301. In some embodiments of the present disclosure, the deaminase comprises a mutation at a position corresponding to amino acid residue 176 of the reference sequence set forth in SEQ ID NO: 301. In some embodiments of the present disclosure, the deaminase comprises a mutation at a position corresponding to amino acid residue 68 of the reference sequence set forth in SEQ ID NO: 301. In some embodiments of the present disclosure, the deaminase comprises a mutation at a position corresponding to amino acid residue 89 of the reference sequence set forth in SEQ ID NO: 301.

In some embodiments of the present disclosure, the deaminase has a mutation to H at the position corresponding to the amino acid residue 122 of the reference sequence set forth in SEQ ID NO: 301. In some embodiments of the present disclosure, the deaminase has a mutation to R at the position corresponding to the amino acid residue 191 of the reference sequence set forth in SEQ ID NO: 301. In some embodiments of the present disclosure, the deaminase has a mutation to T at the position corresponding to the amino acid residue 114 of the reference sequence set forth in SEQ ID NO: 301. In some embodiments of the present disclosure, the deaminase has a mutation to M at the position corresponding to the amino acid residue 2 of the reference sequence set forth in SEQ ID NO: 301. In some embodiments of the present disclosure, the deaminase has a mutation to D at the position corresponding to the amino acid residue 89 of the reference sequence set forth in SEQ ID NO: 301. In some embodiments of the present disclosure, the deaminase has a mutation to F at the position corresponding to the amino acid residue 176 of the reference sequence set forth in SEQ ID NO: 301. In some embodiments of the present disclosure, the deaminase has a mutation to I at the position corresponding to the amino acid residue 68 of the reference sequence set forth in SEQ ID NO: 301. In some embodiments of the present disclosure, the deaminase has a mutation to I at the position corresponding to the amino acid residue 68 and a mutation to T at the position corresponding to the amino acid residue 114 of the reference sequence set forth in SEQ ID NO: 301.

In some embodiments of the present disclosure, the deaminase comprises an amino acid sequence having 100% sequence identity to the sequence set forth in any one of SEQ ID NO: 1-6, 39-164, and 297-312.

In some embodiments of the present disclosure, the deaminase comprises an amino acid sequence having 100% sequence identity to the sequence set forth in any one of SEQ ID NO: 1, 3-6, 43, 46, 48, 51, 63, 114, 126, 160, 163, 164, 297, 299, and 301.

In some embodiments of the present disclosure, the deaminase comprises an amino acid sequence set forth in any one of SEQ ID NO: 1-6, 39-164, and 297-312.

In some embodiments of the present disclosure, the deaminase comprises an amino acid sequence set forth in any one of SEQ ID NO: 1, 3-6, 43, 46, 48, 51, 63, 114, 126, 160, 163, 164, 297, 299, and 301.

In some embodiments of the present disclosure, the amino acid sequence of the deaminase is set forth in any one of SEQ ID NO: 1-6, 39-164, and 297-312.

In some embodiments of the present disclosure, the amino acid sequence of the deaminase is set forth in any one of SEQ ID NO: 1, 3-6, 43, 46, 48, 51, 63, 114, 126, 160, 163, 164, 297, 299, and 301.

In some embodiments of the present disclosure, the amino acid sequence of the deaminase is not any one of SEQ ID NO: 1-5 and 54-60.

In some embodiments of the present disclosure, the deaminase is a Q122H, S191R, I114T, L2M, or N89D mutant of deaminase-145 (the amino acid sequence set forth in SEQ ID NO: 301).

In some embodiments of the present disclosure, the deaminase may deaminate a base in a nucleic acid sequence together with a nucleic acid-binding polypeptide. For example, the deaminase may deaminate a base in a DNA sequence together with a TALEN or a ZFN.

In some embodiments of the present disclosure, the deaminase may form a complex with the nucleic acid-binding polypeptide to deaminate the base in the nucleic acid sequence.

In some embodiments of the present disclosure, the deaminase is capable of deaminating a base in a target polynucleotide together with a nucleic acid-binding polypeptide and a guide RNA (gRNA).

In some embodiments of the present disclosure, the deaminase is capable of forming a complex together with the nucleic acid-binding polypeptide and the guide RNA (gRNA), the guide RNA guides the complex to bind to the target polynucleotide and deaminate the base in the target polynucleotide sequence.

The deaminase and the nucleic acid-binding polypeptide in the complex may be two separate molecules, or may be a single molecule formed by linking the deaminase and the nucleic acid-binding polypeptide (for example, forming a fusion protein).

A non-limiting example is: the deaminase may be used to replace a deaminase domain in a tBE base editing system as described in patent application WO2022206986A1 or WO2023155901A1, thereby obtaining a new base editing system.

In some embodiments, the deaminase comprises a homologous or heterologous domain. In some embodiments, the deaminase comprises a nucleic acid-binding polypeptide. In some embodiments, the deaminase comprises a cellular localization signal; further, the deaminase comprises a nuclear localization signal, a nuclear export signal, a mitochondrial localization signal, or a chloroplast localization signal. In some embodiments, the deaminase comprises a uracil glycosylase inhibitor (UGI) domain.

One aspect of the present disclosure provides a fusion protein, wherein the fusion protein comprises the deaminase as described in the present disclosure.

In some embodiments of the present disclosure, the fusion protein comprises: a) a nucleic acid-binding polypeptide that binds to a target polynucleotide; and b) the deaminase as described in the present disclosure.

The fusion protein may be used for targeted editing of a nucleic acid (e.g., DNA nucleic acid, RNA nucleic acid) in vitro, ex vivo, or in vivo, for example, for generating mutant cells. These mutant cells can be in a plant or an animal. The fusion protein may also be used to introduce targeted mutations, for example, for correction of genetic defects in vitro in mammalian cells (e.g., in cells obtained from a subject, the cells subsequently being reintroduced into the same or another subject); and to introduce targeted mutations, for example, in disease-associated genes in a mammalian subject to correct genetic defects or introduce mutations. The fusion protein may also be used to introduce targeted mutations in plant cells, for example, for introducing beneficial or agronomically valuable traits or alleles. The targeted editing is, for example, deaminating at least one nucleotide in a nucleic acid.

Optionally, the nucleic acid-binding polypeptide is a DNA binding polypeptide.

Optionally, the nucleic acid-binding polypeptide is an RNA binding polypeptide.

In some embodiments of the present disclosure, the nucleic acid-binding polypeptide is a TALEN nuclease, a zinc finger nuclease (ZFN), a CRISPR-Cas nuclease, or a meganuclease.

In some embodiments of the present disclosure, the nucleic acid-binding polypeptide is an RNA-guided nuclease. Optionally, the DNA binding polypeptide is a CRISPR-associated nuclease, i.e., a Cas enzyme (also referred to as a Cas protein, CRISPR-Cas nuclease).

In some embodiments of the present disclosure, the nucleic acid-binding polypeptide is selected from Cas1, Cas1B, Cas2, Cas3, Cas4, Cas5, Cas5d, Cas5t, Cas5h, Cas5a, Cas6, Cas7, Cas8, Cas8a, Cas8b, Cas8c, Cas9, Cas10, Cas10d, Cas12a/Cpf1, Cas12b/C2c1, Cas12c/C2c3, Cas12d/CasY, Cas12e/CasX, Cas12f/CasZ, Cas12g, Cas12h, Cas12i, Csy1, Csy2, Csy3, Csy4, Cse1, Cse2, Cse3, Cse4, Cse5e, Csc1, Csc2, Csa5, Csn1, Csn2, Csm1, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx1S, Csx11, Csf1, Csf2, CsO, Csf4, Csd1, Csd2, Cst1, Cst2, Csh1, Csh2, Csa1, Csa2, Csa3, Csa4, Csa5, Cas13a, Cas13b, Cas13c, Cas13d, Cas13e, Cas13f, TnpB, IscB, IsrB, and Fancor; or a fragment of any one of them. A non-limiting example of the fragment is, for example, a nucleic acid binding domain fragment.

In some embodiments of the present disclosure, the nucleic acid-binding polypeptide is selected from Cas9, Cas12, Cas13, TnpB, IscB, IsrB, and a Fancor nuclease; or a fragment of any one of them, the fragment including but not limited to the nucleic acid binding domain fragment.

In some embodiments of the present disclosure, the Cas9 is selected from SpCas9, SaCas9, Nme2Cas9, Nme3Cas9, CjCas9, NmCas9, FnCas9, PpnCas9, FrCas9, SauCas9, SauriCas9, ScaCas9, St1Cas9, BlatCas9, CdiCas9, GeoCas9, or a fragment of any one of them, and a mutant of any one of them or a fragment of the mutant.

In some embodiments of the present disclosure, the nucleic acid-binding polypeptide is selected from AsCpf1, enAsCas12a (addgene plasmid #196724), dFnCas12a (addgene plasmid #136379), ErCas12a, LbCas12a D832A, LbCas12a H759A, LbCas12a E795L, FnCas12a3, FnCas12a D917A, AsCas12a R1226A, AsCas12a D908A, AsCas12a E174R/S542R, AsCas12a (S542R/K548V/N552R), PrCas12a, PxCas12a, PcCas12a, PdCas12a, Mb2Cas12a, Mb3Cas12a, MlCas12a, CMaCas12a, CMtCas12a, HkCas12a, Lb5Cas12a, ErCas12a, TsCas12a, FnCpf1, LbCas12a, dLbCpf1, ttHsCas12a, AaCas12b, AaCas12b D570A, AaCas12b Q119F/E475R/E758R, BhCas12b, BvCas12b, BrCas12b, AkCas12b, AmCas12b, BsCas12b, OspCas12c, Cas12c2 (addgene plasmid #183072), Cas12c_4 (addgene plasmid #183071), Cas12c1 (addgene plasmid #120872), CasY.1 (from Katanobacteria), CasY.2 (from Vogelbacteria), CasY.3 (from Vogelbacteria), CasY.4 (from Parcubacteria), CasY.5 (from Komeilibacteria), CasY.6 (from Kerfeldbacteria), PlmCasX, DpbCasX, Un1Cas12f, CnCas12f1, enRhCas12f1, AsCas12f1, SpaCas12f1, Cas12g1 (addgene plasmid #120879), Cas12h of WO2021113522A1 (SEQ ID NO: 1 of that patent application), Cas12i1 (addgene plasmid #171670), Cas12i2 (addgene plasmid #188275), Cas12i1 (addgene plasmid #120882), Cas12i2 (addgene plasmid #120883), Cas12i proteins designated Cas12f.4/Cas12f.5/Cas12f.6 in CN111757889B, dSiCas12i(D1049A), SiCas12i, Si2Cas12i, WiCas12i, Wi2Cas12i, Wi3Cas12i, SaCas12i, Sa2Cas12i, Sa3Cas12i, WaCas12i, Wa2Cas12i, xCas12i, hfCas12Max, Cas12i-Max (addgene plasmid #188276), Cas12i1 D647A (addgene plasmid #171671), Cas12i-HiFi (addgene plasmid #188269), Cas12i1 D647A, Cas12j3 (addgene plasmid #188497), Cas12j2 (addgene plasmid #188498), AsCas12j-2 (addgene plasmid #191655), Cas12j-8 (addgene plasmid #194966), ShCas12k, N7Cas12k, AcCas12k, Cas12k-TniQ (addgene plasmid #181787), Cas12k-TnsC (addgene plasmid #181789), Cas12l, MmCas12m, MmCas12m ΔZF (H549A, C552A), dCas12m-ΔZF (D485A, H549A, C552A), AcCas12n, dAcCas12n(D240), TnpB Actinomadura_cellulosilytica_strain_DSM_45823, TnpB Actinomadura_namibiensis_strain_DSM_44197, TnpB Actinomadura_umbrina_strain_DSM_43927_$, TnpB Actinoplanes_lobatus_strain_DSM_43150 (TnpB-1 and TnpB-2), TnpB Alicyclobacillus_macrosporagiidus_strain_DSM_17980, TnpB Haloactinospora_alba_Strain_DSM_45015, TnpB Lipingzhangella_halophila_strain_DSM_102030, TnpB Meiothermus_Silvanus_DSM_9946, TnpB QNFX01000004, ISDra2 TnpB(PDB: 8H1J), KraIscB-1, AwaIscB, OgeuIscB, GtFz1 (from Guillardia theta), SpuFz1 (from Spizellomyces punctatus), NlovFz2 (from Percolozoa Naegleria lovaniensis), MmeFz2 (from Mercenaria mercenaria), a fragment of any one of them, and a mutant of any one of them or a fragment of the mutant.

In some embodiments of the present disclosure, the DNA binding polypeptide is capable of cleaving one strand of a double-stranded nucleic acid molecule (e.g., a double-stranded DNA molecule). Optionally, the DNA binding polypeptide is an RNA-guided nuclease having nickase activity.

In some embodiments of the present disclosure, the Cas enzyme cleaves a target strand of a double-stranded nucleic acid molecule, meaning that the Cas enzyme cleaves a strand that base-pairs with (is complementary to) a gRNA (e.g., an sgRNA) bound to the Cas enzyme.

In some embodiments of the present disclosure, the nucleic acid-binding polypeptide is an RNA-guided nuclease with inactivated nuclease activity.

In some embodiments of the present disclosure, the nucleic acid-binding polypeptide is a variant of an RNA-guided nuclease with completely inactivated nuclease activity compared to a wild-type form of the RNA-guided nuclease, such as a dCas enzyme, including but not limited to Cas9, Cas12, Cas13, TnpB, IscB, IsrB, and Fancor each of which has completely inactivated nuclease activity (which may be referred to as dead Cas9, dead Cas12, dead Cas13, dead TnpB, dead IscB, dead IsrB, and dead Fancor); or a fragment of any one of them or a mutant of any one of them, such as a SpRY Cas9 mutant.

In some embodiments of the present disclosure, the nucleic acid-binding polypeptide is a variant of an RNA-guided nuclease with partially inactivated nuclease activity compared to a wild-type form of the RNA-guided nuclease, such as an nCas enzyme, for example, a polypeptide fragment that retains the nuclease activity of cleaving a single strand of double-stranded DNA, including but not limited to nickase Cas9 and nickase Cas12.

In some embodiments of the present disclosure, the fusion protein further comprises one, two, three, four, or more Uracil Glycosylase Inhibitors ( UGIs ). In some embodiments of the present disclosure, the fusion protein comprises one UGI. In some embodiments of the present disclosure, the fusion protein comprises two UGIs. In some embodiments of the present disclosure, the fusion protein comprises three UGIs. The UGI may inhibit human UDG activity.

In some embodiments of the present disclosure, the UGI comprises an amino acid sequence having at least 60%, at least 65%, at least 70%, at least 75%, at least 80%, at least 85%, at least 90%, at least 95%, at least 96%, at least 97%, at least 98%, at least 99%, or at least 99.5% sequence identity to an amino acid sequence set forth in SEQ ID NO: 38. In some embodiments, the UGI comprises a fragment or a homolog of the sequence set forth in SEQ ID NO: 38.

In a preferred embodiment of the present disclosure, the fusion protein comprises one or more nuclear export signals (NESs), for example, one, two, three, four, or more NESs.

In a preferred embodiment of the present disclosure, the fusion protein comprises one or more nuclear localization signals (NLSs), for example, one, two, three, four, or more NLSs.

Nuclear localization signals are known in the art and generally comprise a stretch of basic amino acids (see, e.g., Lange et al., J. Biol. Chem. (2007) 282:5101-5105). In specific embodiments, the fusion protein comprises two, three, four, five, six, or more nuclear localization signals. The one or more nuclear localization signals may be heterologous NLSs. Non-limiting examples of nuclear localization signals of RNA-guided nucleases useful for the present disclosure are the nuclear localization signals of SV40 large T-antigen, nucleoplasmin, and c-Myc (see, e.g., Ray et al., (2015) Bioconjug Chem 26(6):1004-7). The fusion protein may comprise one or more NLS sequences at its N-terminus, C-terminus, or both N-terminus and C-terminus. For example, the fusion protein may comprise two NLS sequences in the N-terminal region and four NLS sequences in the C-terminal region.

Optionally, the NLS comprises an amino acid sequence set forth in SPKKKRKVEAS (SEQ ID NO: 14), GPKKKRKVAAA (SEQ ID NO: 15), PKKKRKV (SEQ ID NO: 16), KRPAATKKAGQAKKKK (SEQ ID NO: 17), PAAKRVKLD (SEQ ID NO: 18), RQRRNELKRSP (SEQ ID NO: 19), NQSSNFGPMKGGNFGGRSSGPYGGGGQYFAKPRNQGGY (SEQ ID NO: 20), RMRIZFKNKGKDTAELRRRRVEVSVELRKAKKDEQILKRRNV (SEQ ID NO: 21), VSRKRPRP (SEQ ID NO: 22), PPKKARED (SEQ ID NO: 23), POPKKKPL (SEQ ID NO: 24), SALIKKKKKMAP (SEQ ID NO: 25), DRLRR (SEQ ID NO: 26), PKQKKRK (SEQ ID NO: 27), RKLKKKIKKL (SEQ ID NO: 28), REKKKFLKRR (SEQ ID NO: 29), KRKGDEVDGVDEVAKKKSKK (SEQ ID NO: 30), RKCLQAGMNLEARKTKK (SEQ ID NO: 31), and PAAKKKKLD (SEQ ID NO: 32).

In some embodiments of the present disclosure, the nucleic acid-binding polypeptide, the deaminase, the one or more UGIs, and the one or more NLSs are linked directly or via linkers.

In some embodiments of the present disclosure, when the nucleic acid-binding polypeptide, the deaminase, the one or more UGIs, and the one or more NLSs are linked via the linkers, these linkers may be the same or different.

In some embodiments of the present disclosure, the linkers comprise a sequence set forth in any one of SEQ ID NO: 33-37 and EFE, or a fragment of any one of them or any one variant of any one of them.

In some embodiments of the present disclosure, the linkers comprise amino acids set forth in SGGSSGGSSGSETPGTSESATPESSGGSSGGS (SEQ ID NO: 33) and/or SGGSGGSGGS (SEQ ID NO: 34). In some embodiments of the present disclosure, the deaminase and the nucleic acid-binding polypeptide are linked via SEQ ID NO: 33; and/or the nucleic acid-binding polypeptide and the UGI are linked via SEQ ID NO: 34.

In a preferred embodiment of the present disclosure, the fusion protein comprises the following structures sequentially from N-terminus to C-terminus: a deaminase and a nucleic acid-binding polypeptide. Preferably, the fusion protein comprises the following structures sequentially from N-terminus to C-terminus: a deaminase, a linker 1, and a nucleic acid-binding polypeptide.

More preferably, the fusion protein comprises the following structures sequentially from N-terminus to C-terminus: a deaminase, a linker 1, a nucleic acid-binding polypeptide, a linker 2, and a UGI.

Further preferably, the fusion protein comprises the following structures sequentially from N-terminus to C-terminus: a deaminase, a linker 1, a nucleic acid-binding polypeptide, a linker 2, a UGI, and an NLS.

Still further preferably, the fusion protein comprises the following structures sequentially from N-terminus to C-terminus: an NLS, a deaminase, a linker 1, a nucleic acid-binding polypeptide, a linker 2, a UGI, and an NLS.

In a specific embodiment of the present disclosure, the fusion protein comprises the following structures sequentially from N-terminus to C-terminus: an SV 40 NLS, a deaminase, a linker 1, a nucleic acid-binding polypeptide, a linker 2, a UGI, and an SV 40 NLS.

The numbers in the linker 1 and the linker 2 are only used to distinguish nouns of the same type, and do not indicate any actual meaning.

In some embodiments of the present disclosure, the linker 1 and/or the linker 2 are/is optionally selected from sequences set forth in SEQ ID NO: 33-37 and EFE.

In some embodiments of the present disclosure, the linker 1 and/or the linker 2 are/is SEQ ID NO: 33.

In some embodiments of the present disclosure, the linker 1 and/or the linker 2 are/is SEQ ID NO: 34.

In the present disclosure, the SV 40 refers to Simian vacuolating virus 40.

In some embodiments of the present disclosure, a structure of the fusion protein is deaminase-XTEN linker-dead Cas.

In some embodiments of the present disclosure, the structure of the fusion protein is deaminase-XTEN linker-dead Cas-UGI.

In some embodiments of the present disclosure, the structure of the fusion protein is deaminase-XTEN linker-nickase Cas-UGI.

In some embodiments of the present disclosure, the fusion protein is obtained by replacing a deaminase in YE1-BE3, YE2-BE3, EE-BE3, YEE-BE3, or HF-CBE3 in the prior art with the deaminase of the present disclosure.

In some embodiments of the present disclosure, the structure of the fusion protein is deaminase-XTEN linker-dead Cas-UGI-UGI.

In some embodiments of the present disclosure, the structure of the fusion protein is deaminase-XTEN linker-nickase Cas-UGI-UGI.

In some embodiments of the present disclosure, the structure of the fusion protein is Gamdeaminase-XTEN linker-nickase Cas9-UGI-UGI. The Gam is a Gam protein from bacteriophage Mu or an analog or homolog thereof.

In some embodiments of the present disclosure, the fusion protein is capable of forming a complex with a guide RNA (gRNA), and the gRNA guides the complex to bind to a target polynucleotide and deaminates a base in a target polynucleotide.

Another aspect of the present disclosure provides an isolated nucleic acid encoding the deaminase as described in the present disclosure or the fusion protein as described in the present disclosure.

In some embodiments of the present disclosure, the nucleic acid is codon-optimized, for example, prokaryotic codon-optimized for a prokaryotic expression system, or eukaryotic codon-optimized for a eukaryotic expression system.

In some embodiments of the present disclosure, the isolated nucleic acid comprises a sequence set forth in any one of SEQ ID NO: 165-296 and 313-328. In some embodiments of the present disclosure, the sequence of the isolated nucleic acid is set forth in any one of SEQ ID NO: 165-296 and 313-328.

Another aspect of the present disclosure provides an expression vector, wherein the expression vector comprises the nucleic acid described in the present disclosure.

A person skilled in the art may select an appropriate expression vector, for example, the expression vector may include, but is not limited to, a viral expression vector, a bacterial expression vector, a fungal expression vector, an animal expression vector (e.g., insect, drosophila, nematode, fish, zebrafish, mammal, mouse, rat, rabbit, pig, monkey, human, etc.), a plant expression vector, and the like.

In some embodiments of the present disclosure, the expression vector comprises a regulatory sequence. The regulatory sequence is used to regulate the expression of the deaminase or the fusion protein.

In some embodiments of the present disclosure, the regulatory sequence is any one or more selected from: a promoter, an enhancer, an internal ribosome entry site, and a transcription termination signal; the promoter is, for example, a constitutive promoter, an inducible promoter, a broad-spectrum promoter, or a tissue-specific promoter, and/or the transcription termination signal is, for example, a polyadenylation signal or a poly-U sequence.

In some embodiments, the expression vector comprises a pol III promoter (e.g., U6 and H1 promoters), a pol II promoter (e.g., retroviral Rous sarcoma virus (RSV), a LTR promoter (optionally with an RSV enhancer), a cytomegalovirus (CMV) promoter (optionally with a CMV enhancer), an SV40 promoter, a dihydrofolate reductase promoter, a β-actin promoter, a phosphoglycerate kinase (PGK) promoter, or an EF1α promoter.

In some embodiments, the promoter is a constitutive promoter, which is continuously active and not regulated by external signals or molecules. Suitable constitutive promoters include, but are not limited to, CMV, RSV, SV40, EF1α, CAG, and β-actin promoters. In some embodiments, the promoter is an inducible promoter regulated by an external signal or molecule (e.g., a transcription factor).

In some embodiments, the promoter is a tissue-specific promoter, which may be used to drive tissue-specific expression of the deaminase or the fusion protein. Suitable muscle-specific promoters include, but are not limited to, CK8, MHCK7, a myoglobin promoter (Mb), a desmin promoter, a muscle creatine kinase promoter (MCK) and variants thereof, and a SPc5-12 synthetic promoter. Suitable immune cell-specific promoters include, but are not limited to, a B29 promoter (B cells), a CD14 promoter (monocytes), a CD43 promoter (leukocytes and platelets), CD68 (macrophages), and an SV40/CD43 promoter (leukocytes and platelets). Suitable blood cell-specific promoters include, but are not limited to, a CD43 promoter (leukocytes and platelets), a CD45 promoter (hematopoietic cells), INF-β (hematopoietic cells), a WASP promoter (hematopoietic cells), an SV40/CD43 promoter (leukocytes and platelets), and an SV40/CD45 promoter (hematopoietic cells). Suitable pancreas-specific promoters include, but are not limited to, an elastase-1 promoter. Suitable endothelial cell-specific promoters include, but are not limited to, a Fit-1 promoter and an ICAM-2 promoter. Suitable neuronal tissue/cell-specific promoters include, but are not limited to, a GFAP promoter (astrocytes), a SYN1 promoter (neurons), and NSE/RU5' (mature neurons). Suitable kidney-specific promoters include, but are not limited to, an NphsI promoter (podocytes). Suitable bone-specific promoters include, but are not limited to, an OG-2 promoter (osteoblasts, odontoblasts). Suitable lung-specific promoters include, but are not limited to, an SP-B promoter (lung). Suitable liver-specific promoters include, but are not limited to, an SV40/Alb promoter. Suitable heart-specific promoters include, but are not limited to, α-MHC.

Another aspect of the present disclosure provides a pharmaceutical composition, wherein the pharmaceutical composition comprises the deaminase described in the present disclosure, the fusion protein described in the present disclosure, the nucleic acid described in the present disclosure, or the expression vector described in the present disclosure, and a pharmaceutically acceptable delivery carrier.

In some embodiments of the present disclosure, the delivery carrier is selected from: a viral vector, virus-like particles (VLPs), nanoparticles such as lipid nanoparticles (LNPs), liposomes, extracellular vesicles (EVs) such as exosomes and microvesicles, and a gene gun.

In some embodiments of the present disclosure, the viral vector is an adeno-associated virus, an adenovirus, a lentivirus, or a herpes virus.

In some embodiments of the present disclosure, the adeno-associated virus is a recombinant adeno-associated virus vector of serotype AAV1, AAV2, AAV3, AAV4, AAV5, AAV6, AAV7, AAV8, AAV9, AAV10, AAV11, AAV12, AAV13, AAV PHP.B, AAV PHP.B2, AAV PHP.B3, AAV PHP.A, AAV PHP.eB, AAV PHP.eS, AAV2.7m8, AAV8.7m8, AAV ShH10, AAVrh10, or AAVrh74.

In some embodiments of the present disclosure, the deaminase, the fusion protein, the nucleic acid, or the expression vector described in the present disclosure is packaged in an AAV vector, for example, packaged into a capsid of the AAV1, AAV2, AAV3, AAV4, AAV5, AAV6, AAV7, AAV8, AAV9, AAV10, AAV11, AAV12, AAV13, AAV PHP.B, AAV PHP.B2, AAV PHP.B3, AAV PHP.A, AAV PHP.eB, AAV PHP.eS, AAV2.7m8, AAV8.7m8, AAV ShH10, AAVrh10, or AAVrh74.

In some embodiments of the present disclosure, the lentiviral vector is pseudotyped with an envelope protein. Preferably, the nucleic acid is linked to an aptamer sequence.

In some embodiments of the present disclosure, in the VLPs, the nucleic acid is linked to a gene encoding a gag protein.

Another aspect of the present disclosure provides an expression system, wherein the expression system comprises the expression vector described in the present disclosure, the pharmaceutical composition described in the present disclosure, or the nucleic acid described in the present disclosure exogenously integrated in a genome. The expression system may be a host cell, which may express the fusion protein described in the present disclosure, and the fusion protein may cooperate with a gRNA, so that the fusion protein may be localized to a target polynucleotide to achieve base editing of the target polynucleotide.

In some embodiments of the present disclosure, the host cell may be a eukaryotic cell and/or a prokaryotic cell. The eukaryotic cell includes a fungus, a plant cell, an animal cell, for example, a mammalian cell, such as a mouse cell and a human cell, etc., for example, a mouse brain neuroblastoma cell, a human embryonic kidney cell, a human cervical cancer cell, a human colon cancer cell, or a human osteosarcoma cell, etc., more specifically, an N2a cell, a HEK293FT cell, a Hela cell, an HCT116 cell, or a U2OS cell, etc.

Another aspect of the present disclosure provides a system for modifying a target polynucleotide, the system comprising: a) one or more guide RNAs (gRNAs) or one or more nucleic acids encoding the one or more gRNAs, the one or more gRNAs being capable of hybridizing with the target polynucleotide.

The system further comprises any one or more of the following (a)-(f): b) the deaminase described in the present disclosure, or a nucleic acid encoding the deaminase; and a nucleic acid-binding polypeptide, or a nucleic acid encoding the nucleic acid-binding polypeptide; c) the fusion protein described in the present disclosure, or a nucleic acid encoding the fusion protein; d) the expression vector described in the present disclosure; e) the pharmaceutical composition described in the present disclosure; f) the expression system described in the present disclosure.

The target polynucleotide may also be referred to as a target nucleic acid.

In some embodiments of the present disclosure, the deaminase in b) and the nucleic acid-binding polypeptide form a complex to deaminate a base in a nucleic acid sequence.

In some embodiments of the present disclosure, b) together with a) forms a complex to deaminate a base in the target polynucleotide.

In some embodiments of the present disclosure, b), c), d), e), and/or f) are guided by a) to the target polynucleotide to deaminate the base in the target polynucleotide.

Another aspect of the present disclosure provides a method for modifying a target polynucleotide, the method comprising contacting the target polynucleotide with the deaminase described in the present disclosure, the fusion protein described in the present disclosure, the nucleic acid described in the present disclosure, the expression vector described in the present disclosure, the pharmaceutical composition described in the present disclosure, the expression system described in the present disclosure, or the system for modifying a target polynucleotide described in the present disclosure.

In some embodiments of the present disclosure, the expression vector or expression system comprises a nucleotide sequence encoding the deaminase or the fusion protein.

In some embodiments of the present disclosure, the modification is that at least one nucleotide in the target polynucleotide is deaminated.

Optionally, the method is for non-therapeutic or non-diagnostic purposes.

Based on common knowledge in the art, the above preferred conditions may be arbitrarily combined to obtain various preferred examples of the present disclosure.

The reagents and raw materials used in the present disclosure are all commercially available.

The present disclosure has the following advantageous effects: (1) the fusion proteins of some deaminases provided by the present disclosure have relatively high editing efficiency; (2) the fusion proteins obtained from some deaminases provided by the present disclosure have a relatively wide editing window; (3) the fusion proteins obtained from some deaminases of the present disclosure have a narrow editing window.

### BRIEF DESCRIPTION OF THE DRAWINGS

FIG. 1 is a plasmid map of CBE-27-pCDNA3.1.
FIG. 2 is a schematic diagram illustrating a Sanger analysis result for CBE-27 single base editing in Example 2.
FIG. 3 is a schematic diagram illustrating a Sanger analysis result for CBE-29 single base editing in Example 2.
FIG. 4 is a schematic diagram illustrating a Sanger analysis result for NC group single base editing in Example 2.
FIG. 5 is a schematic diagram illustrating a Sanger analysis result for CBE-136 single base editing in Example 3.
FIG. 6 is a schematic diagram illustrating a Sanger analysis result for CBE-140 single base editing in Example 3.
FIG. 7 is a schematic diagram illustrating a Sanger analysis result for CBE-139 single base editing in Example 4.
FIG. 8 is a schematic diagram illustrating a Sanger analysis result for CBE-145 single base editing in Example 4.
FIG. 9 is a schematic diagram illustrating a Sanger analysis result for CBE-141 single base editing in Example 4.
FIG. 10 is a schematic diagram illustrating a Sanger analysis result for CBE-143 single base editing in Example 4.
FIG. 11 is a schematic diagram illustrating validation results for single base editors containing deaminase-145 mutants in Example 5.
FIG. 12 is a schematic diagram illustrating validation results for single base editors containing deaminase-145 mutants in Example 6.

### DETAILED DESCRIPTION

### TERMS

### SEQUENCE IDENTITY

As used herein, the term 'identity' (or 'percent identity') refers to sequence matching between two polypeptides or between two nucleic acids. When a position in two sequences being compared is occupied by the same base or amino acid monomer subunit (e.g., when a position in each of two DNA molecules is occupied by adenine, or a position in each of two polypeptides is occupied by lysine), the sequences or molecules are identical at that position. The 'percent identity' between two sequences is a function of the number of matching positions shared by the two sequences divided by the number of positions being compared, multiplied by 100%. For example, if six out of ten positions of two sequences match, then the two sequences have 60% sequence identity. Typically, comparison is performed when aligning two sequences to produce maximum sequence identity. Such an alignment may be performed using publicly available and commercially available alignment algorithms and programs, such as, but not limited to, CLUSTERal Ω, MAFFT, Probcons, T-Coffee, Probalign, BLAST, which may be reasonably selected by a person of ordinary skill in the art. The person of ordinary skill in the art can determine suitable parameters for aligning sequences, (e.g., including any algorithms needed to achieve optimal alignment or optimal comparison for the full length of the sequences being compared, and any algorithms needed to achieve optimal alignment or optimal comparison for a local portion of the sequences being compared).

### DEAMINASE

The term 'deaminase' refers to an enzyme that catalyzes a deamination reaction (i.e., removal of an amino group from a base of a nucleotide). In some embodiments, the deaminase is a cytidine deaminase. The cytidine deaminase catalyzes cytidine deamination to produce uridine, or catalyzes deoxycytidine deamination to produce deoxyuridine; that is, catalyzes cytosine group deamination to produce uracil group. The deaminase may act on DNA and/or RNA.

The terms deaminase and deaminase polypeptide may be used interchangeably.

In some embodiments, the deaminase may further comprise a homologous or heterologous domain. Non-limiting examples include: the deaminase further comprising a nucleic acid-binding polypeptide (e.g., the deaminase comprising a Cas9 nickase domain, the deaminase comprising a Cas12 domain, the deaminase comprising a TnpB domain, or the deaminase comprising a Talen domain), the deaminase further comprising a cellular localization signal (e.g., an NLS, a nuclear export signal, a mitochondrial localization signal, or a chloroplast localization signal), or the deaminase further comprising a UGI domain.

### DEAMINATION

In some embodiments, the deamination refers to catalyzing the deamination of the base in the DNA and/or RNA molecule. In some embodiments, the deamination refers to catalyzing the deamination of the base in a DNA molecule. In some embodiments, the deamination refers to catalyzing the deamination of the base in an RNA molecule. In some embodiments, the deamination refers to catalyzing the cytidine deamination to produce uridine, or catalyzing the deoxycytidine deamination to produce deoxyuridine. In some embodiments, the deamination refers to catalyzing the cytosine group deamination to produce the uracil group.

In some embodiments, an expected edited base is upstream of a PAM site. In some embodiments, the expected edited base is the 1st, 2nd, 3rd, 4th, 5th, 6th, 7th, 8th, 9th, 10th, 11th, 12th, 13th, 14th, 15th, 16th, 17th, 18th, 19th, 20th, 21st, 22nd, 23rd, 24th, 25th, 26th, 27th, 28th, 29th, 30th, 31st, 32nd, 33rd, 34th, 35th, 36th, 37th, 38th, 39th, or 40th nucleotide upstream of the PAM site. In some embodiments, the expected edited base is downstream of the PAM site. In some embodiments, the expected edited base is the 1st, 2nd, 3rd, 4th, 5th, 6th, 7th, 8th, 9th, 10th, 11th, 12th, 13th, 14th, 15th, 16th, 17th, 18th, 19th, 20th, 21st, 22nd, 23rd, 24th, 25th, 26th, 27th, 28th, 29th, 30th, 31st, 32nd, 33rd, 34th, 35th, 36th, 37th, 38th, 39th, or 40th nucleotide downstream of the PAM site. The edited base is a base that undergoes deamination. The position of the edited base is an editing window. In some embodiments, a target sequence comprises the editing window. In some embodiments, the editing window comprises 1 to 10 nucleotides. In some embodiments, the length of the editing window is 1 to 9, 1 to 8, 1 to 7, 1 to 6, 1 to 5, 1 to 4, 1 to 3, 1 to 2, or 1 nucleotide(s). In some embodiments, the length of the editing window is 1, 2, 3, 4, 5, 6, 7, 8, 9, 10, 11, 12, 13, 14, 15, 16, 17, 18, 19, or 20 nucleotide(s).

### FUSION PROTEIN

In the present disclosure, when referring to 'a fusion protein comprising XXXX deaminase' or using a similar description, it is intended to mean that the amino acid sequence of the fusion protein comprises the amino acid sequence constituting the deaminase.

In the present disclosure, when referring to 'a fusion protein comprising XXXX nucleic acid-binding polypeptide' or using a similar description, it is intended to mean that the amino acid sequence of the fusion protein comprises the amino acid sequence constituting the nucleic acid-binding polypeptide.

### PROTEINS, PEPTIDES, AND POLYPEPTIDES

The terms 'protein', 'peptide', and 'polypeptide' may be used interchangeably in the present disclosure and refer to polymers of amino acid residues linked together by peptide (amide) bonds. The term refers to a protein, peptide, or polypeptide of any size, structure, or function. Generally, a protein, peptide, or polypeptide has a length of at least three amino acids. A protein, peptide, or polypeptide may refer to a single protein or a collection of proteins. One or more amino acids in a protein, peptide, or polypeptide may be modified, for example, by adding a chemical entity (e.g., a carbohydrate group, a hydroxyl group, a phosphate group, a farnesyl group, an isofarnesyl group, a fatty acid group, a linker for conjugation, functionalization, or other modifications, or the like). A protein, peptide, or polypeptide may also be a single molecule or a multimolecular complex. A protein, peptide, or polypeptide may merely be a fragment of a naturally occurring protein or peptide. A protein, peptide, or polypeptide may be naturally occurring, recombinant, or synthetic, or any combination thereof.

### Nucleic acid-binding polypeptide

Optionally, the nucleic acid-binding polypeptide is a DNA binding polypeptide.

Optionally, the nucleic acid-binding polypeptide is an RNA binding polypeptide.

Optionally, the nucleic acid-binding polypeptide is a TALEN nuclease, a ZFN, a Cas enzyme, or a meganuclease.

In some embodiments of the present disclosure, the nucleic acid-binding polypeptide is an RNA-guided nuclease (RGN).

In some embodiments of the present disclosure, the DNA binding polypeptide may be the RNA-guided nuclease.

In some embodiments of the present disclosure, the RNA binding polypeptide may be the RNA-guided nuclease.

### RNA-Guided Nuclease

The term RNA-guided nuclease refers to a polypeptide that binds to a specific target polynucleotide in a sequence-specific manner, and the polypeptide is guided to the target polynucleotide by a gRNA molecule that complexes with the polypeptide and hybridizes with the target polynucleotide. Although the RNA-guided nuclease can cleave the target sequence upon binding, the term RNA-guided nuclease also includes a nuclease-inactivated RNA-guided nuclease that is capable of binding but not cleaving the target sequence. The cleavage of the target sequence by the RNA-guided nuclease may result in single-stranded or double-stranded breaks. The RNA-guided nuclease that is capable of cleaving only one strand of a double-stranded nucleic acid molecule is referred to as a nickase in the present disclosure.

Optionally, the DNA binding polypeptide may be a CRISPR-associated nuclease, i.e., a Cas enzyme (also referred to as a Cas protein).

In some embodiments of the present disclosure, the nucleic acid-binding polypeptide is selected from Cas1, Cas1B, Cas2, Cas3, Cas4, Cas5, Cas5d, Cas5t, Cas5h, Cas5a, Cas6, Cas7, Cas8, Cas8a, Cas8b, Cas8c, Cas9, Cas10, Cas10d, Cas12a/Cpf1, Cas12b/C2c1, Cas12c/C2c3, Cas12d/CasY, Cas12e/CasX, Cas12f/CasZ, Cas12g, Cas12h, Cas12i, Csy1, Csy2, Csy3, Csy4, Cse1, Cse2, Cse3, Cse4, Cse5e, Csc1, Csc2, Csa5, Csn1, Csn2, Csm1, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx1S, Csx11, Csf1, Csf2, CsO, Csf4, Csd1, Csd2, Cst1, Cst2, Csh1, Csh2, Csa1, Csa2, Csa3, Csa4, Csa5, Cas13a, Cas13b, Cas13c, Cas13d, Cas13e, Cas13f, TnpB, IscB, IsrB, and a Fancor nuclease; or a fragment of any one of them. A non-limiting example of the fragment may be a nucleic acid binding domain fragment.

In some embodiments of the present disclosure, the nucleic acid-binding polypeptide is selected from Cas9, Cas12, Cas13, TnpB, IscB, IsrB, a Fancor nuclease, or a fragment of any one of them, including but not limited to the nucleic acid binding domain fragment.

In some embodiments of the present disclosure, the Cas9 is selected from SpCas9, SaCas9, Nme2Cas9, Nme3Cas9, CjCas9, NmCas9, FnCas9, PpnCas9, FrCas9, SauCas9, SauriCas9, ScaCas9, St1Cas9, BlatCas9, CdiCas9, and GeoCas9, a fragment of any one of them, and a mutant of any one of them or a fragment of the mutant.

In some embodiments of the present disclosure, the nucleic acid-binding polypeptide is selected from AsCpf1, enAsCas12a (addgene plasmid #196724), dFnCas12a (addgene plasmid #136379), ErCas12a, LbCas12a D832A, LbCas12a H759A, LbCas12a E795L, FnCas12a3, FnCas12a D917A, AsCas12a R1226A, AsCas12a D908A, AsCas12a E174R/S542R, AsCas12a (S542R/K548V/N552R), PrCas12a, PxCas12a, PcCas12a, PdCas12a, Mb2Cas12a, Mb3Cas12a, MlCas12a, CMaCas12a, CMtCas12a, HkCas12a, Lb5Cas12a, ErCas12a, TsCas12a, FnCpf1, LbCas12a, ttHsCas12a, AaCas12b, AaCas12b D570A, AaCas12b Q119F/E475R/E758R, BhCas12b, BvCas12b, BrCas12b, AkCas12b, AmCas12b, BsCas12b, OspCas12c, Cas12c2 (addgene plasmid #183072), Cas12c_4 (addgene plasmid #183071), Cas12c1 (addgene plasmid #120872), CasY.1 (from Katanobacteria), CasY.2 (from Vogelbacteria), CasY.3 (from Vogelbacteria), CasY.4 (from Parcubacteria), CasY.5 (from Komeilibacteria), CasY.6 (from Kerfeldbacteria), PlmCasX, DpbCasX, Un1Cas12f, CnCas12f1, enRhCas12f1, AsCas12f1, SpaCas12f1, Cas12g1 (addgene plasmid #120879), Cas12h of WO2021113522A1 (SEQ ID NO: 1 in the patent application), Cas12i1 (addgene plasmid #171670), Cas12i2 (addgene plasmid #188275), Cas12i1 (addgene plasmid #120882), Cas12i2 (addgene plasmid #120883), Cas12i protein designated Cas12f.4/Cas12f.5/Cas12f.6 in CN111757889B, dSiCas12i(D1049A), SiCas12i, Si2Cas12i, WiCas12i, Wi2Cas12i, Wi3Cas12i, SaCas12i, Sa2Cas12i, Sa3Cas12i, WaCas12i, Wa2Cas12i, xCas12i, hfCas12Max, Cas12i-Max (addgene plasmid #188276), Cas12i1 D647A (addgene plasmid #171671), Cas12i-HiFi (addgene plasmid #188269), Cas12i1 D647A, Cas12j3 (addgene plasmid #188497), Cas12j2 (addgene plasmid #188498), AsCas12j-2 (addgene plasmid #191655), Cas12j-8 (addgene plasmid #194966), ShCas12k, N7Cas12k, AcCas12k, Cas12k-TniQ (addgene plasmid #181787), Cas12k-TnsC (addgene plasmid #181789), Cas12l, MmCas12m, MmCas12m ΔZF (H549A, C552A), dCas12m-ΔZF (D485A, H549A, C552A), AcCas12n, dAcCas12n(D240), TnpB Actinomadura_cellulosilytica_strain_DSM_45823, TnpB Actinomadura_namibiensis_strain_DSM_44197, TnpB Actinomadura_umbrina_strain_DSM_43927, TnpB Actinoplanes_lobatus_strain_DSM_43150 (TnpB-1 and TnpB-2), TnpB Alicyclobacillus_macrosporagiidus_strain_DSM_17980, TnpB Haloactinospora_alba_Strain_DSM_45015, TnpB Lipingzhangella_halophila_strain_DSM_102030, TnpB Meiothermus_Silvanus_DSM_9946, TnpB QNFX01000004, ISDra2 TnpB(PDB: 8H1J), KraIscB-1, AwaIscB, OgeuIscB, GtFz1 (from Guillardia theta), SpuFz1 (from Spizellomyces punctatus), NlovFz2 (from Percolozoa Naegleria lovaniensis) and MmeFz2 (from Mercenaria mercenaria), a fragment of any one of them, and a mutant of any one of them, or a fragment of the mutant.

In some embodiments of the present disclosure, the DNA binding polypeptide can cleave one strand of a double-stranded nucleic acid molecule (e.g., a double-stranded DNA molecule). Optionally, the DNA binding polypeptide is an RNA-guided nuclease having nickase activity.

In some embodiments of the present disclosure, the Cas enzyme cleaves a target strand of a double-stranded nucleic acid molecule, meaning that the Cas enzyme cleaves a strand that is base-paired with (is complementary to) a gRNA (e.g., an sgRNA) bound to the Cas enzyme.

In some embodiments of the present disclosure, the nucleic acid-binding polypeptide may be an RNA-guided nuclease having inactivated nuclease activity.

In some embodiments of the present disclosure, the nucleic acid-binding polypeptide is a variant of an RNA-guided nuclease with completely inactivated nuclease activity compared to a wild-type form of the RNA-guided nuclease, for example, a dCas enzyme, including but not limited to Cas9, Cas12, Cas13, TnpB, IscB, IsrB, and Fancor, each of which has completely inactivated nuclease activity (which may be referred to as dead Cas9, dead Cas12, dead Cas13, dead TnpB, dead IscB, dead IsrB, and dead Fancor).

In some embodiments of the present disclosure, the nucleic acid-binding polypeptide may be a variant of an RNA-guided nuclease with partially inactivated nuclease activity compared to a wild-type form of the RNA-guided nuclease, such as an nCas enzyme, for example, a polypeptide fragment that retains the nuclease activity of cleaving a single strand of double-stranded DNA, including but not limited to nickase Cas9 and nickase Cas12.

A target polynucleotide binds with the nucleic acid-binding polypeptide provided in the present disclosure, and hybridizes with the gRNA associated with the nucleic acid-binding polypeptide. Then, if the nucleic acid-binding polypeptide has nuclease activity, the target sequence may subsequently be cleaved by the nucleic acid-binding polypeptide. For example, the nucleic acid-binding polypeptide may optionally cleave any one single strand of double-stranded DNA or cleave both single strands, or may bind to the target polynucleotide without cleaving any single strand; simultaneously with or after the nucleic acid-binding polypeptide binds or cleaves the target polynucleotide, the deaminase deaminates at least one base of the target polynucleotide.

In some embodiments, the binding depends on the recognition of a PAM sequence by the RNA-guided nuclease. In some embodiments, the binding does not depend on the recognition of a PAM sequence by the RNA-guided nuclease. The PAM sequences recognized by many nucleases described in the present disclosure are known.

The term 'cleave' or 'cleavage' refers to the hydrolysis of at least one phosphodiester bond within the backbone of the target polynucleotide, which may result in single-stranded or double-stranded breaks within the target sequence. The presently disclosed nucleic acid-binding polypeptide may function as an endonuclease or an exonuclease (removing consecutive nucleotides from the 5' and/or 3' ends of a polynucleotide), thereby cleaving nucleotides within the polynucleotide. In other embodiments, the disclosed nucleic acid-binding polypeptide may cleave nucleotides of the target sequence at any position within the polynucleotide, thereby acting as both an endonuclease and an exonuclease. The cleavage of the target polynucleotide by the presently disclosed nucleic acid-binding polypeptide may result in staggered breaks or blunt ends.

### Nuclear localization signal, nuclear localization sequence, NLS

The terms nuclear localization signal, nuclear localization sequence, and NLS may be used interchangeably. The presently disclosed fusion protein may comprise at least one nuclear localization signal (NLS) to enhance the delivery of the nucleic acid-binding polypeptide to the cell nucleus. The nuclear localization signal is known in the art and typically comprises a stretch of basic amino acids (see, e.g., Lange et al., J. Biol. Chem. (2007) 282:5101-5105). In specific embodiments, the fusion protein comprises two, three, four, five, six, or more nuclear localization signals. One or more nuclear localization signals may be heterologous nuclear localization signals. Non-limiting examples of nuclear localization signal applicable to the presently disclosed RGNs are the NLSs of SV40 large T antigen, nucleoplasmin, and c-Myc (see, e.g., Ray et al., (2015) Bioconjug Chem 26(6):1004-7).

### Linker

As used in the present disclosure, the term 'linker' refers to a chemical group or molecule connecting two molecules or portions (e.g., a binding domain and a cleavage domain of a nuclease). Typically, a linker is located between or on both sides of two groups, molecules, or other portions, and is linked to each group, molecule, or portion via a covalent bond, thereby connecting the two. In some embodiments, the linker is an amino acid or a plurality of amino acids (e.g., a peptide or a protein). In some embodiments, the linker may be an organic molecule, a group, a polymer, or a chemical moiety. In some embodiments, the linker has a length of 5 to 100 amino acids (e.g., having a length of 5, 6, 7, 8, 9, 10, 11, 12, 13, 14, 15, 16, 17, 18, 19, 20, 21, 22, 23, 24, 25, 26, 27, 28, 29, 30, 30 to 35, 35 to 40, 40 to 45, 45 to 50, 50 to 60, 60 to 70, 70 to 80, 80 to 90, 90 to 100, 100 to 150, or 150 to 200 amino acids). Longer or shorter linkers may also be considered.

In some embodiments, the linker comprises a sequence set forth in any one of SEQ ID NOs: 33 to 37 and EFE, or a fragment or any one variant of any one of them.

In some embodiments, the linker comprises a (GGGGS)n (SEQ ID NO: 35), (G)n, (EAAAK)n (SEQ ID NO: 36), (XP)n, or (SGG)n motif, or any combination thereof, wherein n is independently an integer from 1 to 30. In some embodiments, n is independently 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30, or, if there is more than one linker or more than one linker motif, then n is independently any combination thereof. Other suitable linker motifs and linker configurations are apparent to those skilled in the art. In some embodiments, suitable linker motifs and configurations include those described in Chen et al., Fusion protein linkers: property, design and functionality (Adv Drug Deliv Rev. 2013; 65(10):1357-69), the entire contents of which are incorporated herein by reference. Based on the present disclosure, other suitable linker sequences are apparent to those skilled in the art.

### UGI

The fusion protein of the present disclosure may comprise suitable UGI sequences, and additional suitable UGI sequences are known to those skilled in the art and include, for example, those disclosed in Wang et al., 1989 J. Biol. Chem. 264:1163-1171; Lundquist et al., 1997 J. Biol. Chem. 272:21408-21419; Ravishankar et al., 1998 Nucleic Acids Res. 26:4880-4887; and Putnam et al., 1999 J. Mol. Biol. 287:331-346 (1999), the entire contents of each of which are incorporated herein by reference.

### Guide RNA, gRNA, guide polynucleotide

The terms guide RNA, gRNA, and guide polynucleotide may be used interchangeably. In some embodiments of the present disclosure, the gRNA comprises a guide sequence and a scaffold sequence. The scaffold sequence interacts with an RNA-guided nuclease. The scaffold sequence is a region of the gRNA that typically remains constant when designing the gRNA molecule. For example, the scaffold sequence may refer to the portion of a gRNA molecule other than the guide sequence. For example, when the nucleic acid-binding polypeptide is Cas9, Cas12, or Cas13, the gRNA may be a crRNA or a complex of crRNA and tracrRNA (e.g., a single molecule gRNA chimerically formed by crRNA and tracrRNA).

In some embodiments of the present disclosure, the guide sequence hybridizes with the target polynucleotide. The guide sequence refers to a continuous nucleotide sequence in the gRNA, which has partial or complete complementarity with the target polynucleotide (specifically, which has partial or complete complementarity with a target sequence in the target polynucleotide [target nucleic acid]), and may hybridize with the target sequence in the target polynucleotide via base pairing facilitated by an RNA-guided nuclease. Complete complementarity of the guide sequence with the target sequence as described in the present disclosure is not required, as long as there is sufficient complementarity to cause hybridization and facilitate the formation of the gene editing complex. As used in the present disclosure, the term 'target sequence' refers to a small segment of sequence in the target polynucleotide, which may be complementary to the guide sequence of the gRNA molecule (fully or partially complementary). The gene editing complex is sequence-specifically localized to the target sequence via the guide sequence and performs a corresponding function at or near this position. The length of the target sequence is often dozens of nucleotides (nt) (e.g., it may be 10 to 60 nt, 15 to 50 nt, 15 to 40 nt, 15 to 35 nt, 20 to 35 nt, 20 to 30 nt, about 10 nt, about 20 nt, about 30 nt, about 40 nt, about 50 nt, or about 60 nt).

In some embodiments of the present disclosure, the guide sequence has at least 50% sequence identity with the target polynucleotide. In some embodiments of the present disclosure, the guide sequence has at least 60%, at least 70%, at least 80%, at least 85%, at least 90%, at least 95%, or 100% sequence identity with the target polynucleotide.

The length of the guide sequence is often dozens of nucleotides (nt) (e.g., it may be 10 to 60 nt, 15 to 50 nt, 15 to 40 nt, 15 to 35 nt, 20 to 35 nt, 20 to 30 nt, about 10 nt, about 20 nt, about 30 nt, about 40 nt, about 50 nt, or about 60 nt).

In some embodiments of the present disclosure, the guide sequence is located at the 3' end or the 5' end of the scaffold sequence. In some embodiments of the present disclosure, the guide sequence is located at the 3' end of the scaffold sequence. In some embodiments of the present disclosure, the guide sequence is located at the 5' end of the scaffold sequence.

In some embodiments of the present disclosure, the gRNA comprises an aptamer sequence.

In some embodiments of the present disclosure, the aptamer sequence is inserted into the loop of the stem-loop structure of the secondary structure of the gRNA.

In some embodiments of the present disclosure, the guide RNA comprises modified nucleotides. The modifications include but are not limited to 2'-O-methyl modification, 2'-O-methyl-3'-thiophosphorothioate modification, or 2'-O-methyl-3'-thioPACE modification. In some embodiments of the present disclosure, the gRNA comprises modified nucleotide, wherein the modified nucleotide is selected from deoxyribonucleotide and locked nucleic acid (LNA). In some embodiments, the gRNA comprises at least one chemically modified nucleotide.

In some embodiments, the gRNA is a hybrid RNA-DNA guide, meaning that a portion of the RNA nucleotides in the gRNA is replaced by DNA nucleotides. In some embodiments, the gRNA is a hybrid RNA-LNA guide, meaning that a portion of the RNA nucleotides in the gRNA is replaced by LNA nucleotides.

In some embodiments of the present disclosure, the target RNA is located in the nucleus and/or the cytoplasm of a eukaryotic cell.

### Target Polynucleotide

According to the context, the term 'target polynucleotide' may be used interchangeably with 'target nucleic acid' and 'target gene'.

The deaminase or the fusion protein of the present disclosure may deaminate a base in the target polynucleotide, catalyzing the deamination of cytidine to uridine, or catalyzing the deamination of deoxycytidine to deoxyuridine. It may be used to achieve base conversion on DNA molecules or base conversion on RNA molecules. For example, it may be used to edit exon regions, intron regions, and/or regulatory regions (e.g., promoter regions, enhancer regions) of genes; and it may be used to edit precursor messenger RNA (pre-mRNA) molecules, mature messenger RNA (mRNA) molecules, or non-coding RNA molecules.

Some disease-causing mutations are disclosed in a patent application with Publication No. CN108513575A, which is incorporated herein by reference. These mutations may be corrected by the deaminase or the fusion protein of the present disclosure, thereby treating the corresponding diseases.

The deaminase or the fusion protein of the present disclosure may edit genes (normal gene copies or mutant gene copies) in Schedule 1, or their corresponding pre-mRNA molecules or mature mRNA molecules, thereby treating the corresponding diseases or disorders.

When referring to an RNA sequence, 't' in the sequence may be used interchangeably with 'u'. When referring to a guide sequence, 't' in the sequence may be used interchangeably with 'u'.

The present disclosure is further illustrated by the following examples, but the present disclosure is not limited to the scope of the described examples. The experimental methods in the following examples where specific conditions are not specified are carried out in accordance with conventional methods and conditions, or as instructed by the product manuals.

### EXAMPLES

The inventors obtained deaminases-23 to deaminases-34 through bioinformatics analysis and screening.

In addition, deaminases-8 to deaminases-22 and deaminases-36 to deaminases-134 were obtained through structure-based de novo design.

### Experimental Example 1: Synthesis of Expression Plasmids for Different Cytosine Deaminases.

Expression cassettes for cytosine base editors composed of nSpRYCas9, UGI, and different cytosine deaminase sequences were designed and synthesized (using the nucleic acid sequences encoding the deaminase as shown in the sequence listing), and expression cassettes for sgRNAs targeting the VEGFA gene were simultaneously synthesized. Then, the two types of cassettes were inserted into a pCDNA3.1 expression vector, thereby obtaining expression vector plasmids for cytosine base editors comprising different deaminases. The number 'n' of a deaminase-n is identical to the number 'n' in an editor CBE-n and is identical to the number 'n' in a vector plasmid CBE-n-pCDNA3.1. For example, deaminase-27 may be constructed to obtain editor CBE-27, and its expression vector plasmid is CBE-27-pCDNA3.1. Other examples described below also adopt the same representation manner.

### Amino Acid Sequences

This example screened and tested a plurality of cytosine deaminase proteins, and the amino acid sequences of a portion of the deaminases are as follows.
Deaminase-27 (SEQ ID NO: 3): 
HYPEIQPKEFKDVYDPSVLPKVTYLLYEIKWGNSTKIWRNWCRNTSTQHAEINFLENAFRERSFNPL
THCSITWFLSWSPCWKCSQSVVEFLKTYPKVNLEMYVARLFWHEDRSNRLGLRDLVMNGVTIRVM
DLSAYNYCWRTFVSHQNVDDYWPWHLTPWIMFFSFELQLILQVSRSLGGKDSIQKLSRDMYR
Deaminase-29 (SEQ ID NO: 5): 
HTADQGRRIKSWEFTSFFDPSQLRKETSLLSEVWWGSSSRLWRSCSQNSTHHVEQSFLDHFQAQ
RHLGPSTTCTITWFLSWSPCWECARAIRTFLDKYPTVKLTIYVARLYWHMDPQNRQGLRNLVKGGV
DVRIMGPSEHYYCWRNFVDYSRGEEEEWPRYHRVPIHLFDLELHCICLDLPACLTISPVKHQQLLSF
SLTLHTCHYQKLPPALLLLLGLVRPPPPQTSLSVAHRGRGPGTVNGGEWDPNNWGWFWVLR
Deaminase-48 (SEQ ID NO: 6): 
RSSKMPEELFYKYFYPKKDRTGLTTTYLCYELRTQVDATTYEVEKGWIEDEDGVHAYLLFLEKFSAL
NLDPDKKYDVTLYASFSPCYECAKALAEFLKAHPNVSITIKVANLYNCEPGSPNKEGLRLLKEAGVTIS
IMDEADYKYVWNTFVDNKGKEYVPPEGLKENSEELQKKLEEILTPPEVEEARRRALREAGL
Deaminase-136 (SEQ ID NO: 57): 
MEPWPSLPPKAYNFNFRNLREAPNRNSTYLCFEVEEEQLDSPESSYWGVFRNQPSHLHQVYPEEP
LHAEQRFLSWFYDALLSAHAYYRVTWYMSWSPCSECAEEVVSFLGEHENVSLSISASRLYKCDDQ
DQQEGLRLLDQAGVEVAMMSPDDFEYCWDNFVDNRRRSFRYWKGIRRNYHALDDELKEILWG
Deaminase-140 (SEQ ID NO: 58):

SLTADSDPLPCSNSLYRPPRSCLMQRKKFLYHYKNVRWAKGRHETYMCYVVKRRESAQTSSLDFG
FLRNTARAHAEVVFLDYIRDWPLDPGLTYRLTWFSSWSPCYDCAARVVHFLQARPNVRLRLFIARLY
YCSEGMRQPEGLRQLHQAGVGLGIMTYKDYFYCWNNFVANKEKSFKAWEGLHENAVRLSRLLRRI
LE

### Nucleic Acid Sequences

CBE-27-pCDNA3.1 Plasmid (SEQ ID NO: 10): 
GCTAGCGCCACCatgaacggacagccgacggaagcgagttcgagtcaccaaagaagaagcggaaagtccactaccccgag
attcagcccaaggagttcaaggacgtgtacgaccctagcgtgctgcccaaggtgacctacctgctgtacgagatcaagtggggcaacagc
accaagatctggagaaactggtgcagaaacacaagcacacagcacgccgagatcaacttcctggagaacgccttcagagagcggagct
tcaacccccctgacccactgcagcatcacctggttcctgagctggagcccctgctggaagtgcagccaaagcgtggtggagttcctcaagac
ctaccccaaggtgaacctggagatgtacgtggctagactgttctggcacgaggacagaagcaacagactgggcctgcgggacctggtgat
gaacggcgtgaccatcagagtgatggacctgagcgcctacaactactgctggagaaccttcgtgagccatcagaacgtggacgactactg
gccctggcacctgacccccctggatcatgttcttcagcttcgagctgcagctgatcctgcaagtgagcagaagcctgggcggcaaggacagc
attcagaagctgagcagagacatgtacagatctggaggatctagcggaggatcctctggcagcgagacaccaggaacaagcgagtcag
caacaccagagagcagtggcggcagcagcggcggcagcgacaagaagtacagcatcggcctggccatcggcaccaactctgtgggct
gggccgtgatcaccgacgagtacaaggtgcccagcaagaaattcaaggtgctgggcaacaccgaccggcacagcatcaagaagaacc
tgatcggagccctgctgttcgacagcggcgaaacagccgagagaacccggctgaagagaaccgccagaagaagatacaccagacgg
aagaaccggatctgctatctgcaagagatcttcagcaacgagatggccaaggtggacgacagcttcttccacagactggaagagtccttcct
ggtggaagaggataagaagcacgagcggcaccccatcttcggcaacatcgtggacgaggtggcctaccacgagaagtaccccaccatc
taccacctgagaaagaaactggtggacagcaccgacaaggccgacctgcggctgatctatctggccctggcccacatgatcaagttccgg
ggccacttcctgatcgagggcgacctgaaccccgacaacagcgacgtggacaagctgttcatccagctggtgcagacctacaaccagctg
ttcgaggaaaaccccatcaacgccagcggcgtggacgccaaggccatcctgtctgccagactgagcaagagcagacggctggaaaatc
tgatcgcccagctgcccggcgagaagaagaatggcctgttcggaaacctgattgccctgagcctgggcctgacccccaacttcaagagca
acttcgacctggccgaggatgccaaactgcagctgagcaaggacacctacgacgacgacctggacaacctgctggcccagatcggcga
 
ccagtacgccgacctgtttctggccgccaagaacctgtccgacgccatcctgctgagcgacatcctgagagtgaacaccgagatcaccaa
ggcccccctgagcgcctctatgatcaagagatacgacgagcaccaccaggacctgaccctgctgaaagctctcgtgcggcagcagctgc
ctgagaagtacaaagagattttcttcgaccagagcaagaacggctacgccggctacattgacggcggagccagccaggaagagttctac
aagttcatcaagcccatcctggaaaagatggacggcaccgaggaactgctcgtgaagctgaacagagaggacctgctgcggaagcagc
ggaccttcgacaacggcagcatcccccaccagatccacctgggagagctgcacgccattctgcggcggcaggaagatttttacccattcct
gaaggacaaccgggaaaagatcgagaagatcctgaccttccgcatcccctactacgtgggccctctggccaggggaaacagcagattcg
cctggatgaccagaaagagcgaggaaaccatcaccccctggaacttcgaggaagtggtggacaagggcgcttccgcccagagcttcatc
gagcggatgaccaacttcgataagaacctgcccaacgagaaggtgctgcccaagcacagcctgctgtacgagtacttcaccgtgtataac
gagctgaccaaagtgaaatacgtgaccgagggaatgagaaagcccgccttcctgagcggcgagcagaaaaaggccatcgtggacctg
ctgttcaagaccaaccggaaagtgaccgtgaagcagctgaaagaggactacttcaagaaaatcgagtgcttcgactccgtggaaatctcc
ggcgtggaagatcggttcaacgcctccctgggcacataccacgatctgctgaaaattatcaaggacaaggacttcctggacaatgaggaa
aacgaggacattctggaagatatcgtgctgaccctgacactgtttgaggacagagagatgatcgaggaacggctgaaaacctatgcccac
ctgttcgacgacaaagtgatgaagcagctgaagcggcggagatacaccggctggggcaggctgagccggaagctgatcaacggcatcc
gggacaagcagtccggcaagacaatcctggatttcctgaagtccgacggcttcgccaacagaaacttcatgcagctgatccacgacgaca
gcctgacctttaaagaggacatccagaaagcccaggtgtccggccagggcgatagcctgcacgagcacattgccaatctggccggcagc
cccgccattaagaagggcatcctgcagacagtgaaggtggtggacgagctcgtgaaagtgatgggccggcacaagcccgagaacatcg
tgatcgaaatggccagagagaaccagaccacccagaagggacagaagaacagccgcgagagaatgaagcggatcgaagagggca
tcaaagagctgggcagccagatcctgaaagaacaccccgtggaaaacacccagctgcagaacgagaagctgtacctgtactacctgca
gaatggcgggatatgtacgtggaccaggaactggacatcaaccggctgtccgactacgatgtggaccatatcgtgcctcagagctttctga
aggacgactccatcgacaacaaggtgctgaccagaagcgacaagaaccggggcaagagcgacaacgtgccctccgaagaggtcgtg
aagaagatgaagaactactggcggcagctgctgaacgccaagctgattacccagagaaagttcgacaatctgaccaaggccgagagag
gcggcctgagcgaactggataaggccggcttcatcaagagacagctggtggaaacccggcagatcacaaagcacgtggcacagatcct
ggactcccggatgaacactaagtacgacgagaatgacaagctgatccgggaagtgaaagtgatcaccctgaagtccaagctggtgtccg
atttccggaaggatttccagttttacaaagtgcgcgagatcaacaactaccaccacgcccacgacgcctacctgaacgccgtcgtgggaac
cgccctgatcaaaaagtaccctaagctggaaagcgagttcgtgtacggcgactacaaggtgtacgacgtgcggaagatgatcgccaaga
gcgagcaggaaatcggcaaggctaccgccaagtacttcttctacagcaacatcatgaactttttcaagaccgagattaccctggccaacgg
cgagatccggaagcggcctctgatcgagacaaacggcgaaaccggggagatcgtgtgggataagggccgggattttgccaccgtgcgg
aaagtgctgagcatgccccaagtgaatatcgtgaaaaagaccgaggtgcagacaggcggcttcagcaaagagtctatcagacccaaga
ggaacagcgataagctgatcgccagaaagaaggactgggaccctaagaagtacggcggcttcctgtggcccaccgtggcctattctgtgct
ggtggtggccaaagtggaaaagggcaagtccaagaaactgaagagtgtgaaagagctgctggggatcaccatcatggaaagaagcag
cttcgagaagaatcccatcgactttctggaagccaagggctacaaagaagtgaaaaaggacctgatcatcaagctgcctaagtactccctg
ttcgagctggaaaacggccggaagagaatgctggcctctgccaagcagctgcagaagggaaacgaactggccctgccctccaaatatgt
gaacttcctgtacctggccagccactatgagaagctgaagggctcccccgaggataatgagcagaaacagctgtttgtggaacagcacaa
gcactacctggacgagatcatcgagcagatcagcgagttctccaagagagtgatcctggccgacgctaatctggacaaagtgctgtccgcc
tacaacaagcaccgggataagcccatcagagagcaggccgagaatatcatccacctgtttaccctgaccagactgggagcccctagagc
cttcaagtactttgacaccaccatcgaccccaagcagtacagaagcaccaaagaggtgctggacgccaccctgatccaccagagcatca
ccggcctgtacgagacacggatcgacctgtctcagctgggaggtgacagcggcgggagcggcgggagcgggggagcactaatctga
gcgacatcattgagaaggagactgggaaacagctggtcattcaggagtccatcctgatgctgcctgaggaggtggaggaagtgatcggca
 
acaagccagagtctgacatcctggtgcacaccgcctacgacgagtccacagatgagaatgtgatgctgctgacctctgacgcccccgagta
taagccttgggccctggtcatccaggattctaacggcgagaataagatcaagatgctggaattcgagcccaagaagaagaggaaagtcta
gCTCGAGAATAAAGGAAATTTATTTTCATTGCAATAGTGTGTTGGAATTTTTTGTGTCTCTCAGTTA
ACAAAAAAGCACCGACTCGGTGCCACTTTTTCAAGTTGATAACGGACTAGCCTTATTTTAACTTG
CTATTTCTAGCTCTAAAACGAGGCGGGGTGGAGGGGGTCGGTGTTTCGTCCTTTCCACAAGATA
TATAAAGCCAAGAAATCGAAATACTTTCAAGTTACGGTAAGCATATGATAGTCCATTTTAAAACATA
ATTTTAAAACTGCAAACTACCCAAGAAATTATTACTTTCTACGTCACGTATTTTGTACTAATATCTTT
GTGTTTACAGTCAAATTAATTCCAATTATCTCTCTAACAGCCTTGTATCGTATATGCAAATATGAAGG
AATCATGGGAAATAGGCCCTCTTCGAATAGCAGGCATGCTGGGGATGCGGTGGGCTCTATGGCT
TCTGAGGCGGAAAGAACCAGCTGGGGCTCTAGGGGGTATCCCCACGCGCCCTGTAGCGGCGC
ATTAAGCGCGGCGGGTGTGGTGGTTACGCGCAGCGTGACCGCTACACTTGCCAGCGCCCTAGC
GCCCGCTCCTTTCGCTTTCTTCCCTTCCTTTCTCGCCACGTTCGCCGGCTTTCCCCGTCAAGCT
CTAAATCGGGGGCTCCCTTTAGGGTTCCGATTTAGTGCTTTACGGCACCTCGACCCCAAAAAAC
TTGATTAGGGTGATGGTTCACGTAGTGGGCCATCGCCCTGATAGACGGTTTTTCGCCCTTTGAC
GTTGGAGTCCACGTTCTTTAATAGTGGACTCTTGTTCCAAACTGGAACAACACTCAACCCTATCT
CGGTCTATTCTTTTGATTTATAAGGGATTTTGCCGATTTCGGCCTATTGGTTAAAAAATGAGCTGAT
TTAACAAAAATTTAACGCGAATTAATTCTGTGGAATGTGTGTCAGTTAGGGTGTGGAAAGTCCCC
AGGCTCCCCAGCAGGCAGAAGTATGCAAAGCATGCATCTCAATTAGTCAGCAACCAGGTGTGGA
AAGTCCCCAGGCTCCCCAGCAGGCAGAAGTATGCAAAGCATGCATCTCAATTAGTCAGCAACCA
TAGTCCCGCCCCTAACTCCGCCCATCCCGCCCCTAACTCCGCCCAGTTCCGCCCATTCTCCGCC
CCATGGCTGACTAATTTTTTTTATTTATGCAGAGGCCGAGGCCGCCTCTGCCTCTGAGCTATTCC
AGAAGTAGTGAGGAGGCTTTTTTGGAGGCCTAGGCTTTTGCAAAAAGCTCCCGGGAGCTTGTAT
ATCCATTTTCGGATCTGATCAAGAGACAGGATGAGGATCGTTTCGCATGATTGAACAAGATGGAT
TGCACGCAGGTTCTCCGGCCGCTTGGGTGGAGAGGCTATTCGGCTATGACTGGGCACAACAGA
CAATCGGCTGCTCTGATGCCGCCGTGTTCCGGCTGTCAGCGCAGGGGCGCCCGGTTCTTTTTG
TCAAGACCGACCTGTCCGGTGCCCTGAATGAACTGCAGGACGAGGCAGCGCGGCTATCGTGG
CTGGCCACGACGGGCGTTCCTTGCGCAGCTGTGCTCGACGTTGTCACTGAAGCGGGAAGGGA
CTGGCTGCTATTGGGCGAAGTGCCGGGGCAGGATCTCCTGTCATCTCACCTTGCTCCTGCCGA
GAAAGTATCCATCATGGCTGATGCAATGCGGCGGCTGCATACGCTTGATCCGGCTACCTGCCCA
TTCGACCACCAAGCGAAACATCGCATCGAGCGAGCACGTACTCGGATGGAAGCCGGTCTTGTC
GATCAGGATGATCTGGACGAAGAGCATCAGGGGCTCGCGCCAGCCGAACTGTTCGCCAGGCTC
AAGGCGCGCATGCCCGACGGCGAGGATCTCGTCGTGACCCATGGCGATGCCTGCTTGCCGAAT
ATCATGGTGGAAAATGGCCGCTTTTCTGGATTCATCGACTGTGGCCGGCTGGGTGTGGCGGAC
CGCTATCAGGACATAGCGTTGGCTACCCGTGATATTGCTGAAGAGCTTGGCGGCGAATGGGCTG
ACCGCTTCCTCGTGCTTTACGGTATCGCCGCTCCCGATTCGCAGCGCATCGCCTTCTATCGCCT
TCTTGACGAGTTCTTCTGAGCGGGACTCTGGGGTTCGAAATGACCGACCAAGCGACGCCCAAC
CTGCCATCACGAGATTTCGATTCCACCGCCGCCTTCTATGAAAGGTTGGGCTTCGGAATCGTTTT
CCGGGACGCCGGCTGGATGATCCTCCAGCGCGGGGATCTCATGCTGGAGTTCTTCGCCCACCC
 
CAACTTGTTTATTGCAGCTTATAATGGTTACAAATAAAGCAATAGCATCACAAATTTCACAAATAAA
GCATTTTTTTCACTGCATTCTAGTTGTGGTTTGTCCAAACTCATCAATGTATCTTATCATGTCTGTA
TACCGTCGACCTCTAGCTAGAGCTTGGCGTAATCATGGTCATAGCTGTTTCCTGTGTGAAATTGT
TATCCGCTCACAATTCCACACAACATACGAGCCGGAAGCATAAAGTGTAAAGCCTGGGGTGCCT
AATGAGTGAGCTAACTCACATTAATTGCGTTGCGCTCACTGCCCGCTTTCCAGTCGGGAAACCT
GTCGTGCCAGCTGCATTAATGAATCGGCCAACGCGCGGGGAGAGGCGGTTTGCGTATTGGGCG
CTCTTCCGCTTCCTCGCTCACTGACTCGCTGCGCTCGGTCGTTCGGCTGCGGCGAGCGGTATC
AGCTCACTCAAAGGCGGTAATACGGTTATCCACAGAATCAGGGGATAACGCAGGAAAGAACATG
TGAGCAAAAGGCCAGCAAAAGGCCAGGAACCGTAAAAAGGCCGCGTTGCTGGCGTTTTTCCAT
AGGCTCCGCCCCCCTGACGAGCATCACAAAAATCGACGCTCAAGTCAGAGGTGGCGAAACCCG
ACAGGACTATAAAGATACCAGGCGTTTCCCCCTGGAAGCTCCCTCGTGCGCTCTCCTGTTCCGA
CCCTGCCGCTTACCGGATACCTGTCCGCCTTTCTCCCTTCGGGAAGCGTGGCGCTTTCTCATAG
CTCACGCTGTAGGTATCTCAGTTCGGTGTAGGTCGTTCGCTCCAAGCTGGGCTGTGTGCACGAA
CCCCCCGTTCAGCCCGACCGCTGCGCCTTATCCGGTAACTATCGTCTTGAGTCCAACCCGGTAA
GACACGACTTATCGCCACTGGCAGCAGCCACTGGTAACAGGATTAGCAGAGCGAGGTATGTAGG
CGGTGCTACAGAGTTCTTGAAGTGGTGGCCTAACTACGGCTACACTAGAAGAACAGTATTTGGTA
TCTGCGCTCTGCTGAAGCCAGTTACCTTCGGAAAAAGAGTTGGTAGCTCTTGATCCGGCAAACA
AACCACCGCTGGTAGCGGTTTTTTTGTTTGCAAGCAGCAGATTACGCGCAGAAAAAAAGGATCT
CAAGAAGATCCTTTGATCTTTTCTACGGGGTCTGACGCTCAGTGGAACGAAAACTCACGTTAAG
GGATTTTGGTCATGAGATTATCAAAAAGGATCTTCACCTAGATCCTTTTAAATTAAAAATGAAGTTT
TAAATCAATCTAAAGTATATATGAGTAAACTTGGTCTGACAGTTACCAATGCTTAATCAGTGAGGCA
CCTATCTCAGCGATCTGTCTATTTCGTTCATCCATAGTTGCCTGACTCCCCGTCGTGTAGATAACT
ACGATACGGGAGGGCTTACCATCTGGCCCCAGTGCTGCAATGATACCGCGAGACCCACGCTCA
CCGGCTCCAGATTTATCAGCAATAAACCAGCCAGCCGGAAGGGCCGAGCGCAGAAGTGGTCCT
GCAACTTTATCCGCCTCCATCCAGTCTATTAATTGTTGCCGGGAAGCTAGAGTAAGTAGTTCGCC
AGTTAATAGTTTGCGCAACGTTGTTGCCATTGCTACAGGCATCGTGGTGTCACGCTCGTCGTTTG
GTATGGCTTCATTCAGCTCCGGTTCCCAACGATCAAGGCGAGTTACATGATCCCCCATGTTGTGC
AAAAAAGCGGTTAGCTCCTTCGGTCCTCCGATCGTTGTCAGAAGTAAGTTGGCCGCAGTGTTAT
CACTCATGGTTATGGCAGCACTGCATAATTCTCTTACTGTCATGCCATCCGTAAGATGCTTTTCTG
TGACTGGTGAGTACTCAACCAAGTCATTCTGAGAATAGTGTATGCGGCGACCGAGTTGCTCTTG
CCCGGCGTCAATACGGGATAATACCGCGCCACATAGCAGAACTTTAAAAGTGCTCATCATTGGAA
AACGTTCTTCGGGGCGAAAACTCTCAAGGATCTTACCGCTGTTGAGATCCAGTTCGATGTAACC
CACTCGTGCACCCAACTGATCTTCAGCATCTTTTACTTTCACCAGCGTTTCTGGGTGAGCAAAAA
CAGGAAGGCAAAATGCCGCAAAAAAGGGAATAAGGGCGACACGGAAATGTTGAATACTCATACT
CTTCCTTTTTCAATATTATTGAAGCATTTATCAGGGTTATTGTCTCATGAGCGGATACATATTTGAAT
GTATTTAGAAAAATAAACAAATAGGGGTTCCGCGCACATTTCCCCGAAAAGTGCCACCTGACGTC
GACGGATCGGGAGATCTCCCGATCCCCTATGGTGCACTCTCAGTACAATCTGCTCTGATGCCGC
ATAGTTAAGCCAGTATCTGCTCCCTGCTTGTGTGTTGGAGGTCGCTGAGTAGTGCGCGAGCAAA
 
ATTTAAGCTACAACAAGGCAAGGCTTGACCGACAATTGCATGAAGAATCTGCTTAGGGTTAGGCG
TTTTGCGCTGCTTCGCGATGTACGGGCCAGATATACGCGTTGACATTGATTATTGACTAGTTATTA
ATAGTAATCAATTACGGGGTCATTAGTTCATAGCCCATATATGGAGTTCCGCGTTACATAACTTACG
GTAAATGGCCCGCCTGGCTGACCGCCCAACGACCCCCGCCCATTGACGTCAATAATGACGTATG
TTCCCATAGTAACGCCAATAGGGACTTTCCATTGACGTCAATGGGTGGAGTATTTACGGTAAACT
GCCCACTTGGCAGTACATCAAGTGTATCATATGCCAAGTACGCCCCCTATTGACGTCAATGACGG
TAAATGGCCCGCCTGGCATTATGCCCAGTACATGACCTTATGGGACTTTCCTACTTGGCAGTACA
TCTACGTATTAGTCATCGCTATTACCATGGTGATGCGGTTTTGGCAGTACATCAATGGGCGTGGAT
AGCGGTTTGACTCACGGGGATTTCCAAGTCTCCACCCCATTGACGTCAATGGGAGTTTGTTTTG
GCACCAAAATCAACGGGACTTTCCAAAATGTCGTAACAACTCCGCCCCATTGACGCAAATGGGC
GGTAGGCGTGTACGGTGGGAGGTCTATATAAGCAGAGCTCTCTGGCTAACTAGAGAACCCACTG
CTTACTGGCTTATCGAAATTAATACGACTCACTATAGGGAGACCCAAGCTG
CBE-29-pCDNA3.1 Plasmid (SEQ ID NO: 12):

GCTAGCGCCACCatgaaacggacagccgacggaagcgagttcgagtcaccaaagaagaagcggaaagtccacaccgccgac
caaggcagaagaatcaagagctgggagttcacatccttcttcgaccctagccaactgagaaaggagacaagcctgctgagcgaggtgtg
gtggggcagcagcagcagactgtggagaagctgcagccaaaacagcacccaccacgtggagcagagcttcctggaccacttccaagct
cagagacacctgggccctagcaccacctgcaccatcacctggttcctgagctggagcccctgctgggagtgcgctagagccatcagaacc
ttcctggacaagtaccccaccgtgaagctgaccatctacgtggctagactgtactggcacatggaccctcagaacagacaaggcctgaga
aacctggtgaagggcggcgtggacgtgagaatcatgggccctagcgagcactactgctggagaaacttcgtggactacagcagagg
cgaggaagaggagtggcctagataccacagagtgcccatccacctgttcgacctggagctgcactgcatctgcctggacctgcccgcctgc
ctgaccatcagccccgtgaagcatcagcagctgctgagcttcagcctgaccctgcacacctgtcactatcagaaactgcccccgctctgct
cctgctcctcggcctggtcagaccccccctccccaaacaagcctctccgtcgcccacagagggagaggccccggcaccgtcaacggcg
gcgagtgggaccccaacaactggggctggttctgggtgctgagatctggaggatctagcggaggatcctctggcagcgagacaccagga
acaagcgagtcagcaacaccagagagcagtggcggcagcagcggcggcagcgacaagaagtacagcatcggcctggccatcggca
ccaactctgtgggctgggccgtgatcaccgacgagtacaaggtgcccagcaagaaattcaaggtgctgggcaacaccgaccggcacag
catcaagaagaacctgatcggagccctgctgttcgacagcggcgaaacagccgagagaacccggctgaagagaaccgccagaagaa
gatacaccagacggaagaaccggatctgctatctgcaagagatcttcagcaacgagatggccaaggtggacgacagcttcttccacagac
tggaagagtccttcctggtggaagaggataagaagcacgagcggcaccccatcttcggcaacatcgtggacgaggtggcctaccacgag
aagtaccccaccatctaccacctgagaaagaaactggtggacagcaccgacaaggccgacctgcggctgatctatctggccctggccca
catgatcaagttccggggccacttcctgatcgagggcgacctgaaccccgacaacagcgacgtggacaagctgttcatccagctggtgca
gacctacaaccagctgttcgaggaaaaccccatcaacgccagcggcgtggacgccaaggccatcctgtctgccagactgagcaagagc
agacggctggaaaatctgatcgcccagctgcccggcgagaagaagaatggcctgttcggaaacctgattgccctgagcctgggcctgacc
cccaacttcaagagcaacttcgacctggccgaggatgccaaactgcagctgagcaaggacacctacgacgacgacctggacaacctgct
ggcccagatcggcgaccagtacgccgacctgtttctggccgccaagaacctgtccgacgccatcctgctgagcgacatcctgagagtgaa
caccgagatcaccaaggcccccctgagcgcctctatgatcaagagatacgacgagcaccaccaggacctgaccctgctgaaagctctcg
tgcggcagcagctgcctgagaagtacaaagagattttcttcgaccagagcaagaacggctacgccggctacattgacggcggagccagc
caggaagagttctacaagttcatcaagcccatcctggaaaagatggacggcaccgaggaactgctcgtgaagctgaacagagaggacct
gctgcggaagcagcggaccttcgacaacggcagcatcccccaccagatccacctgggagagctgcacgccattctgcggcggcaggaa

gattttacccattcctgaaggacaaccgggaaaagatcgagaagatcctgaccttccgcatcccctactacgtgggccctctggccagggg
aaacagcagattcgcctggatgaccagaaagagcgaggaaaccatcacccccctggaacttcgaggaagtggtggacaagggcgcttcc
gcccagagcttcatcgagcggatgaccaacttcgataagaacctgcccaacgagaaggtgctgcccaagcacagcctgctgtacgagta
cttcaccgtgtataacgagctgaccaaagtgaaatacgtgaccgagggaatgagaaagcccgccttcctgagcggcgagcagaaaaag
gccatcgtggacctgctgttcaagaccaaccggaaagtgaccgtgaagcagctgaaagaggactacttcaagaaaatcgagtgcttcgac
tccgtggaaatctccggcgtggaagatcggttcaacgcctccctgggcacataccacgatctgctgaaaattatcaaggacaaggacttcct
ggacaatgaggaaaacgaggacattctggaagatatcgtgctgaccctgacactgtttgaggacagagagatgatcgaggaacggctga
aaacctatgcccacctgttcgacgacaaagtgatgaagcagctgaagcggcggagatacaccggctggggcaggctgagccggaagct
gatcaacggcatccgggacaagcagtccggcaagacaatcctggatttcctgaagtccgacggcttcgccaacagaaacttcatgcagct
gatccacgacgacagcctgacctttaaagaggacatccagaaagcccaggtgtccggccagggcgatagcctgcacgagcacattgcc
aatctggccggcagccccgccattaagaagggcatcctgcagacagtgaaggtggtggacgagctcgtgaaagtgatgggccggcaca
agcccgagaacatcgtgatcgaaatggccagagagaaccagaccacccagaagggacagaagaacagccgcgagagaatgaagc
ggatcgaagagggcatcaaagagctgggcagccagatcctgaaagaacaccccgtggaaaacacccagctgcagaacgagaagctg
tacctgtactacctgcagaatgggcgggatatgtacgtggaccaggaactggacatcaaccggctgtccgactacgatgtggaccatatcgt
gcctcagagctttctgaaggacgactccatcgacaacaaggtgctgaccagaagcgacaagaaccggggcaagagcgacaacgtgcc
ctccgaagaggtcgtgaagaagatgaagaactactggcggcagctgctgaacgccaagctgattacccagagaaagttcgacaatctga
ccaaggccgagagaggcggcctgagcgaactggataaggccggcttcatcaagagacagctggtggaaacccggcagatcacaaag
cacgtggcacagatcctggactcccggatgaacactaagtacgacgagaatgacaagctgatccgggaagtgaaagtgatcaccctgaa
gtccaagctggtgtccgatttccggaaggatttccagttttacaaagtgcgcgagatcaacaactaccaccacgcccacgacgcctacctga
acgccgtcgtgggaaccgccctgatcaaaaagtaccctaagctggaaagcgagttcgtgtacggcgactacaaggtgtacgacgtgcgg
aagatgatcgccaagagcgagcaggaaatcggcaaggctaccgccaagtacttcttctacagcaacatcatgaactttttcaagaccgag
attaccctggccaacggcgagatccggaagcggcctctgatcgagacaaacggcgaaaccggggagatcgtgtgggataagggccgg
gattttgccaccgtgcggaaagtgctgagcatgccccaagtgaatatcgtgaaaaagaccgaggtgcagacaggcggcttcagcaaaga
gtctatcagacccaagaggaacagcgataagctgatcgccagaaagaaggactgggaccctaagaagtacggcggcttcctgtggccc
accgtggcctattctgtgctggtggtggccaaagtggaaaagggcaagtccaagaaactgaagagtgtgaaagagctgctggggatcacc
atcatggaaagaagcagcttcgagaagaatcccatcgactttctggaagccaagggctacaaagaagtgaaaaaggacctgatcatcaa
gctgcctaagtactccctgttcgagctggaaaacggccggaagagaatgctggcctctgccaagcagctgcagaagggaaacgaactgg
ccctgccctccaaatatgtgaacttcctgtacctggccagccactatgagaagctgaagggctcccccgaggataatgagcagaaacagct
gtttgtggaacagcacaagcactacctggacgagatcatcgagcagatcagcgagttctccaagagagtgatcctggccgacgctaatctg
gacaaagtgctgtccgcctacaacaagcaccgggataagcccatcagagagcaggccgagaatatcatccacctgtttaccctgaccag
actgggagcccctagagccttcaagtactttgacaccaccatcgaccccaagcagtacagaagcaccaaagaggtgctggacgccaccc
tgatccaccagagcatcaccggcctgtacgagacacggatcgacctgtctcagctgggaggtgacagcggcgggagcggcgggagcgg
ggggagcactaatctgagcgacatcattgagaaggagactgggaaacagctggtcattcaggagtccatcctgatgctgcctgaggaggt
ggaggaagtgatcggcaacaagccagagtctgacatcctggtgcacaccgcctacgacgagtccacagatgagaatgtgatgctgctga
cctctgacgcccccgagtataagccttgggccctggtcatccaggattctaacggcgagaataagatcaagatgctggaattcgagcccaa
gaagaagaggaaagtctagCTCGAGAATAAAGGAAATTTATTTTCATTGCAATAGTGTGTTGGAATTTTTT
GTGTCTCTCAGTTAACAAAAAGCACCGACTCGGTGCCACTTTTTCAAGTTGATAACGGACTAGC
CTTATTTTAACTTGCTATTTCTAGCTCTAAAACGAGGCGGGGTGGAGGGGGTCGGTGTTTCGTC

CTTTCCACAAGATATATAAAGCCAAGAAATCGAAATACTTTCAAGTTACGGTAAGCATATGATAGTC
CATTTTAAAACATAATTTTAAAACTGCAAACTACCCAAGAAATTATTACTTTCTACGTCACGTATTTT
GTACTAATATCTTTGTGTTTACAGTCAAATTAATTCCAATTATCTCTCTAACAGCCTTGTATCGTATAT
GCAAATATGAAGGAATCATGGGAAATAGGCCCTCTTCGAATAGCAGGCATGCTGGGGATGCGGT
GGGCTCTATGGCTTCTGAGGCGGAAAGAACCAGCTGGGGCTCTAGGGGGTATCCCCACGCGC
CCTGTAGCGGCGCATTAAGCGCGGCGGGTGTGGTGGTTACGCGCAGCGTGACCGCTACACTTG
CCAGCGCCCTAGCGCCCGCTCCTTTCGCTTTCTTCCCTTCCTTTCTCGCCACGTTCGCCGGCTT
TCCCCGTCAAGCTCTAAATCGGGGGCTCCCTTTAGGGTTCCGATTTAGTGCTTTACGGCACCTC
GACCCCAAAAAACTTGATTAGGGTGATGGTTCACGTAGTGGGCCATCGCCCTGATAGACGGTTT
TTCGCCCTTTGACGTTGGAGTCCACGTTCTTTAATAGTGGACTCTTGTTCCAAACTGGAACAACA
CTCAACCCTATCTCGGTCTATTCTTTTGATTTATAAGGGATTTTGCCGATTTCGGCCTATTGGTTAA
AAAATGAGCTGATTTAACAAAAATTTAACGCGAATTAATTCTGTGGAATGTGTGTCAGTTAGGGTG
TGGAAAGTCCCCAGGCTCCCCAGCAGGCAGAAGTATGCAAAGCATGCATCTCAATTAGTCAGCA
ACCAGGTGTGGAAAGTCCCCAGGCTCCCCAGCAGGCAGAAGTATGCAAAGCATGCATCTCAATT
AGTCAGCAACCATAGTCCCGCCCCTAACTCCGCCCATCCCGCCCCTAACTCCGCCCAGTTCCG
CCCATTCTCCGCCCCATGGCTGACTAATTTTTTTTATTTATGCAGAGGCCGAGGCCGCCTCTGCC
TCTGAGCTATTCCAGAAGTAGTGAGGAGGCTTTTTTGGAGGCCTAGGCTTTTGCAAAAAGCTCC
CGGGAGCTTGTATATCCATTTTCGGATCTGATCAAGAGACAGGATGAGGATCGTTTCGCATGATT
GAACAAGATGGATTGCACGCAGGTTCTCCGGCCGCTTGGGTGGAGAGGCTATTCGGCTATGAC
TGGGCACAACAGACAATCGGCTGCTCTGATGCCGCCGTGTTCCGGCTGTCAGCGCAGGGGCG
CCCGGTTCTTTTTGTCAAGACCGACCTGTCCGGTGCCCTGAATGAACTGCAGGACGAGGCAGC
GCGGCTATCGTGGCTGGCCACGACGGGCGTTCCTTGCGCAGCTGTGCTCGACGTTGTCACTGA
AGCGGGAAGGGACTGGCTGCTATTGGGCGAAGTGCCGGGGCAGGATCTCCTGTCATCTCACCT
TGCTCCTGCCGAGAAAGTATCCATCATGGCTGATGCAATGCGGCGGCTGCATACGCTTGATCCG
GCTACCTGCCCATTCGACCACCAAGCGAAACATCGCATCGAGCGAGCACGTACTCGGATGGAA
GCCGGTCTTGTCGATCAGGATGATCTGGACGAAGAGCATCAGGGGCTCGCGCCAGCCGAACTG
TTCGCCAGGCTCAAGGCGCGCATGCCCGACGGCGAGGATCTCGTCGTGACCCATGGCGATGC
CTGCTTGCCGAATATCATGGTGGAAAATGGCCGCTTTTCTGGATTCATCGACTGTGGCCGGCTG
GGTGTGGCGGACCGCTATCAGGACATAGCGTTGGCTACCCGTGATATTGCTGAAGAGCTTGGC
GGCGAATGGGCTGACCGCTTCCTCGTGCTTTACGGTATCGCCGCTCCCGATTCGCAGCGCATC
GCCTTCTATCGCCTTCTTGACGAGTTCTTCTGAGCGGGACTCTGGGGTTCGAAATGACCGACCA
AGCGACGCCCAACCTGCCATCACGAGATTTCGATTCCACCGCCGCCTTCTATGAAAGGTTGGGC
TTCGGAATCGTTTTCCGGGACGCCGGCTGGATGATCCTCCAGCGCGGGGATCTCATGCTGGAG
TTCTTCGCCCACCCCAACTTGTTTATTGCAGCTTATAATGGTTACAAATAAAGCAATAGCATCACAA
ATTTCACAAATAAAGCATTTTTTTCACTGCATTCTAGTTGTGGTTTGTCCAAACTCATCAATGTATC
TTATCATGTCTGTATACCGTCGACCTCTAGCTAGAGCTTGGCGTAATCATGGTCATAGCTGTTTCC
TGTGTGAAATTGTTATCCGCTCACAATTCCACACAACATACGAGCCGGAAGCATAAAGTGTAAAG
CCTGGGGTGCCTAATGAGTGAGCTAACTCACATTAATTGCGTTGCGCTCACTGCCCGCTTTCCA

GTCGGGAAACCTGTCGTGCCAGCTGCATTAATGAATCGGCCAACGCGCGGGGAGAGGCGGTTT
GCGTATTGGGCGCTCTTCCGCTTCCTCGCTCACTGACTCGCTGCGCTCGGTCGTTCGGCTGCG
GCGAGCGGTATCAGCTCACTCAAAGGCGGTAATACGGTTATCCACAGAATCAGGGGATAACGCA
GGAAAGAACATGTGAGCAAAAGGCCAGCAAAAGGCCAGGAACCGTAAAAAGGCCGCGTTGCTG
GCGTTTTTCCATAGGCTCCGCCCCCCTGACGAGCATCACAAAAATCGACGCTCAAGTCAGAGGT
GGCGAAACCCGACAGGACTATAAAGATACCAGGCGTTTCCCCCTGGAAGCTCCCTCGTGCGCT
CTCCTGTTCCGACCCTGCCGCTTACCGGATACCTGTCCGCCTTTCTCCCTTCGGGAAGCGTGG
CGCTTTCTCATAGCTCACGCTGTAGGTATCTCAGTTCGGTGTAGGTCGTTCGCTCCAAGCTGGG
CTGTGTGCACGAACCCCCCGTTCAGCCCGACCGCTGCGCCTTATCCGGTAACTATCGTCTTGAG
TCCAACCCGGTAAGACACGACTTATCGCCACTGGCAGCAGCCACTGGTAACAGGATTAGCAGAG
CGAGGTATGTAGGCGGTGCTACAGAGTTCTTGAAGTGGTGGCCTAACTACGGCTACACTAGAAG
AACAGTATTTGGTATCTGCGCTCTGCTGAAGCCAGTTACCTTCGGAAAAAGAGTTGGTAGCTCTT
GATCCGGCAAACAAACCACCGCTGGTAGCGGTTTTTTTGTTTGCAAGCAGCAGATTACGCGCAG
AAAAAAAGGATCTCAAGAAGATCCTTTGATCTTTTCTACGGGGTCTGACGCTCAGTGGAACGAAA
ACTCACGTTAAGGGATTTTGGTCATGAGATTATCAAAAAGGATCTTCACCTAGATCCTTTTAAATTA
AAAATGAAGTTTTAAATCAATCTAAAGTATATATGAGTAAACTTGGTCTGACAGTTACCAATGCTTA
ATCAGTGAGGCACCTATCTCAGCGATCTGTCTATTTCGTTCATCCATAGTTGCCTGACTCCCCGT
CGTGTAGATAACTACGATACGGGAGGGCTTACCATCTGGCCCCAGTGCTGCAATGATACCGCGA
GACCCACGCTCACCGGCTCCAGATTTATCAGCAATAAACCAGCCAGCCGGAAGGGCCGAGCGC
AGAAGTGGTCCTGCAACTTTATCCGCCTCCATCCAGTCTATTAATTGTTGCCGGGAAGCTAGAGT
AAGTAGTTCGCCAGTTAATAGTTTGCGCAACGTTGTTGCCATTGCTACAGGCATCGTGGTGTCAC
GCTCGTCGTTTGGTATGGCTTCATTCAGCTCCGGTTCCCAACGATCAAGGCGAGTTACATGATCC
CCCATGTTGTGCAAAAAAGCGGTTAGCTCCTTCGGTCCTCCGATCGTTGTCAGAAGTAAGTTGG
CCGCAGTGTTATCACTCATGGTTATGGCAGCACTGCATAATTCTCTTACTGTCATGCCATCCGTAA
GATGCTTTTCTGTGACTGGTGAGTACTCAACCAAGTCATTCTGAGAATAGTGTATGCGGCGACCG
AGTTGCTCTTGCCCGGCGTCAATACGGGATAATACCGCGCCACATAGCAGAACTTTAAAAGTGCT
CATCATTGGAAAACGTTCTTCGGGGCGAAAACTCTCAAGGATCTTACCGCTGTTGAGATCCAGTT
CGATGTAACCCACTCGTGCACCCAACTGATCTTCAGCATCTTTTACTTTCACCAGCGTTTCTGGG
TGAGCAAAAACAGGAAGGCAAAATGCCGCAAAAAAGGGAATAAGGGCGACACGGAAATGTTGA
ATACTCATACTCTTCCTTTTTCAATATTATTGAAGCATTTATCAGGGTTATTGTCTCATGAGCGGATA
CATATTTGAATGTATTTAGAAAAATAAACAAATAGGGGTTCCGCGCACATTTCCCCGAAAAGTGCC
ACCTGACGTCGACGGATCGGGAGATCTCCCGATCCCCTATGGTGCACTCTCAGTACAATCTGCT
CTGATGCCGCATAGTTAAGCCAGTATCTGCTCCCTGCTTGTGTGTTGGAGGTCGCTGAGTAGTG
CGCGAGCAAAATTTAAGCTACAACAAGGCAAGGCTTGACCGACAATTGCATGAAGAATCTGCTTA
GGGTTAGGCGTTTTGCGCTGCTTCGCGATGTACGGGCCAGATATACGCGTTGACATTGATTATTG
ACTAGTTATTAATAGTAATCAATTACGGGGTCATTAGTTCATAGCCCATATATGGAGTTCCGCGTTA
CATAACTTACGGTAAATGGCCCGCCTGGCTGACCGCCCAACGACCCCCGCCCATTGACGTCAAT
AATGACGTATGTTCCCATAGTAACGCCAATAGGGACTTTCCATTGACGTCAATGGGTGGAGTATTT

ACGGTAAACTGCCCACTTGGCAGTACATCAAGTGTATCATATGCCAAGTACGCCCCCTATTGACG
TCAATGACGGTAAATGGCCCGCCTGGCATTATGCCCAGTACATGACCTTATGGGACTTTCCTACT
TGGCAGTACATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGTCAATGGG
AGTTTGTTTTGGCACCAAAATCAACGGGACTTTCCAAAATGTCGTAACAACTCCGCCCCATTGAC
GCAAATGGGCGGTAGGCGTGTACGGTGGGAGGTCTATATAAGCAGAGCTCTCTGGCTAACTAGA
GAACCCACTGCTTACTGGCTTATCGAAATTAATACGACTCACTATAGGGAGACCCAAGCTG

Note: the above-mentioned CBE-27-pCDNA3.1 and CBE-29-pCDNA3.1 vector sequences are specially labeled. The lowercase italicized portion is the coding sequence of the cytosine base editor fusion protein, the lowercase underlined portion is the deaminase coding sequence, and the bold, underlined uppercase portion encodes the guide sequence of a gRNA targeting VEGFA.

The sequences of the expression plasmids for cytosine base editors comprising other deaminases are consistent with the sequences of the above-mentioned deaminases-27 and deaminases-29, merely replacing the deaminase coding sequence with their respective specific sequences (selected from the corresponding deaminase coding sequences in the sequence listing).

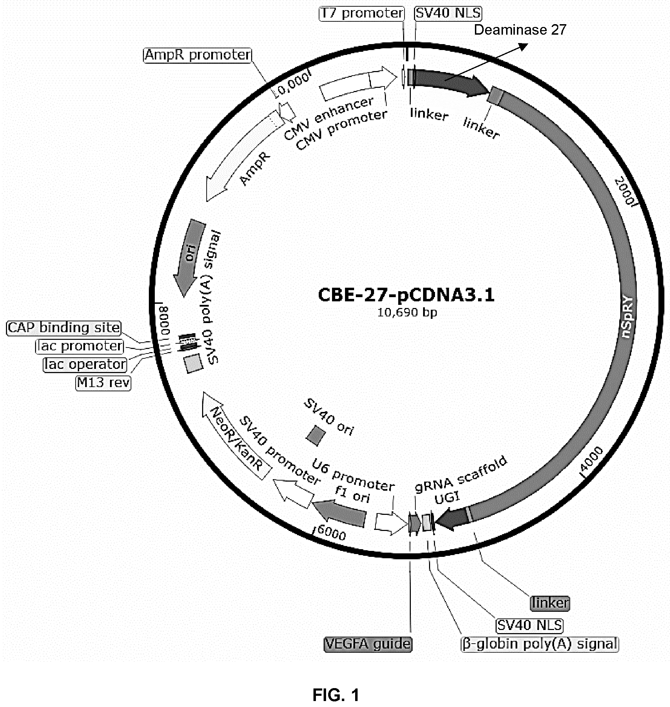

A plasmid map of CBE-27-pCDNA3.1 is exemplarily shown in FIG. 1.

Meanwhile, the deaminase reported in the literature (Huang J et al. Discovery of deaminase functions by structure-based protein clustering. Cell. 2023 Jul 20;186(15):3182-3195.e14. doi: 10.1016/j.cell.2023.05.041. Epub 2023 Jun 27. PubMed Identifier (PMID): 37379837.) was used as a control, wherein the coding sequence of deaminase-03 (numbered as Sdd5 in the literature) reported in the literature is as follows.
CBE-03 (SEQ ID NO: 7):

ATGGGCCCCCTCCTGGACGGGATTGCTGCTCGGCTGGAGGCCGTGAGAGCCGCCCTGCTGGG
GGAGGGCGGGGGCGCCGGGGACGACGAGCCCCCGCCGTGCCCTGGGACAGAGTGGAGAG
ACTGAGAAGAGAGCTGCCCCCTCCCGTGGTGCCCAACACCGGGCAAAAGACCCACGGCAGAT
GGATCGGCCCCGACGGCCAAGCTAGACCCATCGTGAGCGGCCGGGACGACAAGAGCGTGCTG
GTGAACCCCCTGCTGAGAGGCAAGGGCGCCCCCGGCCCCACAAGAAGAGACAGCGACGTGG
AGATGAAGCTGGCCGCCCACATGGCCGCTAGAGGCATCAGACACGCCACCGTGGTGATCAACA
ACACCCCCTGCAGAGGCCCCCTGAGATGCGACACCCTGGTGCCCATCCTGCTGCCCGAGGGC
AGCACCCTGACCGTGCACGGCATCAACGAGAACGGCACAAGAACAAGAATCAGATACACCGGC
GGCGCTAGACCCTGGTGGAGC

An expression plasmid for a base editor fused with deaminase-03, namely CBE-03-pCDNA3.1, was constructed using the same method as described above for subsequent control tests.

### Experimental Example 2: Detection of Editing Efficiency of Base Editors Comprising Different Cytosine Deaminases.

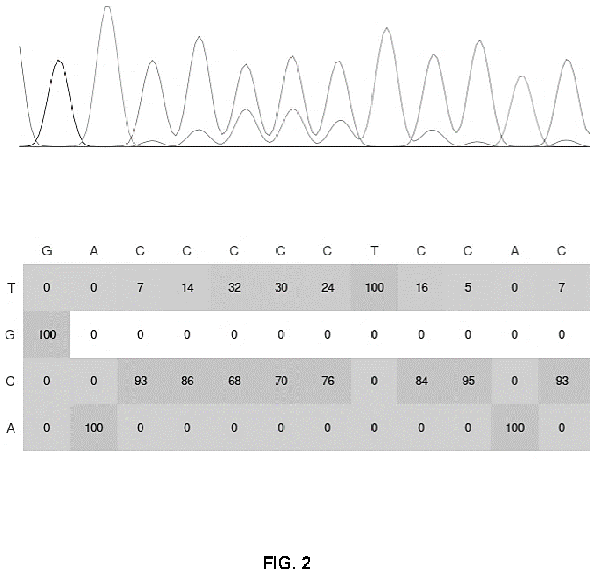

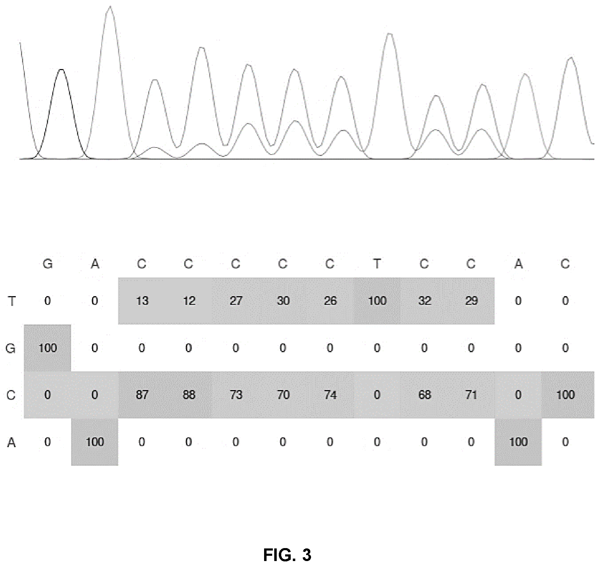

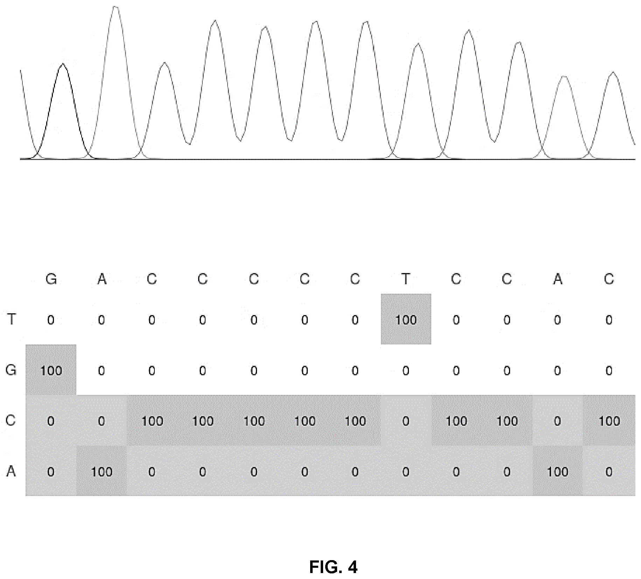

a. Cell Seeding: The 293T cell line was digested when reaching 70%-80% confluency, followed by plating. The seeding density for 24-well plates was 5 × 10⁵ cells per well.
b. Transfection: Transfection was performed after 12-14 hours of cell seeding. For each well of the 24-well plate, 1.5 µl of Polyethylenimine (PEI) (Yeasen Bio, 40815ES03, Polyethylenimine Linear (PEl) MW25000) was added to 100 µl of Opti-MEM, and 500 ng of each of the cytosine base editor plasmids from Example 1 was added separately. After mixing and incubation at room temperature for 20 minutes, the mixture was added to the 293T cells for cell transfection. After overnight transfection, fresh culture medium (Dulbecco's Modified Eagle Medium (DMEM) + 10% FBS) was replaced for continued culture. The negative control group (NC) was not transfected with plasmids.
c. Cell Lysis, Polymerase Chain Reaction (PCR) Amplification Near the Editing Region, Sanger Sequencing: After 72 hours of culture, the cells were washed with PBS, and then 100 µl of cell lysis solution (Viagen, 302-C, DirectPCR^{®} Lysis Reagent (Cell)) was added for lysis, thereby obtaining a lysis solution containing genomic DNA. The region near the genomic DNA target site was amplified, and the PCR product was subjected to Sanger sequencing.
d. Data Analysis: The sequencing chromatograms and the sgRNA guide sequences were submitted to an editing efficiency analysis website (https://moriaritylab.shinyapps.io/editr_v10/) for single-base editing efficiency analysis, thereby obtaining the cytosine editing efficiency of different deaminases at different positions on the target site.
e. Table 1 and FIGS. 2-4 illustrate the editing efficiency of different deaminases for C-to-T base mutation at different positions on the target site.

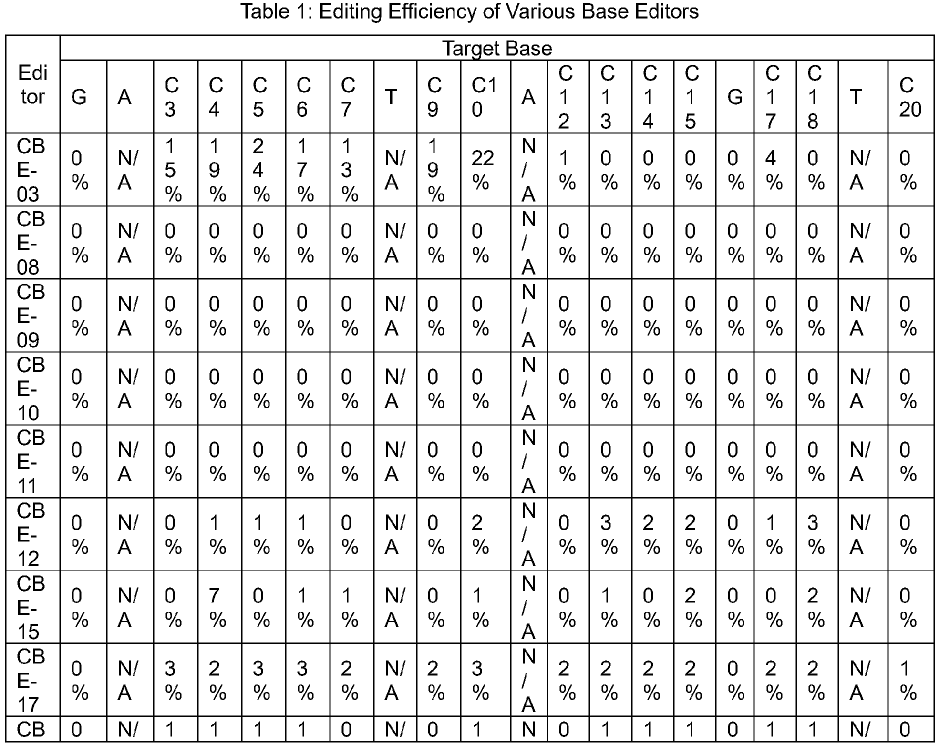

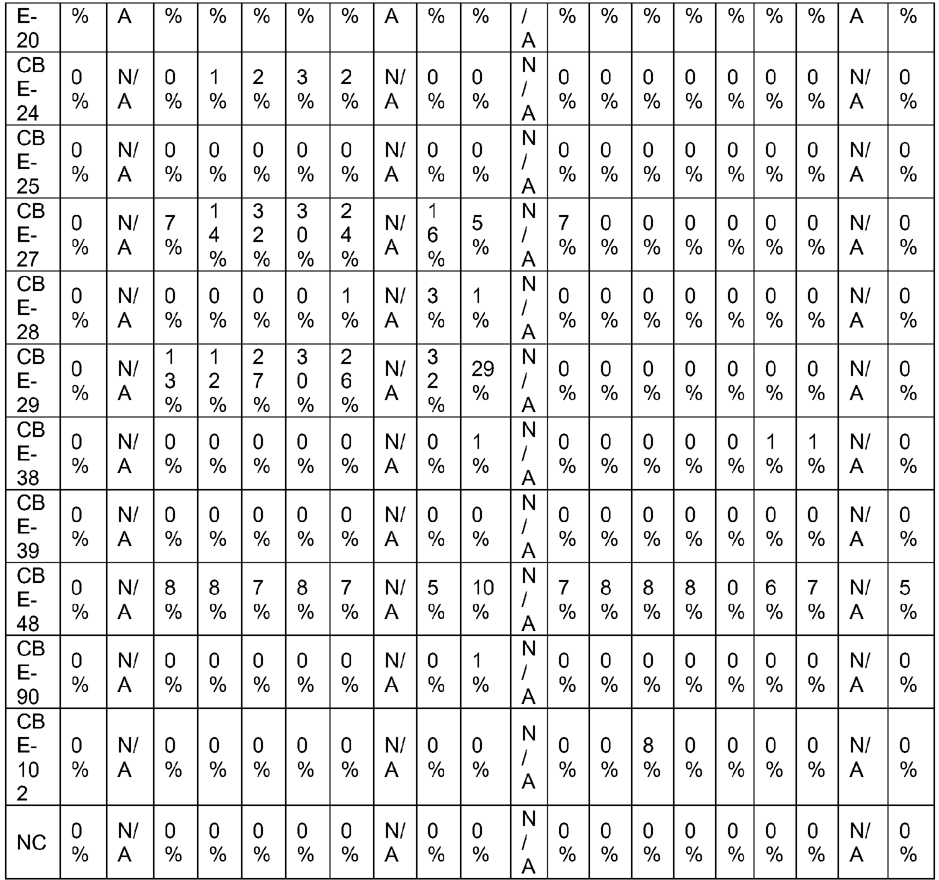

As can be seen from the data in Table 1, deaminases-12, 15, 17, 20, 24, 27 to 29, 38, 48, 90, and 102 are active after being constructed into base editors. Furthermore, the activities of deaminase-27 and deaminase-29 are superior to that of deaminase-03.

The editing window of CBE-48 may be very wide, thereby facilitating its function in scenarios with specific requirements. The editing window of CBE-102 is very narrow, and its specificity is very good, and the position of the editing window is also significantly different from that of other deaminases.

### Experimental Example 3: Editing Efficiency Detection of Base Editors Comprising Different Cytosine Deaminases

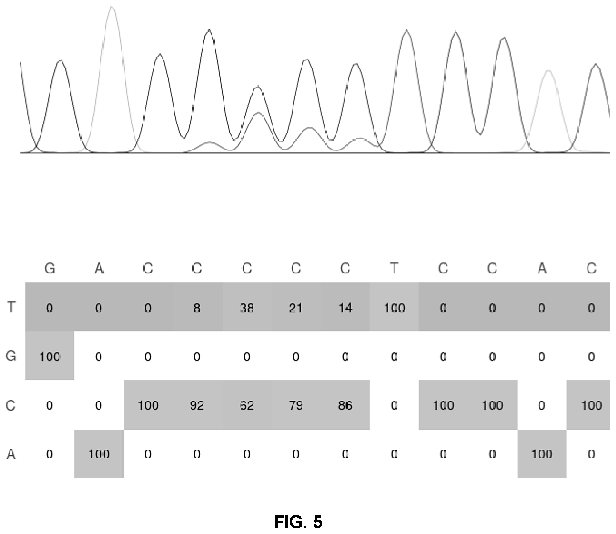

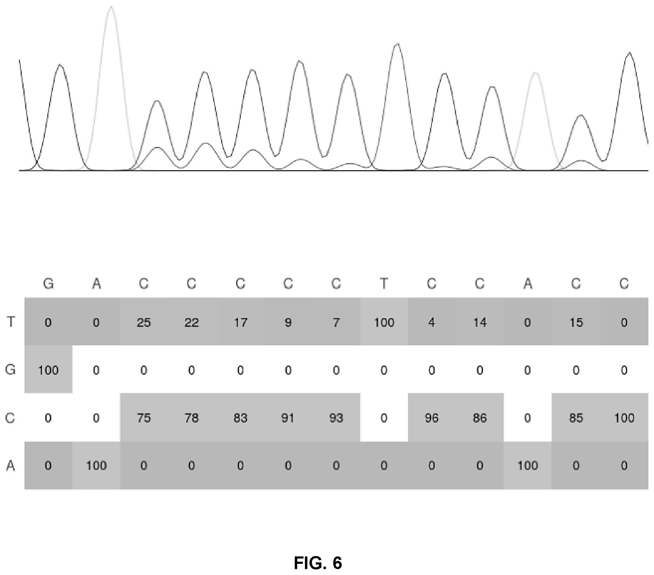

The same manner as in Example 2 was employed to detect the editing efficiency of each base editor in Table 2, and the results shown were obtained. CBE-136 and CBE-140 exhibit high editing efficiency (see FIG. 5 and FIG. 6).

**Table 2: Editing Efficiency of Base Editors**

| Target Base | CBE-136 | CBE-137 | CBE-140 |
|---|---|---|---|
| G | 0% | 0% | 0% |
| A | N/A | N/A | N/A |
| C3 | 0% | 0% | 25% |
| C4 | 8% | 0% | 22% |
| C5 | 38% | 0% | 17% |
| C6 | 21% | 0% | 9% |
| C7 | 14% | 0% | 7% |
| T | N/A | N/A | N/A |
| C9 | 0% | 0% | 4% |
| C10 | 0% | 0% | 14% |
| A | N/A | N/A | N/A |
| C12 | 0% | 0% | 15% |
| C13 | 0% | 0% | 0% |
| C14 | 0% | 0% | 0% |
| C15 | 0% | 0% | 0% |
| G | 0% | 0% | 0% |
| C17 | 0% | 0% | 0% |
| C18 | 0% | 0% | 0% |
| T | N/A | N/A | N/A |
| C20 | 0% | 0% | 0% |

### Experimental Example 4. Detection of Editing Efficiency of Base Editors Comprising Different Cytosine Deaminases

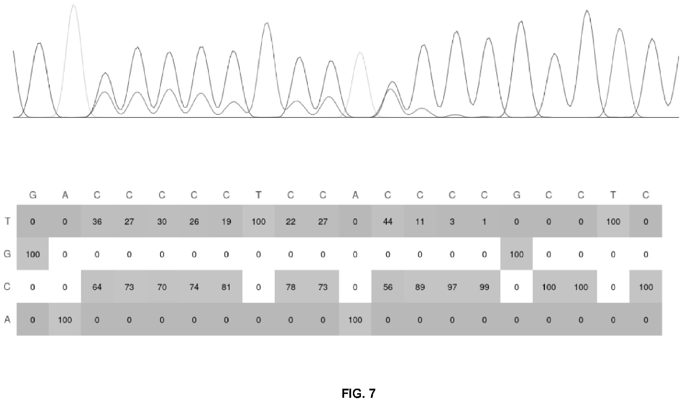

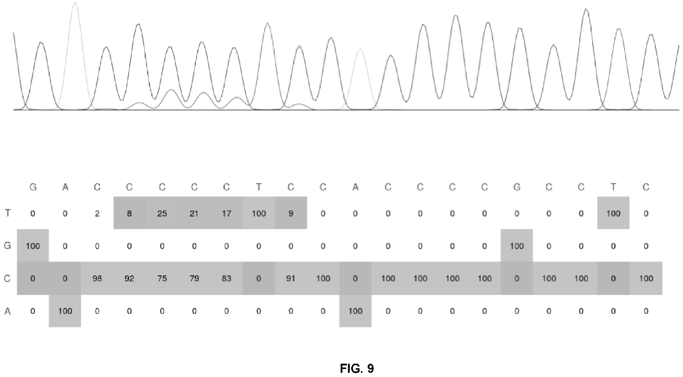

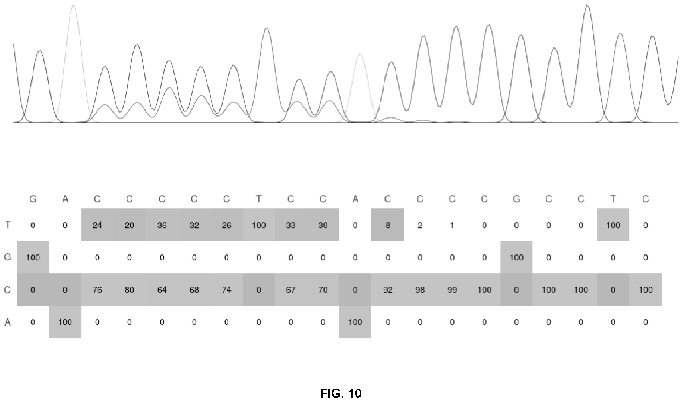

The editing efficiency of the editors comprising deaminase-139, deaminase-145, deaminase-141, and deaminase-143 was detected using the same method as in Example 2. The results are shown in FIGS. 7-10.

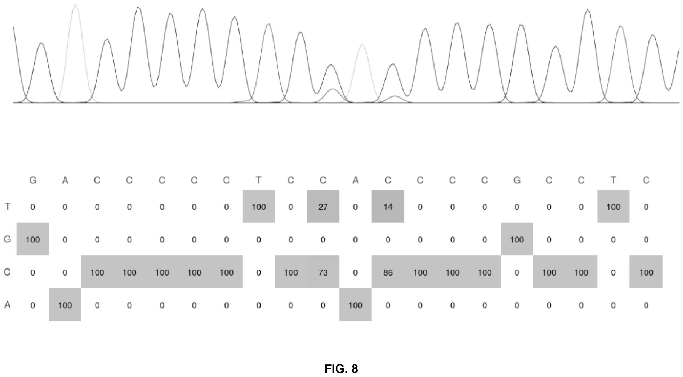

It can be seen from FIG. 8 that CBE-145 has a very narrow editing window and excellent specificity.

### Experimental Example 5. Obtaining and Verifying a Mutant of Deaminase-145

### (1) Screening for Highly Efficient and Highly Specific Mutants of CBE-145

The amino acid sequence of wild-type CBE-145 (207 amino acids) is shown as follows:

MLEIKEDVVHIFRAFWECGVHRGNWCGDTHILYEFGTSPATTDFRNIVTNDGDGGQHAEIKLIYILDN
HIAQMVKCHQRKEIYLNMYMNRSPCYKCAFQLIRFKNYLYNKGYDIRITIKAAQPYMCKRITCSSCVE
GTSHSDGLRELNRNGIFVNAWVWADWQFLAAYLNGLVRVCYDEKFPESGHGKRESRSMADEAAQ
EDFNALR (SEQ ID NO: 301).

Phages expressing deaminase-145 were prepared according to the method described in the literature (Neugebauer M E, Hsu A, Arbab M, et al. Evolution of an adenine base editor into a small, efficient cytosine base editor with low off-target activity[J]. Nature biotechnology, 2023, 41(5): 673-685). Deaminase-145 was evolved by the PANCE method using the screening system and strains of the literature. Finally, the following deaminase-145 mutants were screened out: deaminase-145-P5 (Q122H), deaminase-145-P1 (S191R), deaminase-145-P2 (I114T), deaminase-145-P4 (L2M), and deaminase-145-P6 (N89D).

The amino acid sequence of mutant deaminase-145-P5 (Q122H) is:

MLEIKEDVVHIFRAFWECGVHRGNWCGDTHILYEFGTSPATTDFRNIVTNDGDGGQHAEIKLIYILDN

HIAQMVKCHQRKEIYLNMYMNRSPCYKCAFQLIRFKNYLYNKGYDIRITIKAAHPYMCKRITCSSCVE
GTSHSDGLRELNRNGIFVNAWVWADWQFLAAYLNGLVRVCYDEKFPESGHGKRESRSMADEAAQ
EDFNALR (SEQ ID NO: 329).

### (2) Construction of Vector Plasmids for Verification

Verification vectors were obtained by synthesis from an outsourced service company. These include the CBE-145 expression plasmid vector (SEQ ID NO: 330), which is the same as those described in the preceding Example 1 and Example 4, and expression plasmid vectors comprising deaminase-145 mutants (CBE-145-P5, CBE-145-P1, CBE-145-P2, CBE-145-P4, and CBE-145-P6). The sequence of the vector plasmid CBE-145-P5 for deaminase-145-P5 is SEQ ID NO: 331. The Cas protein portion of the editor fusion protein uses nSPRY. The gRNA targets the VEGFA target site, and the spacer is GACCCCCTCCACCCCGCCTC (SEQ ID NO: 332).

### (3) Transfection of 293T Cells with the Vectors to be Verified

The different verification vectors and control vectors were transfected into 293T cells.

Transfection was performed in a 24-well plate in accordance with the Lipofectamine 2000 (Thermo) instructions, and detection was performed after 72 hours.

### (4) Detection of Changes in the VEGFA Target Gene

The cells 72 hours after transfection were used with a genomic DNA extraction kit (Tiangen Biotech) to extract the genome of the cells. The extracted products were subjected to PCR amplification, and the primers are shown as follows: VEGFA-CBE-F3: ACTTGAATCGGGCCGACG (SEQ ID NO: 333); VEGFA-CBE-R3: CCTTGGCATGGTGGAGGTAGAG (SEQ ID NO: 334).

Amplification was performed using 2X Pro Taq Premix Ver.2 (Accurate Biotech). The product length was 866 bp, and the products were sent to Genewiz for sequencing.

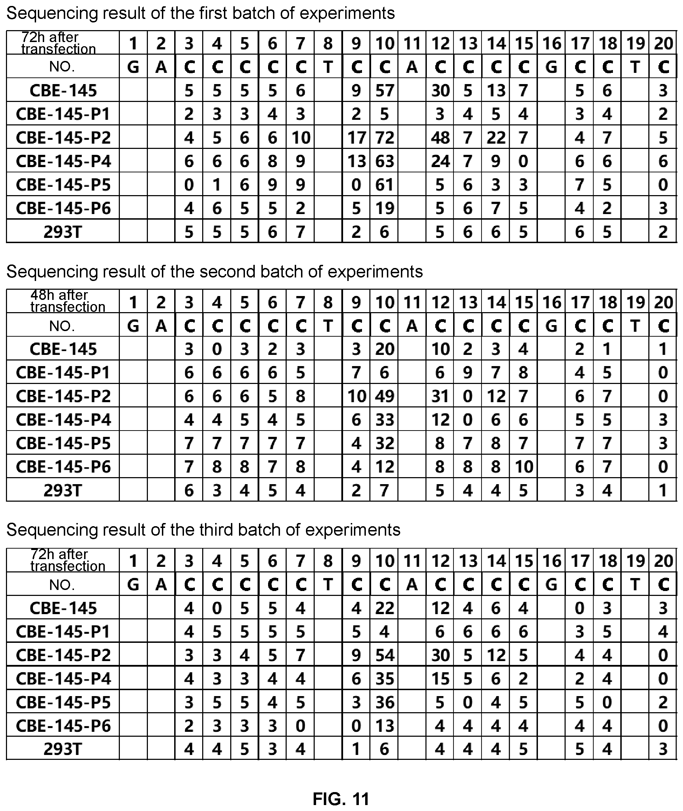

The sequencing results were analyzed. The results of 3 batches are shown in FIG. 11: In the figure, 1-20 represent the nucleotide position numbers of the spacer, and the base C that can be edited by CBE is indicated, and the numbers below the base C represent the percentage of C edited to T (in %). The 293T group serves as an unedited negative control. The present disclosure considers ≥10% as having an editing effect.

In the experiments of 3 batches, the CBE-145 containing wild-type deaminase had an editing effect on the C at positions 10 and 12 (the result of one batch also showed a weak editing effect at the position 14), converting it to T. CBE-145-P5 (Q122H) had an editing effect only at the position 10, with a narrower editing window. Furthermore, in the results of 3 batches, the editing effect of CBE-145-P5 at the position 10 was superior to that of the CBE-145 containing wild-type deaminase. Therefore, the efficiency and specificity of deaminase-145-P5 were both superior to those of deaminase-145.

In the results of 3 batches, the editing effects of CBE-145-P2 and CBE-145-P4 at the position 10 were both superior to that of the CBE-145 containing wild-type deaminase. Therefore, the efficiencies of deaminase-145-P2 and -P4 were both superior to that of deaminase-145.

### Experimental Example 6. Verification of More Mutants of Deaminase-145

### (1) Screening for highly efficient and highly specific CBE-145 mutants

Phages expressing deaminase-145 were prepared in a manner of the literature (Neugebauer M E, Hsu A, Arbab M, et al. Evolution of an adenine base editor into a small, efficient cytosine base editor with low off-target activity[J]. Nature Biotechnology, 2023, 41(5): 673-685), and the deaminase-145 was evolved by the PANCE method using the screening system and strains of the literature, thereby ultimately screening out the deaminase-145 mutants: deaminase-145-P5 (Q122H); deaminase-145-P1 (S191R); deaminase-145-P2 (I114T); deaminase-145-P3 (C176F); deaminase-145-P4 (L2M); deaminase-145-P6 (N89D); deaminase-145-P7 (N68I); deaminase-145-P8 (Q78*, i.e., the codon of the 78th amino acid Q was mutated into a stop codon); and deaminase-145-P11 (N68I and I114T mutations).

### (2) Construction of vector plasmids for verification

Verification vectors were obtained by synthesis from an outsourced service company. These include the CBE-145 expression plasmid vector (SEQ ID NO: 330), which is the same as those described in the preceding Example 1 and Example 4, and expression plasmid vectors comprising the deaminase-145 mutants (CBE-145-P5, CBE-145-P1, CBE-145-P2, CBE-145-P3, CBE-145-P4, CBE-145-P6, CBE-145-P7, CBE-145-P8, and CBE-145-P11). The sequence of the vector plasmid CBE-145-P5 for the deaminase-145-P5 is SEQ ID NO: 331. The Cas protein portion of the editor fusion protein uses nSPRY. The Grna targets the VEGFA target site, and the spacer is GACCCCCTCCACCCCGCCTC (SEQ ID NO: 332).

### (3) Transfection of 293T cells with the vectors to be verified

The different verification vectors and control vectors were transfected into the 293T cells.

Transfection was performed in a 24-well plate in accordance with the Lipofectamine 2000 (Thermo) instructions, and detection was performed after 72 hours.

### (4) Determination of changes in the VEGFA target gene

The cells 72 hours after transfection were used with a genomic DNA extraction kit to extract the genome of the cells. The extracted products were subjected to PCR amplification, and the primers are shown as follows: VEGFA-CBE-F3: ACTTGAATCGGGCCGACG (SEQ ID NO: 333); VEGFA-CBE-R3: CCTTGGCATGGTGGAGGTAGAG (SEQ ID NO: 334).

Amplification was performed using 2X Pro Taq Premix Ver.2 (Accurate Biology), the product length was 866 bp, and the products were sent to GeneWiz for sequencing.

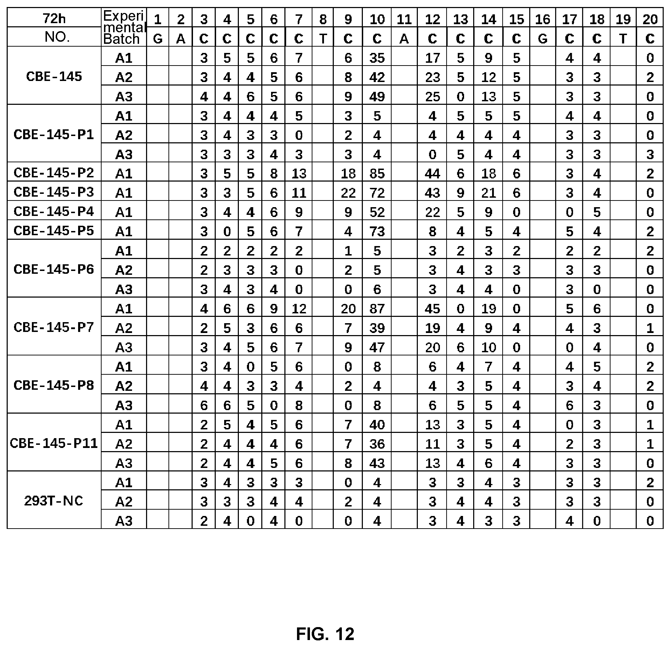

The sequencing results were analyzed. The results of 3 batches are shown in FIG. 12: In the figure, 1 to 20 represent the nucleotide position numbers of the spacer, and the numbers below the base represent the percentage of C edited to T (in %). The 293T group serves as an unedited negative control. The present disclosure considers ≥10% as having an editing effect.

The results of FIG. 12 show that the editing effects of CBE-145-P2, CBE-145-P3, CBE-145-P4, and CBE-145-P5 at the position 10 are all superior to that of the CBE-145 containing the wild-type deaminase. Therefore, the efficiencies of the deaminase-145-P2/P3/P4/P5 variants are all superior to that of the deaminase-145.

The deaminase-145-P1, deaminase-145-P6, and deaminase-145-P8 mutants almost completely lost their activity.

The deaminase-145-P7 and deaminase-145-P11 mutants still have relatively strong activity. The deaminase-145-P11 mutant has editing specificity at the position 10 that is superior to the wild type.

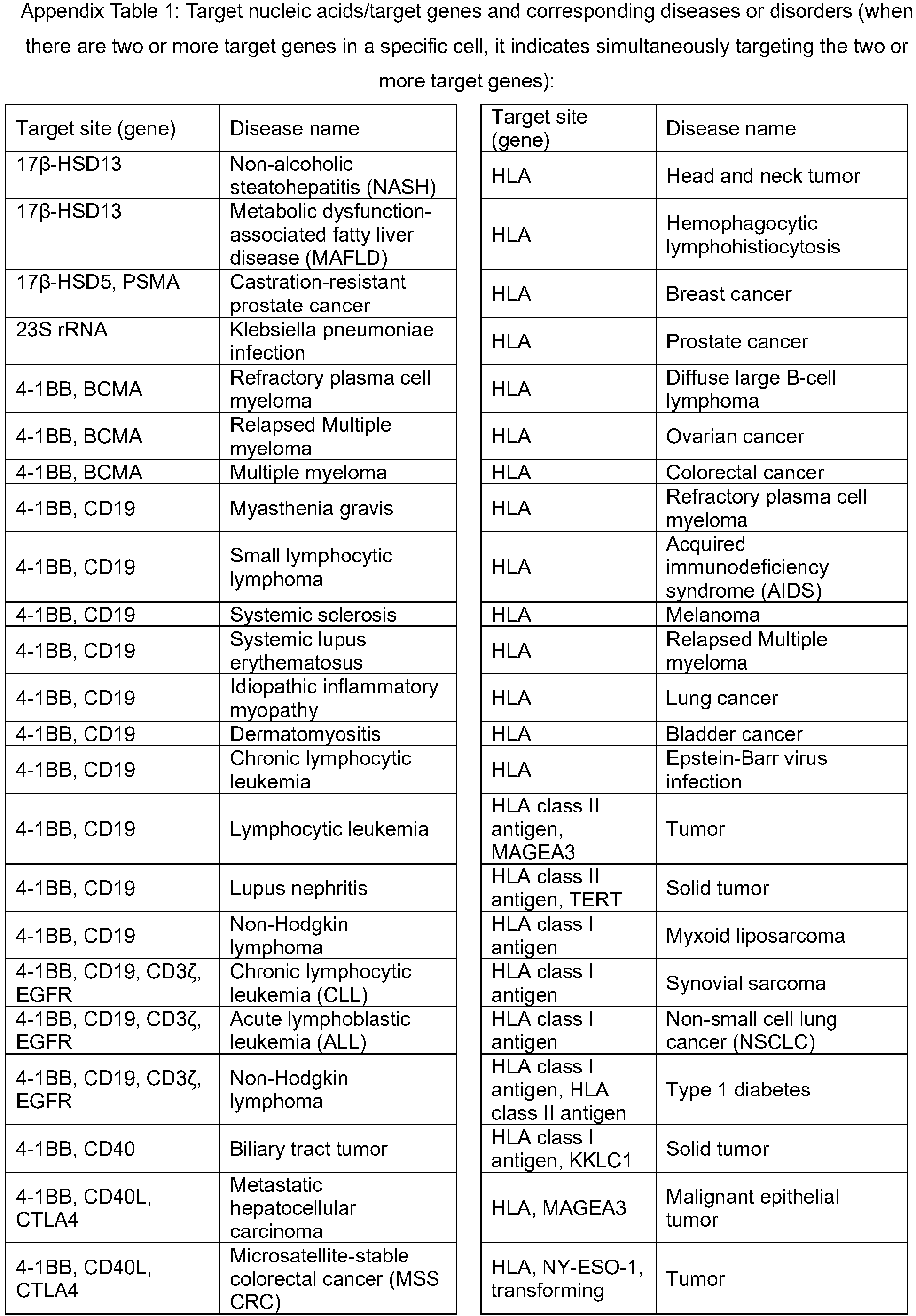

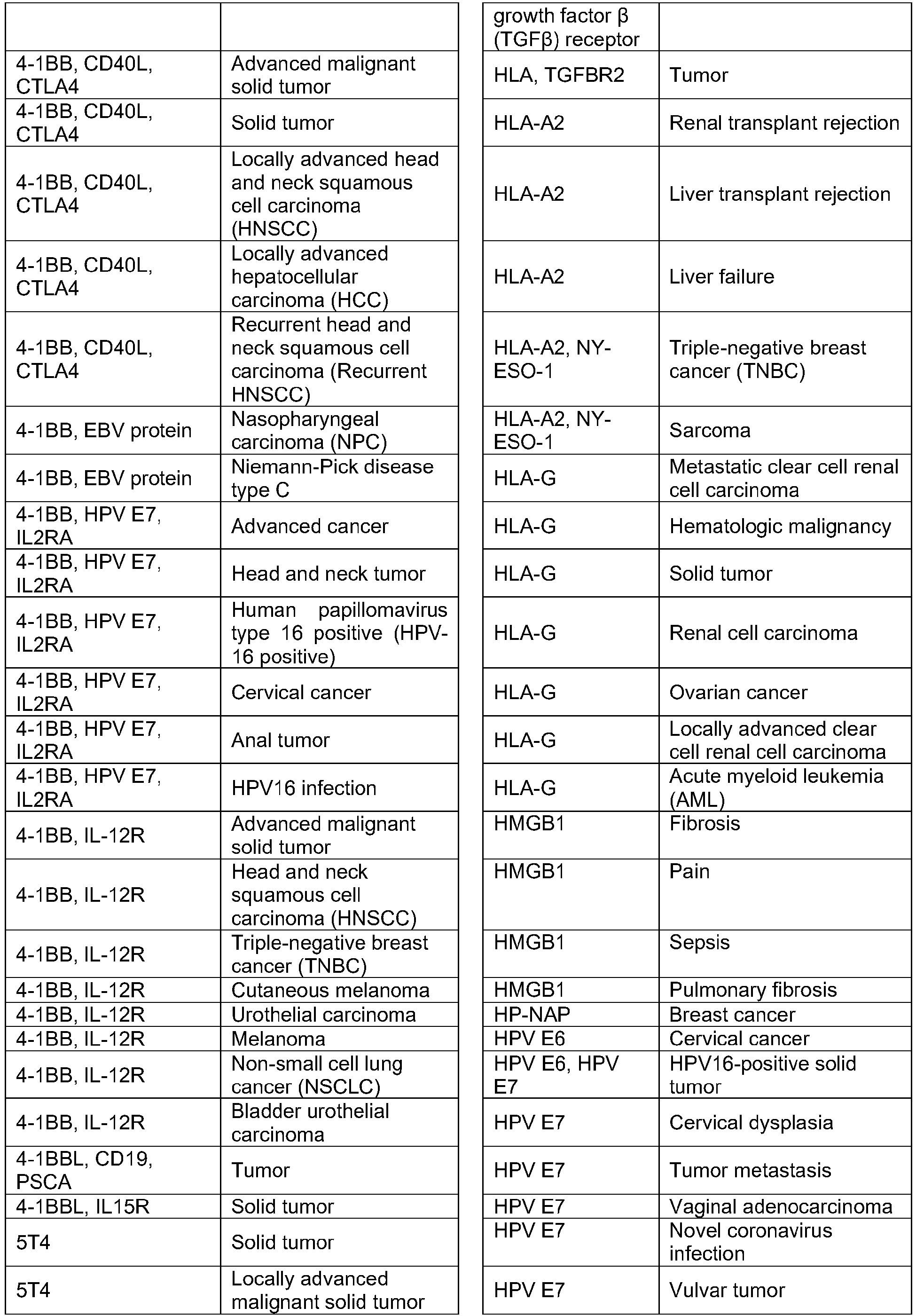

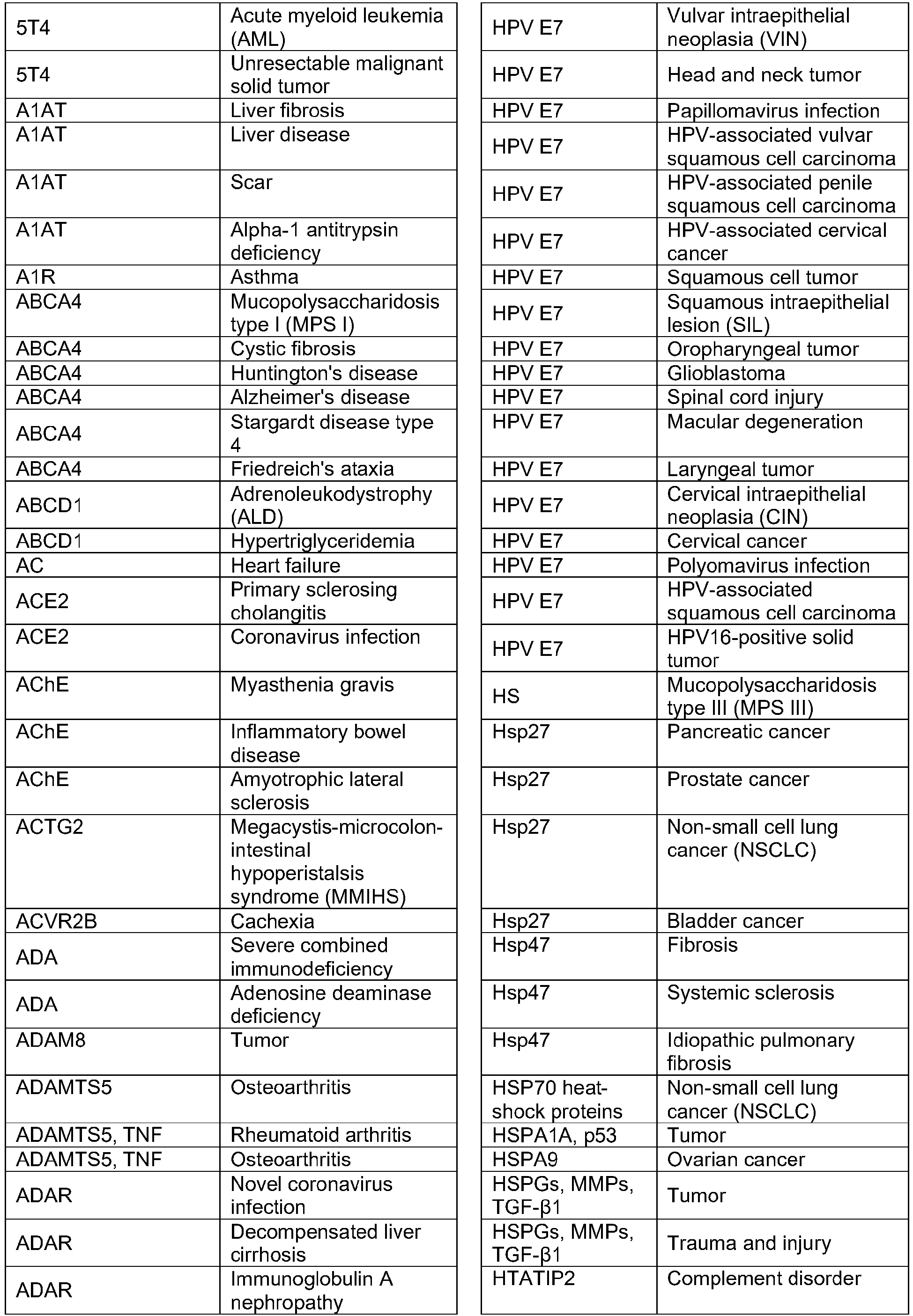

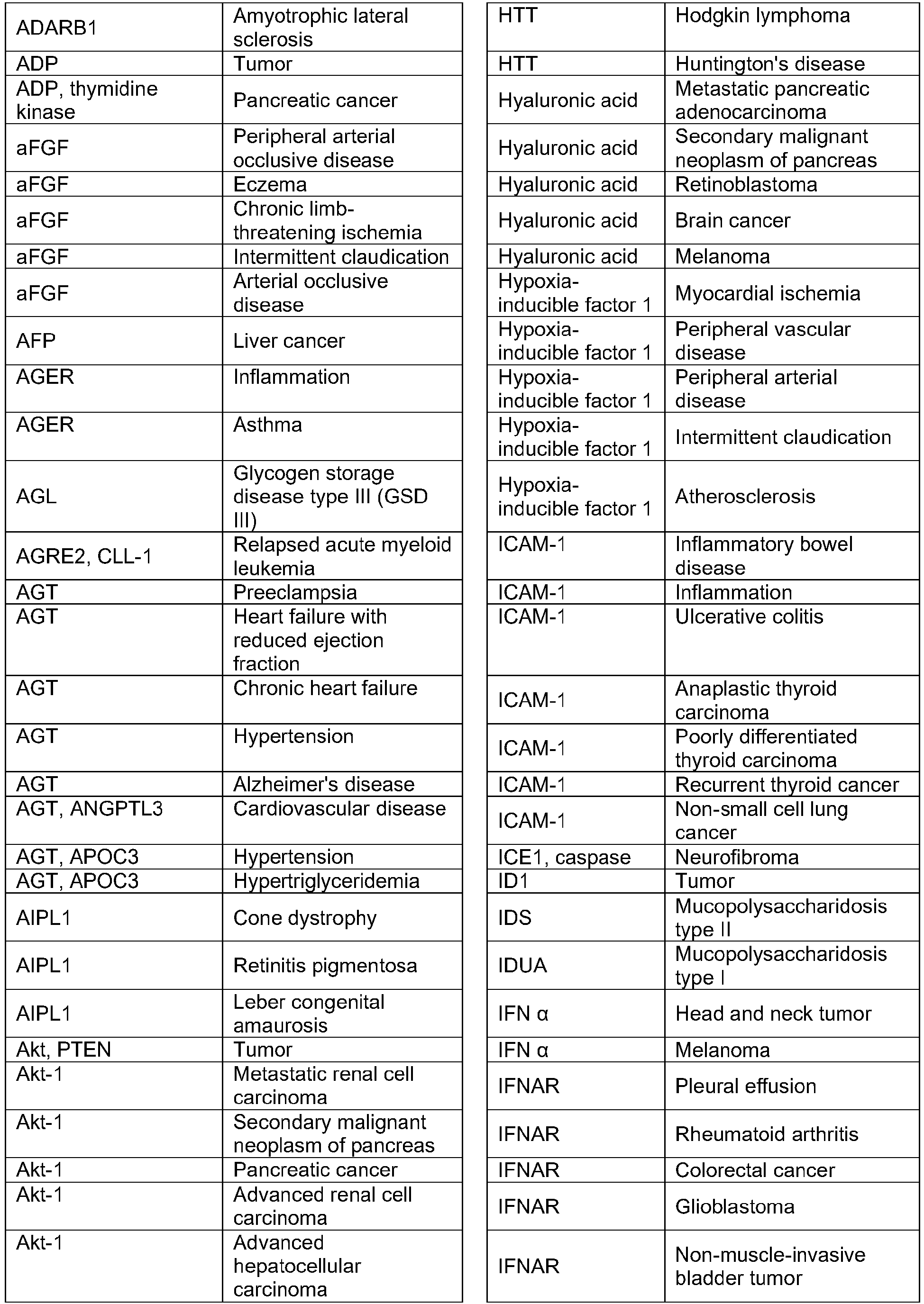

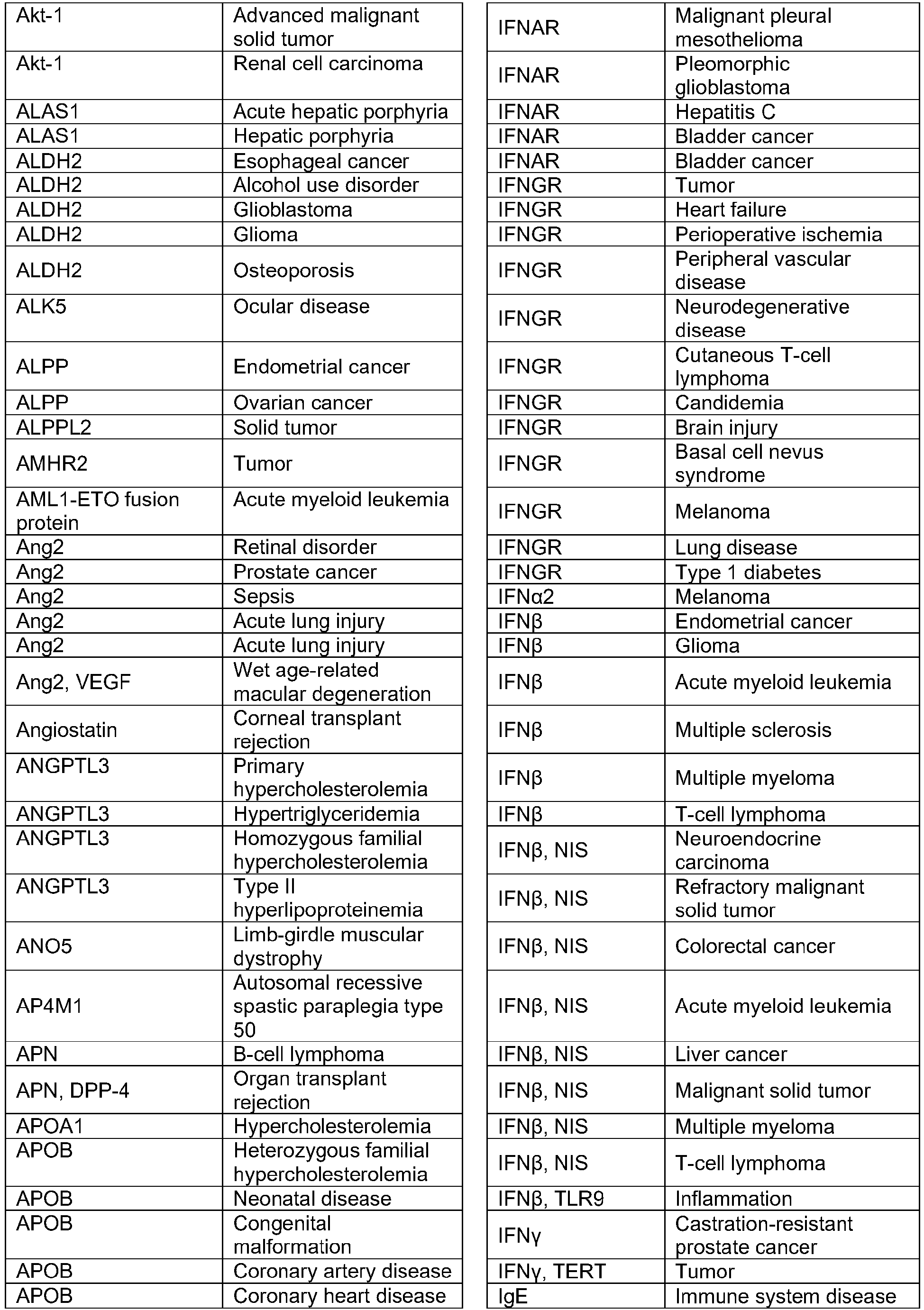

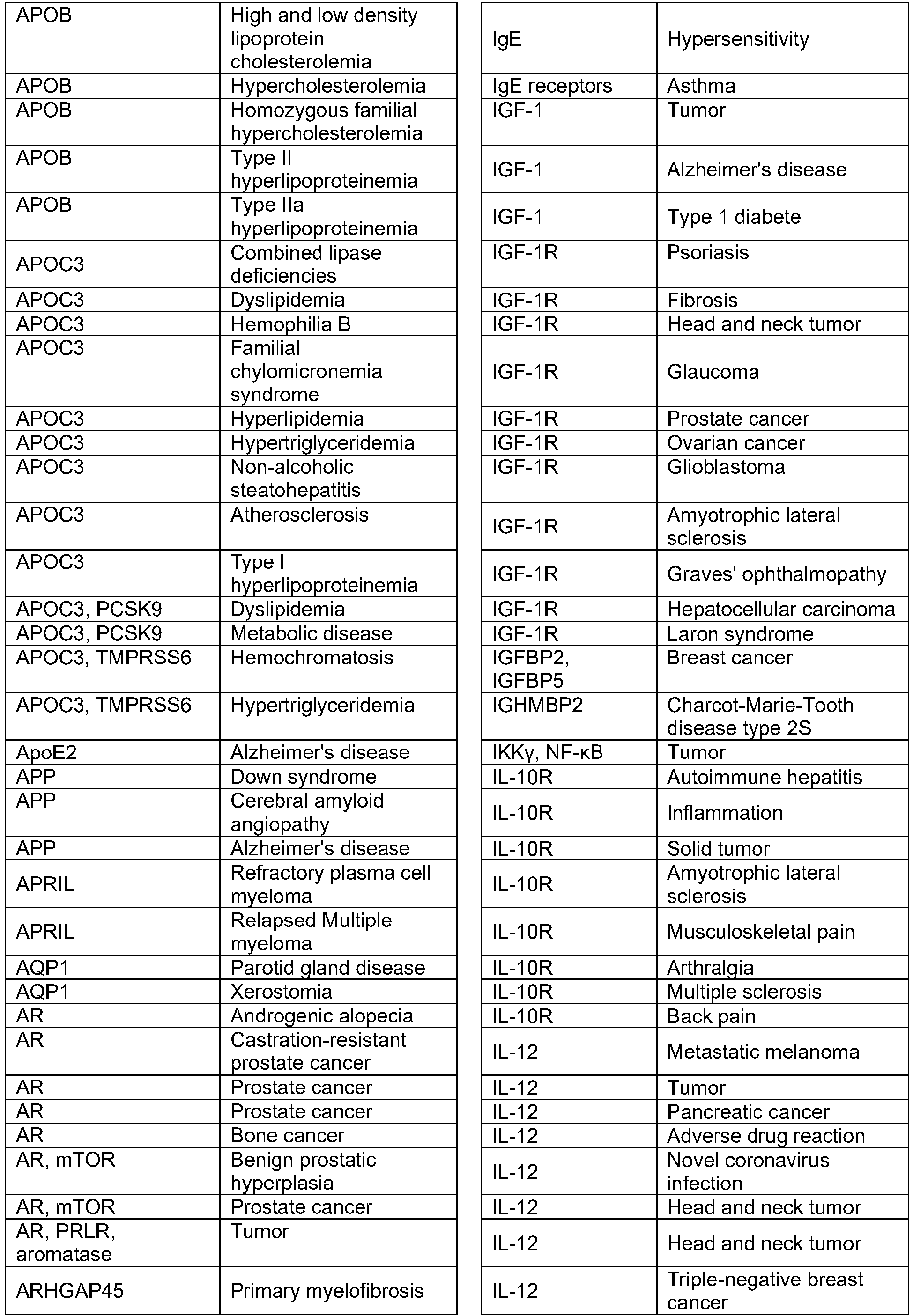

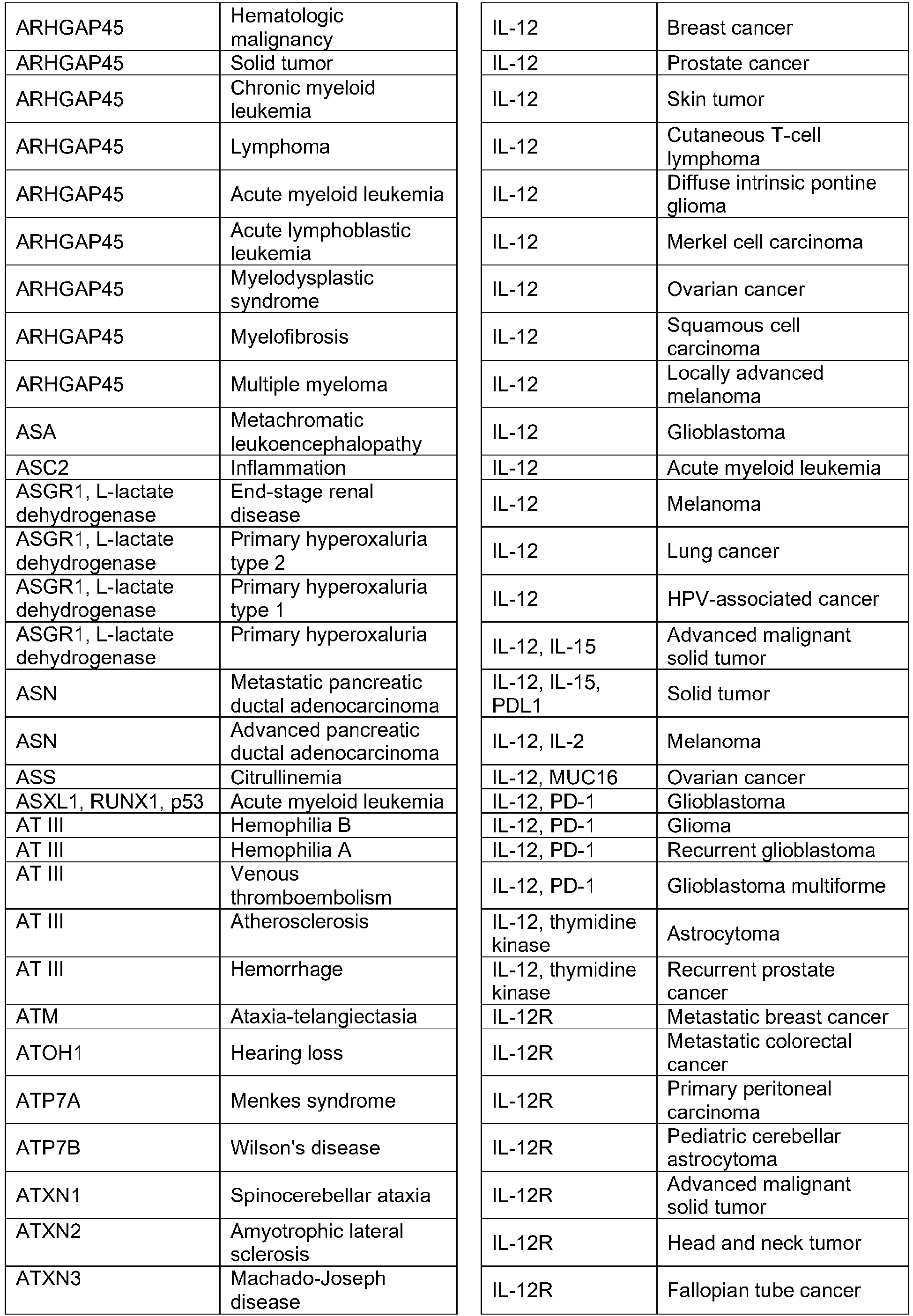

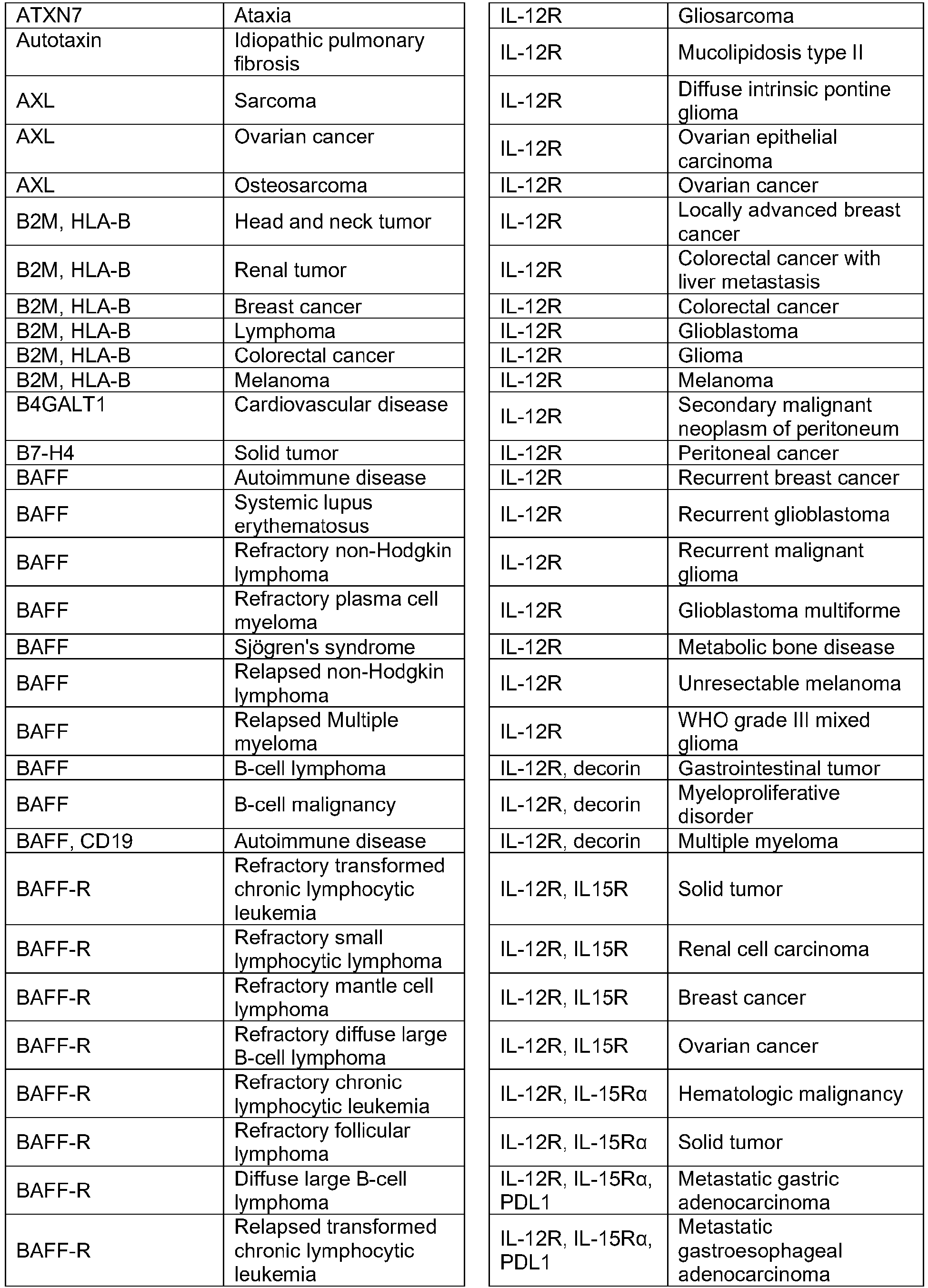

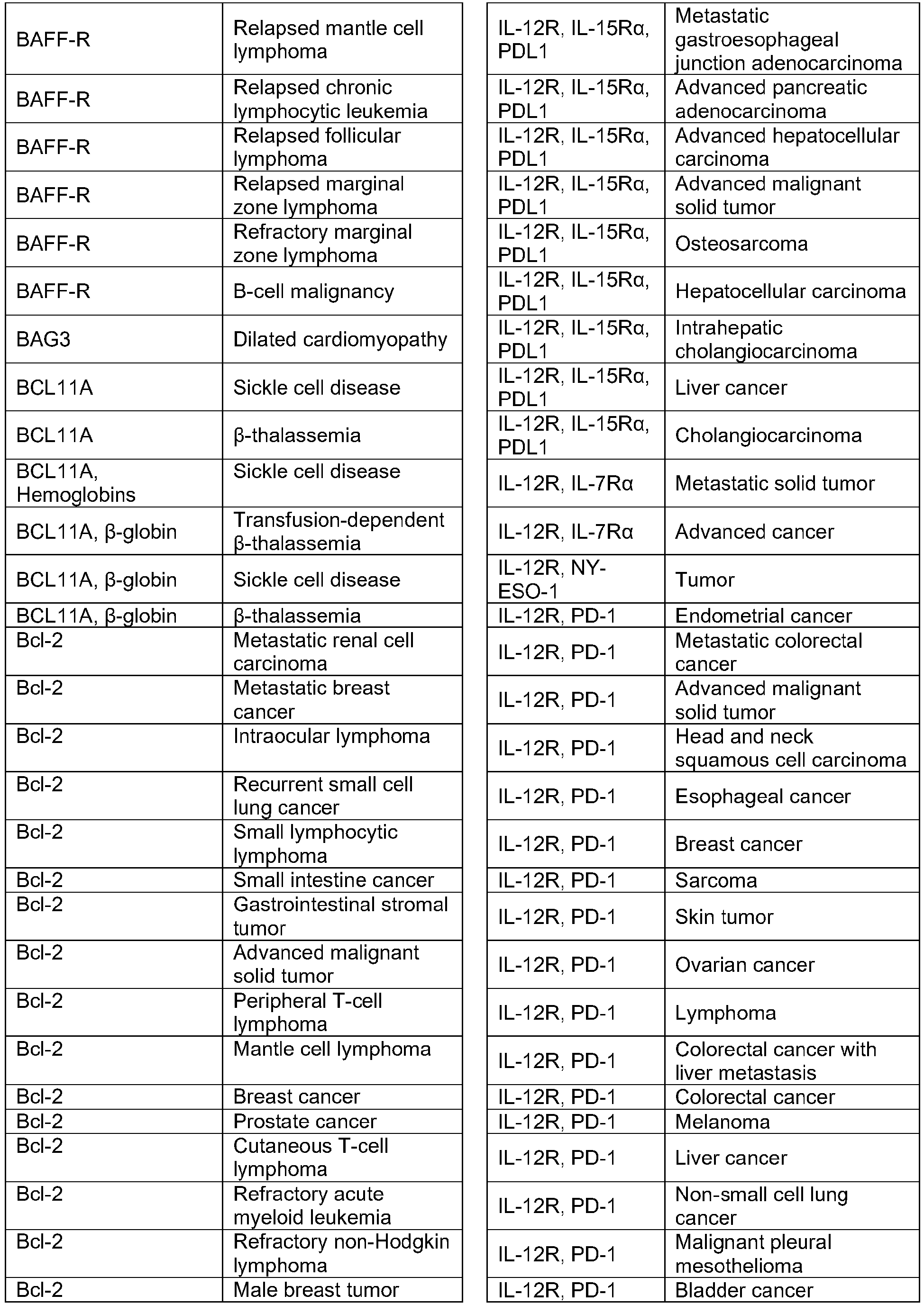

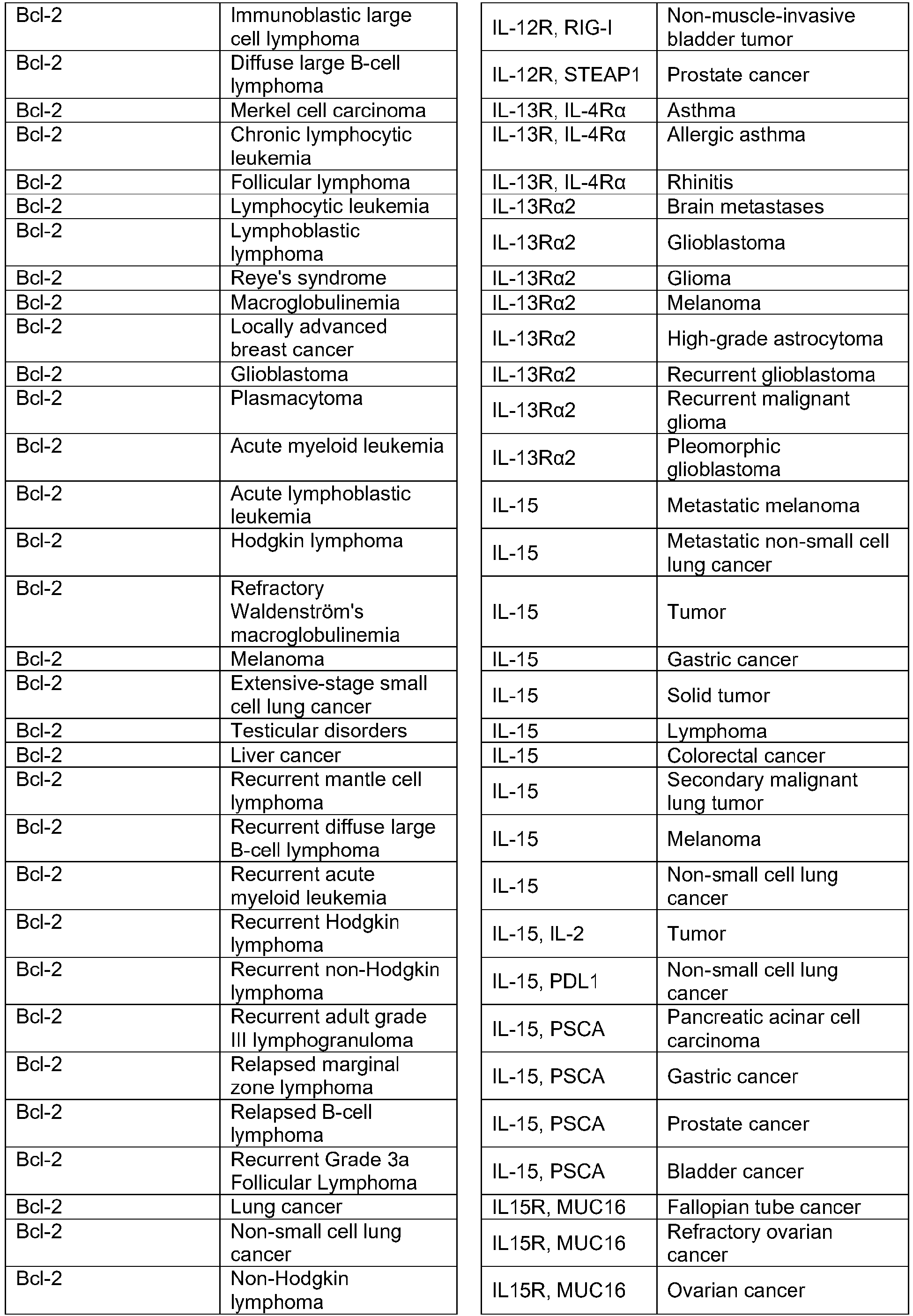

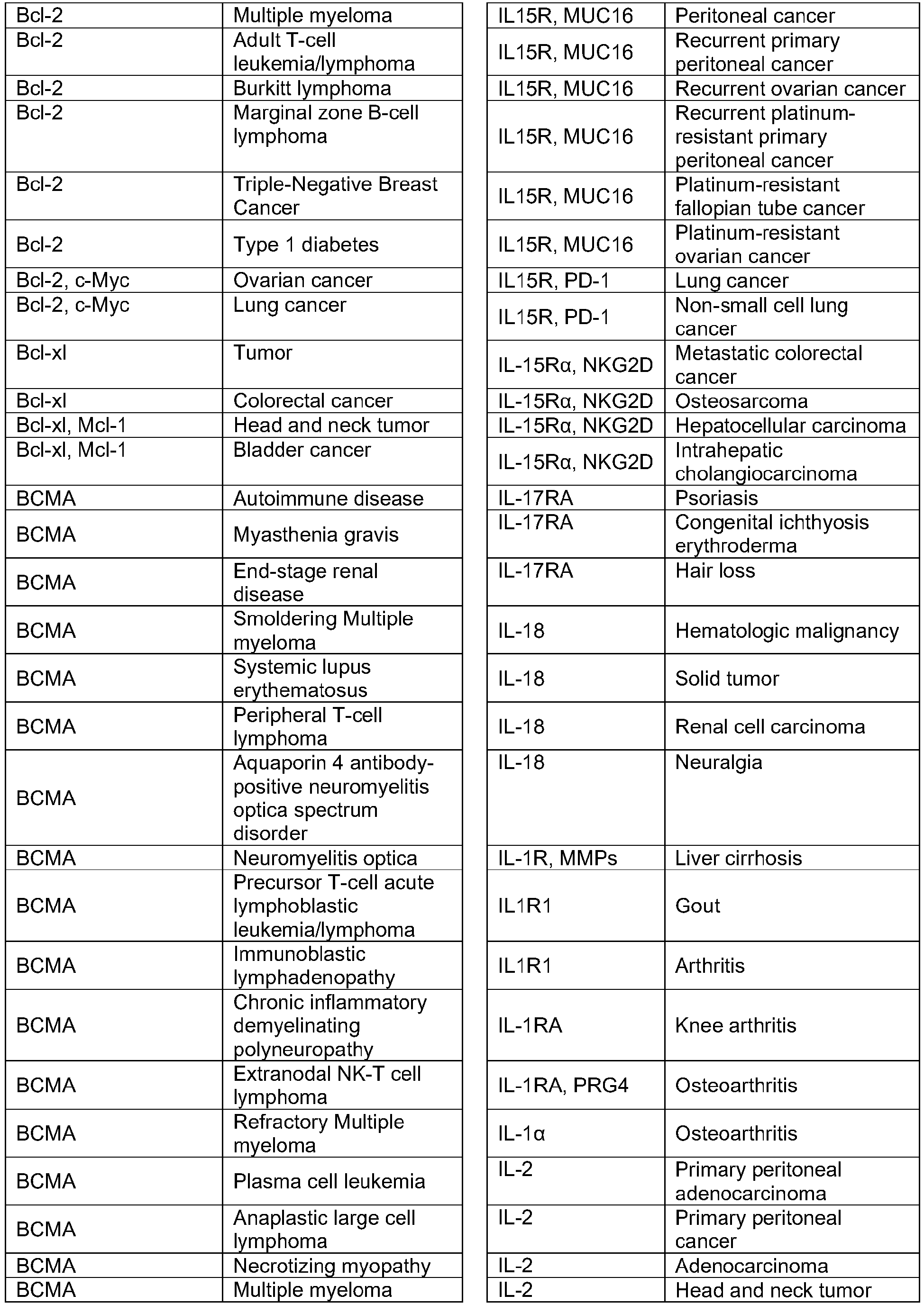

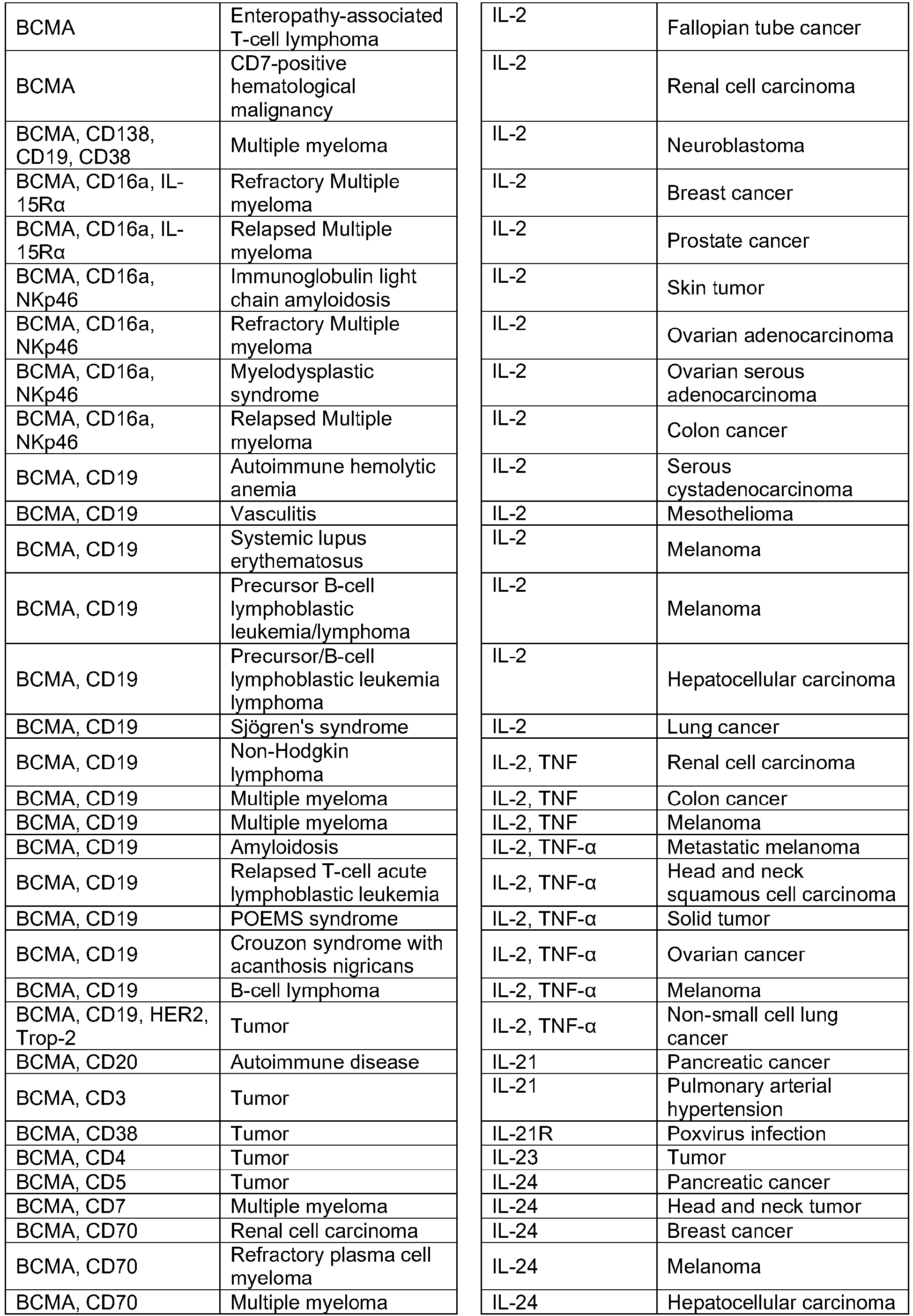

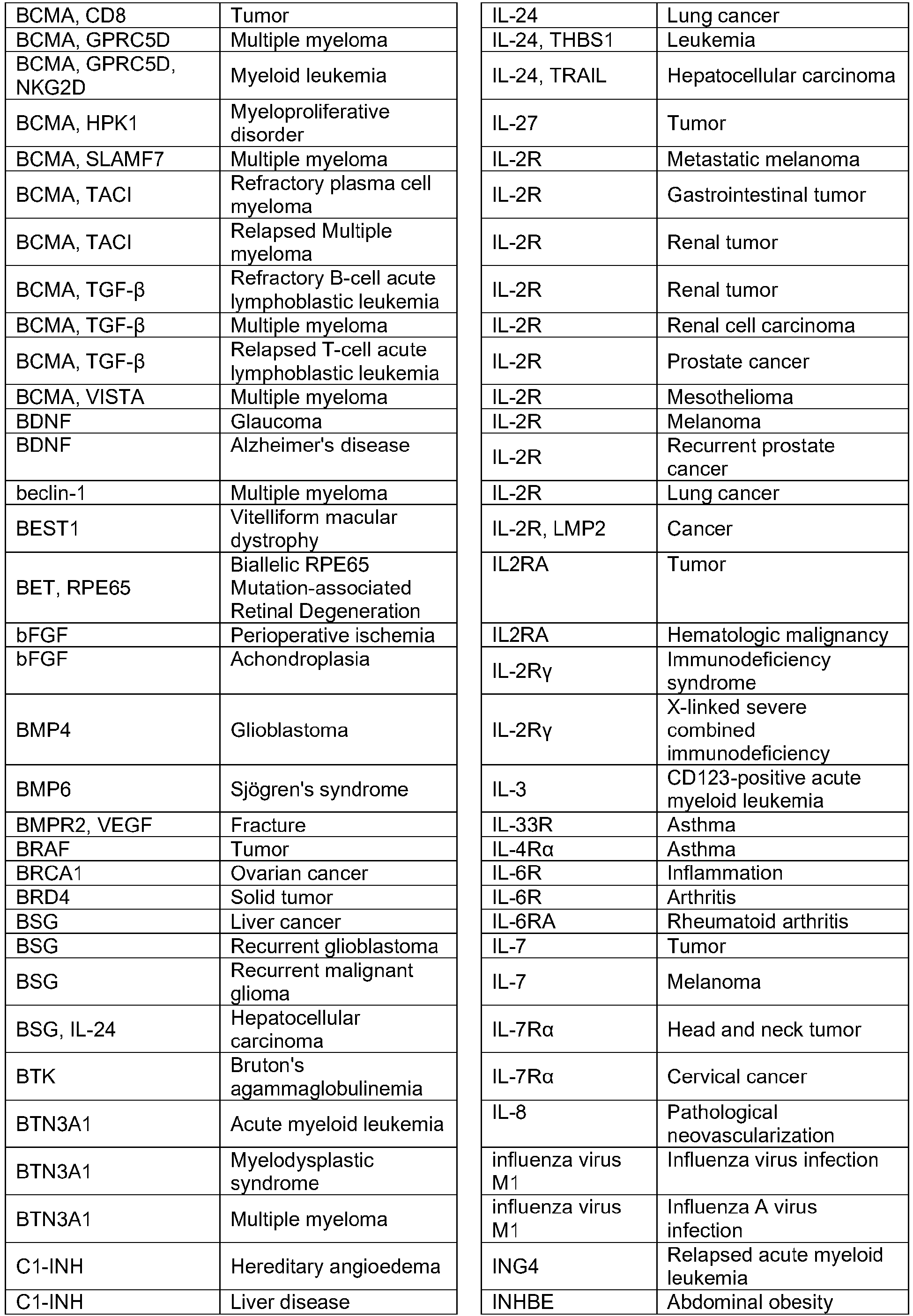

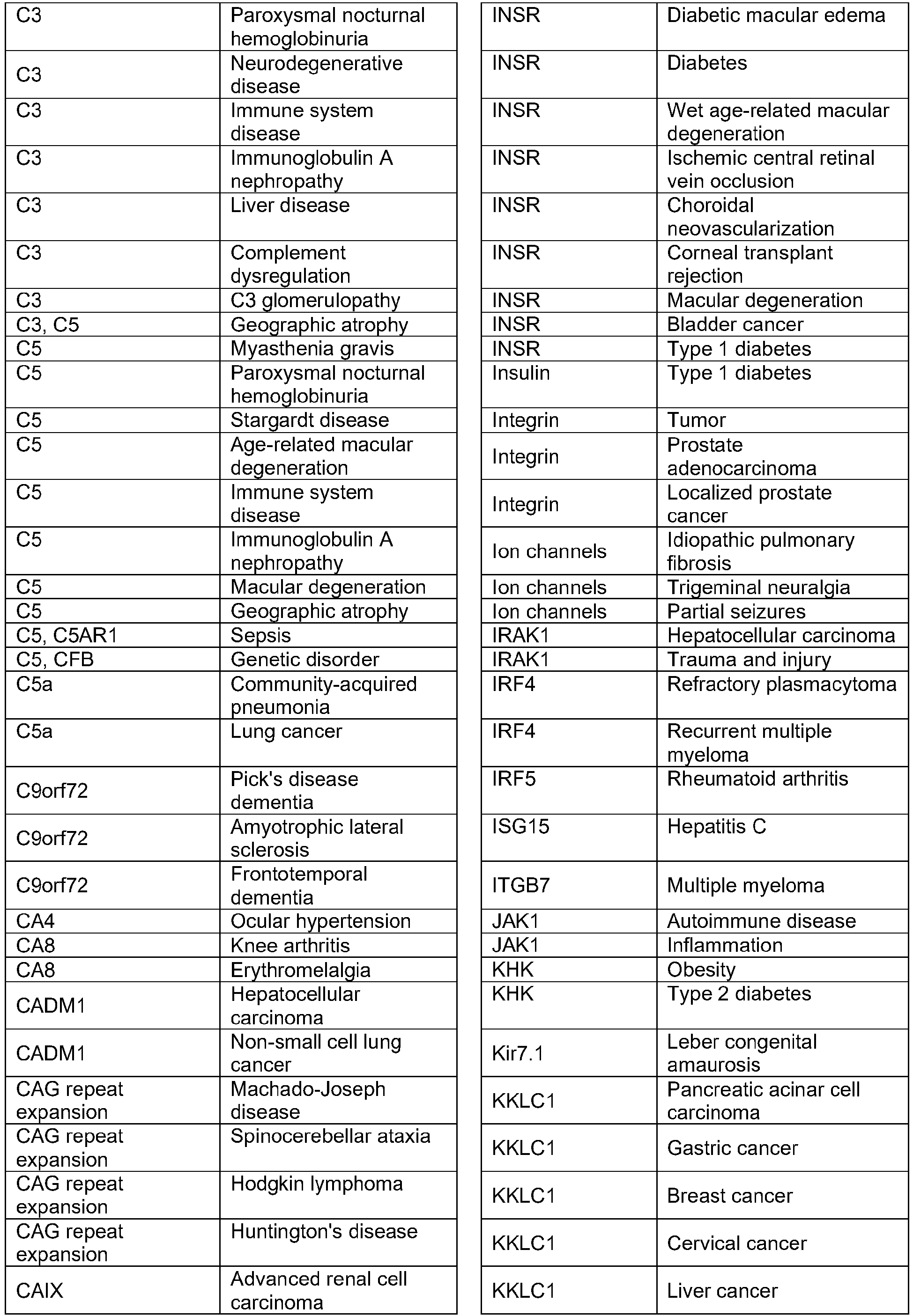

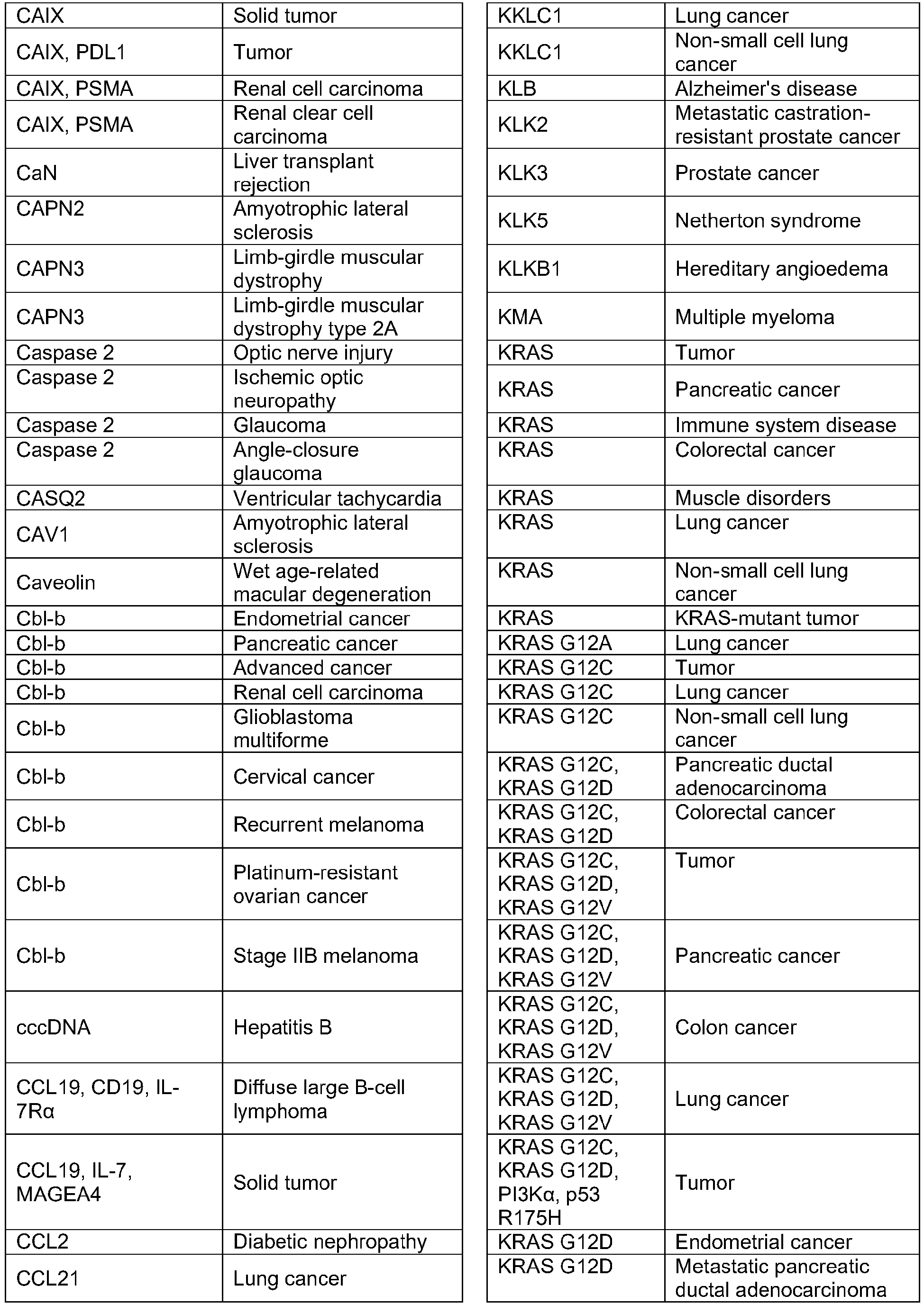

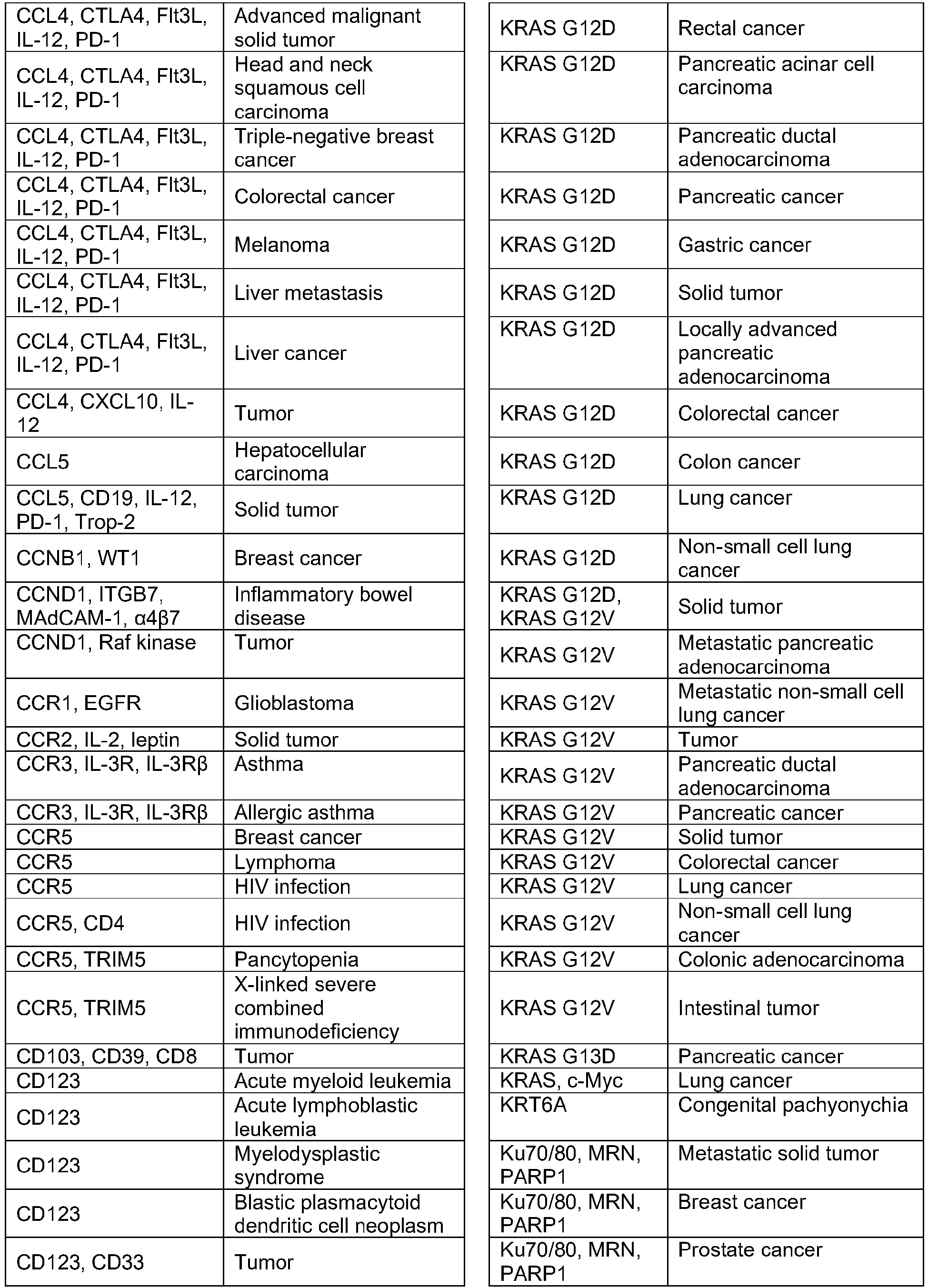

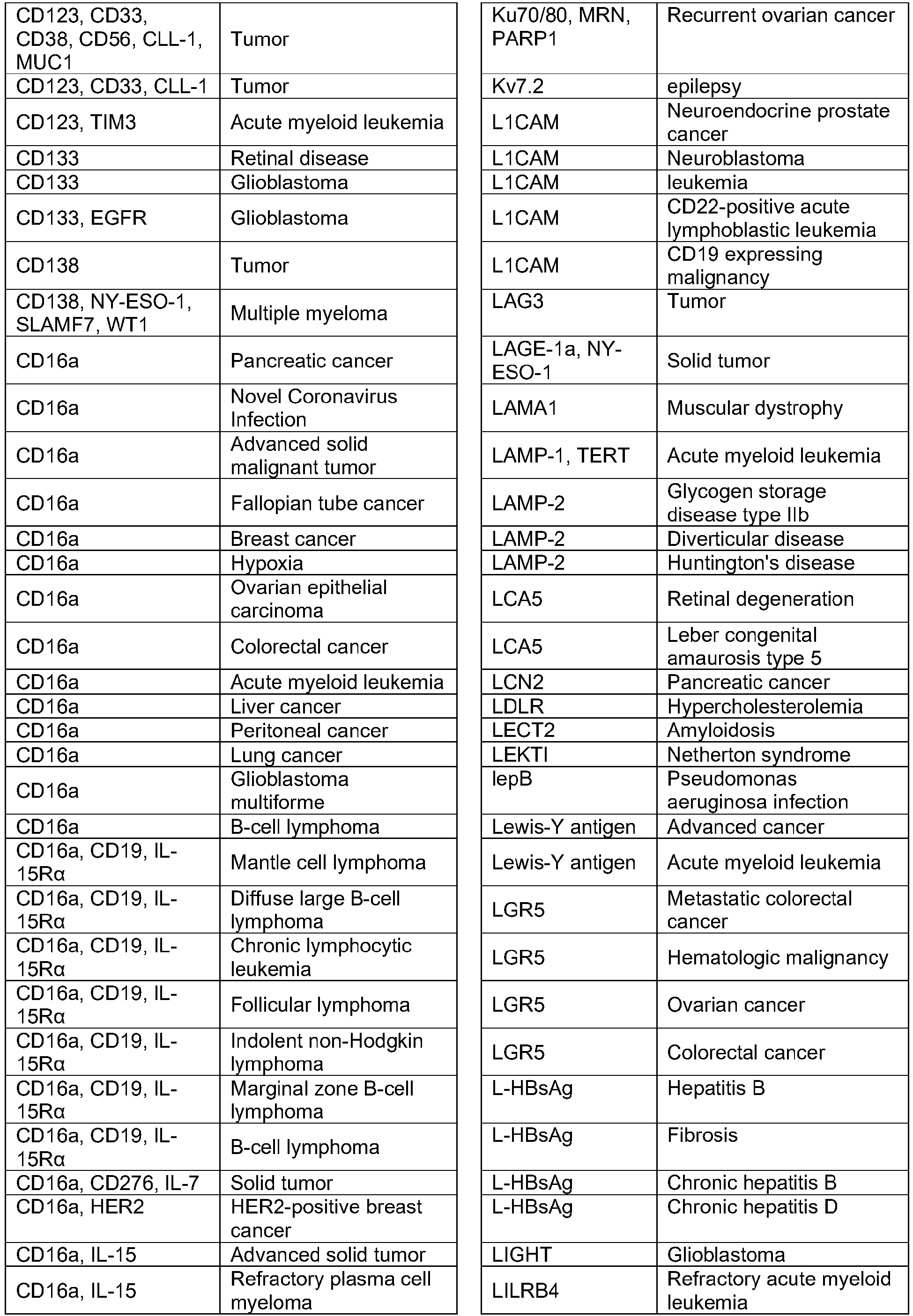

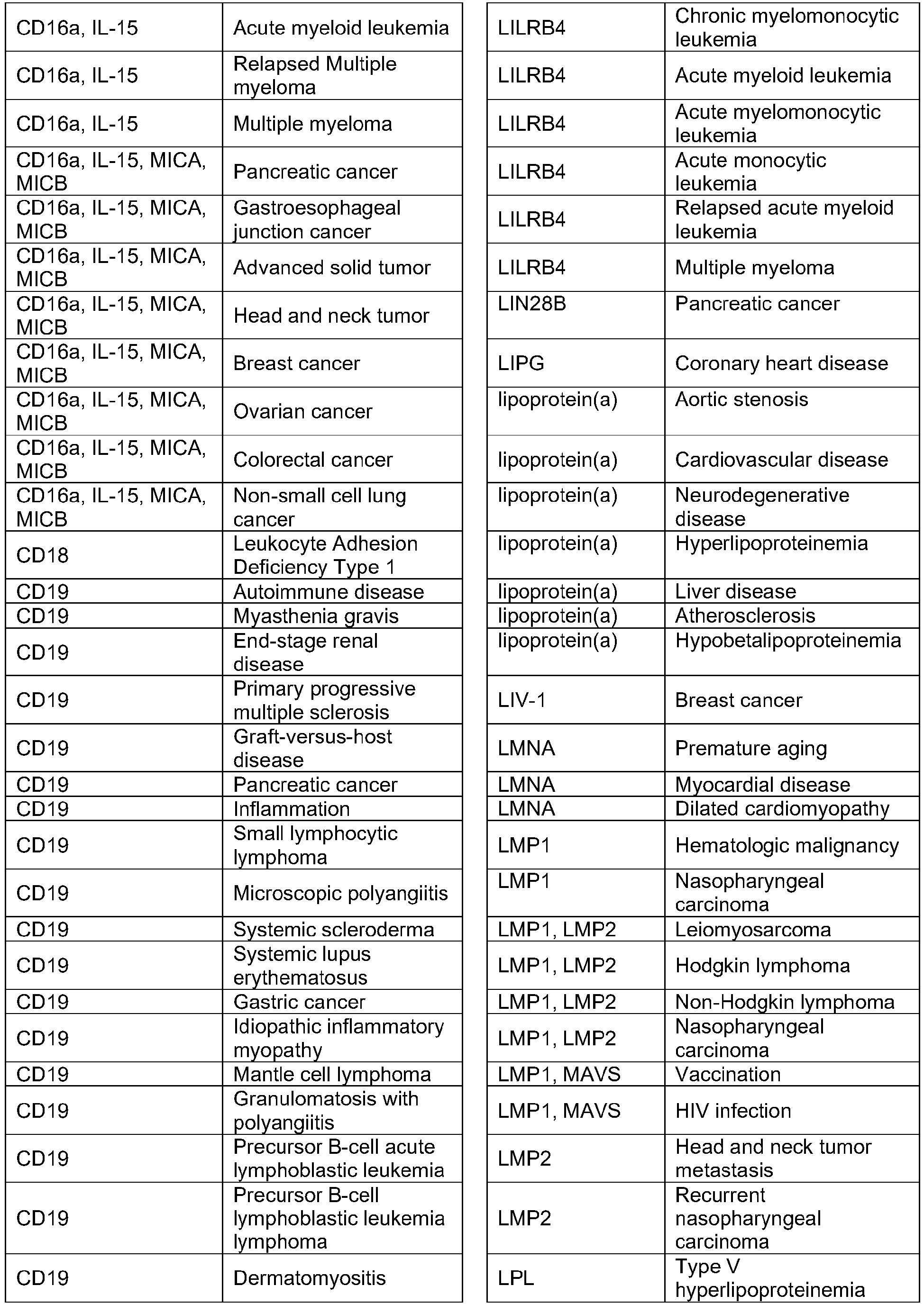

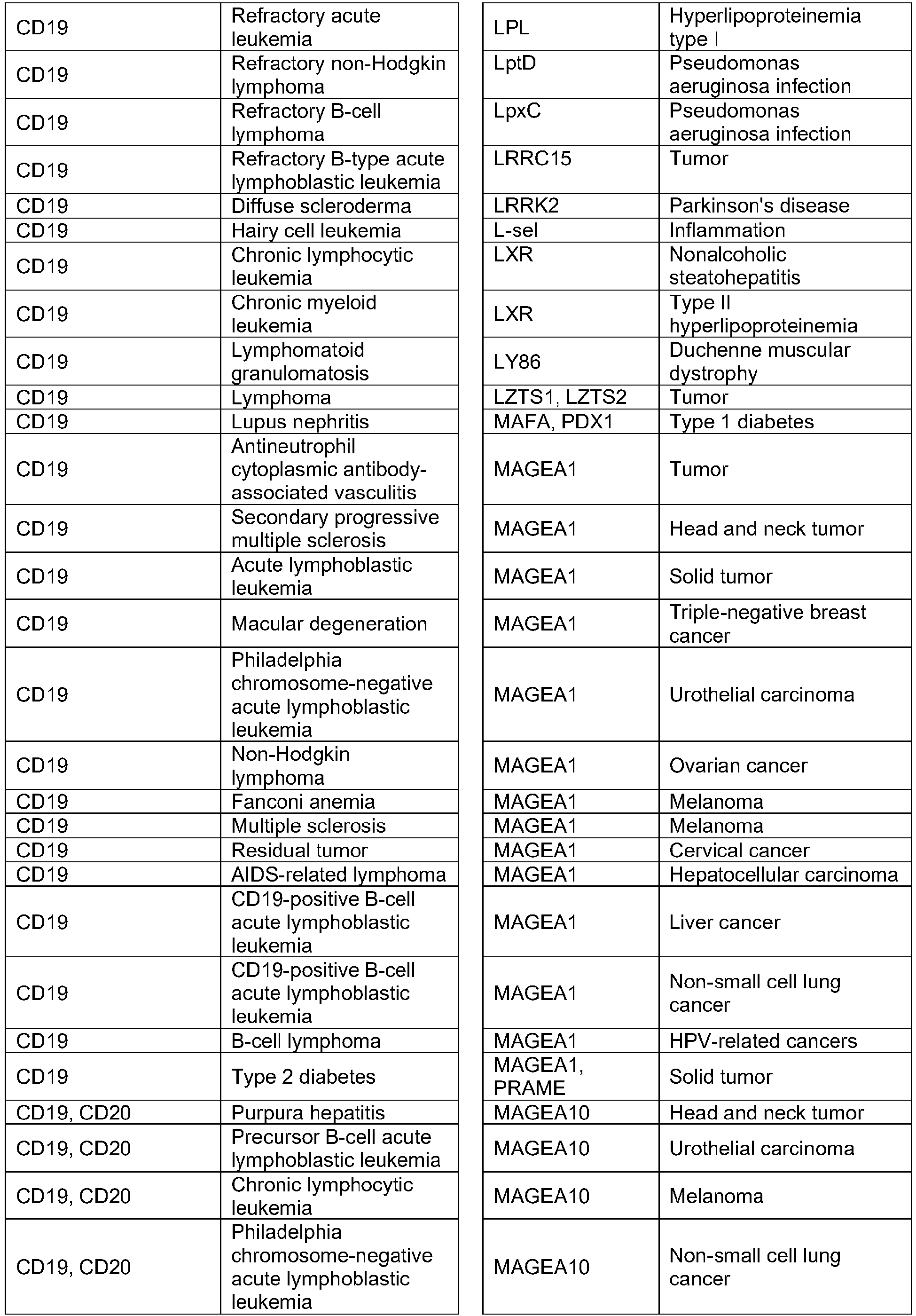

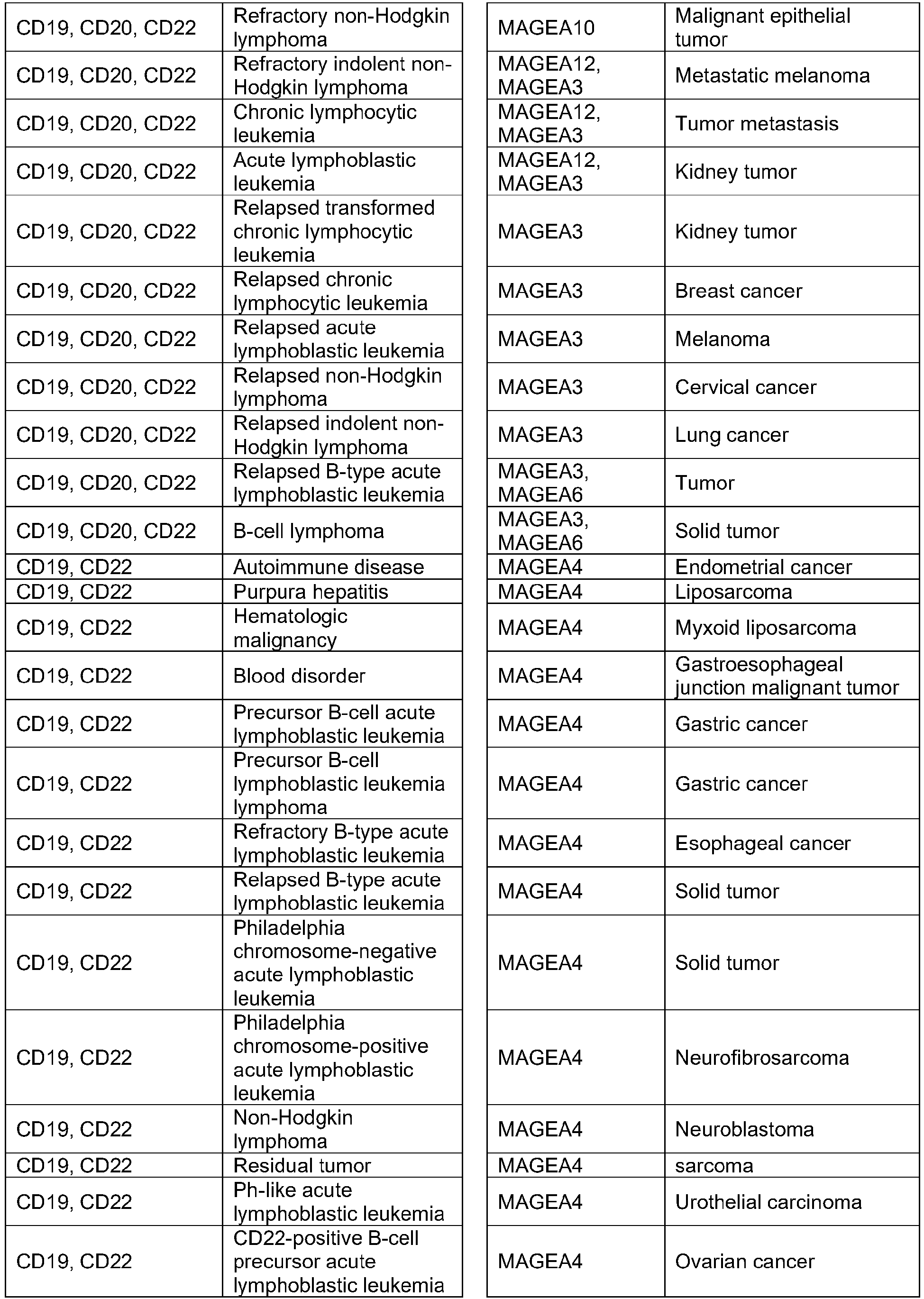

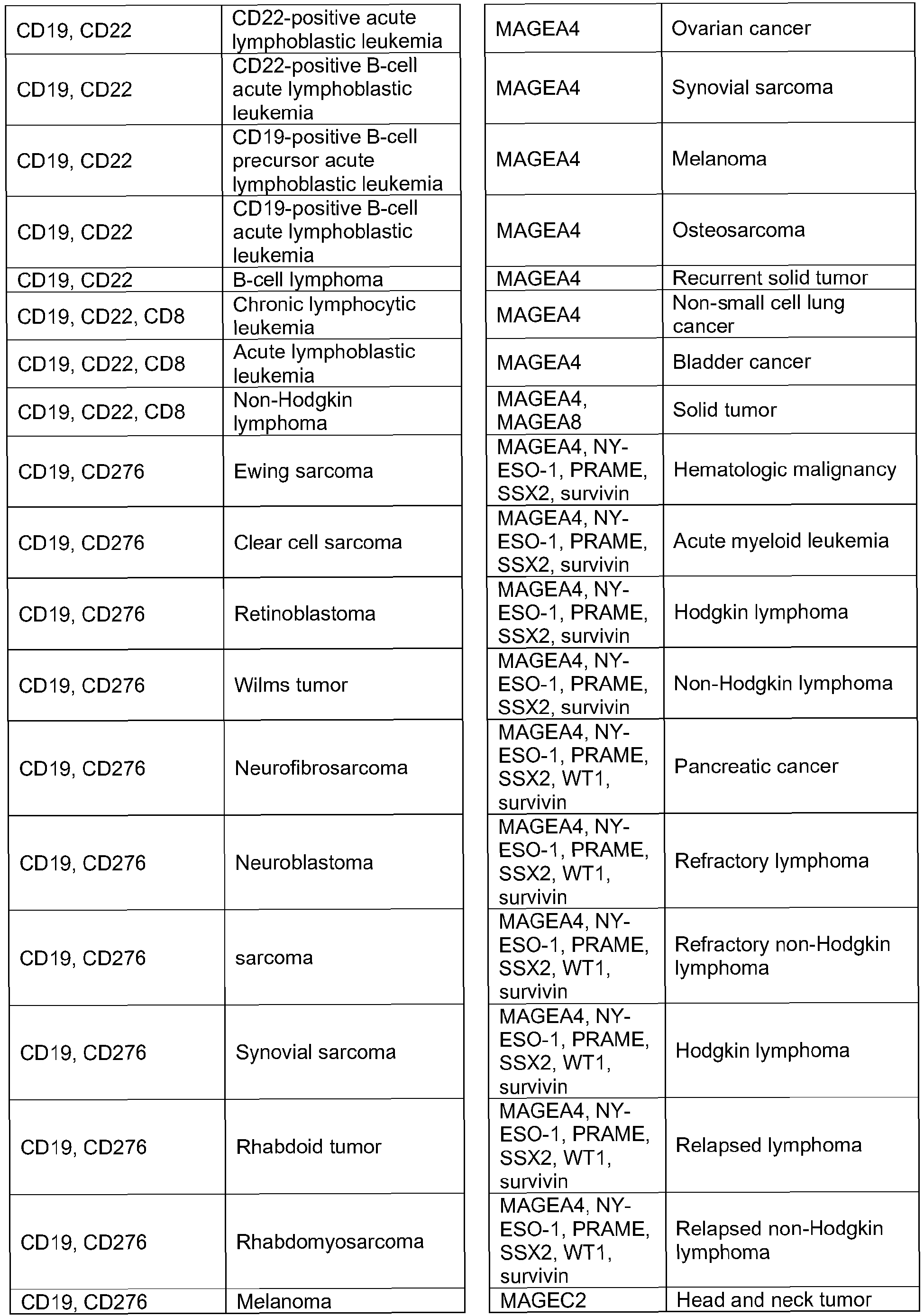

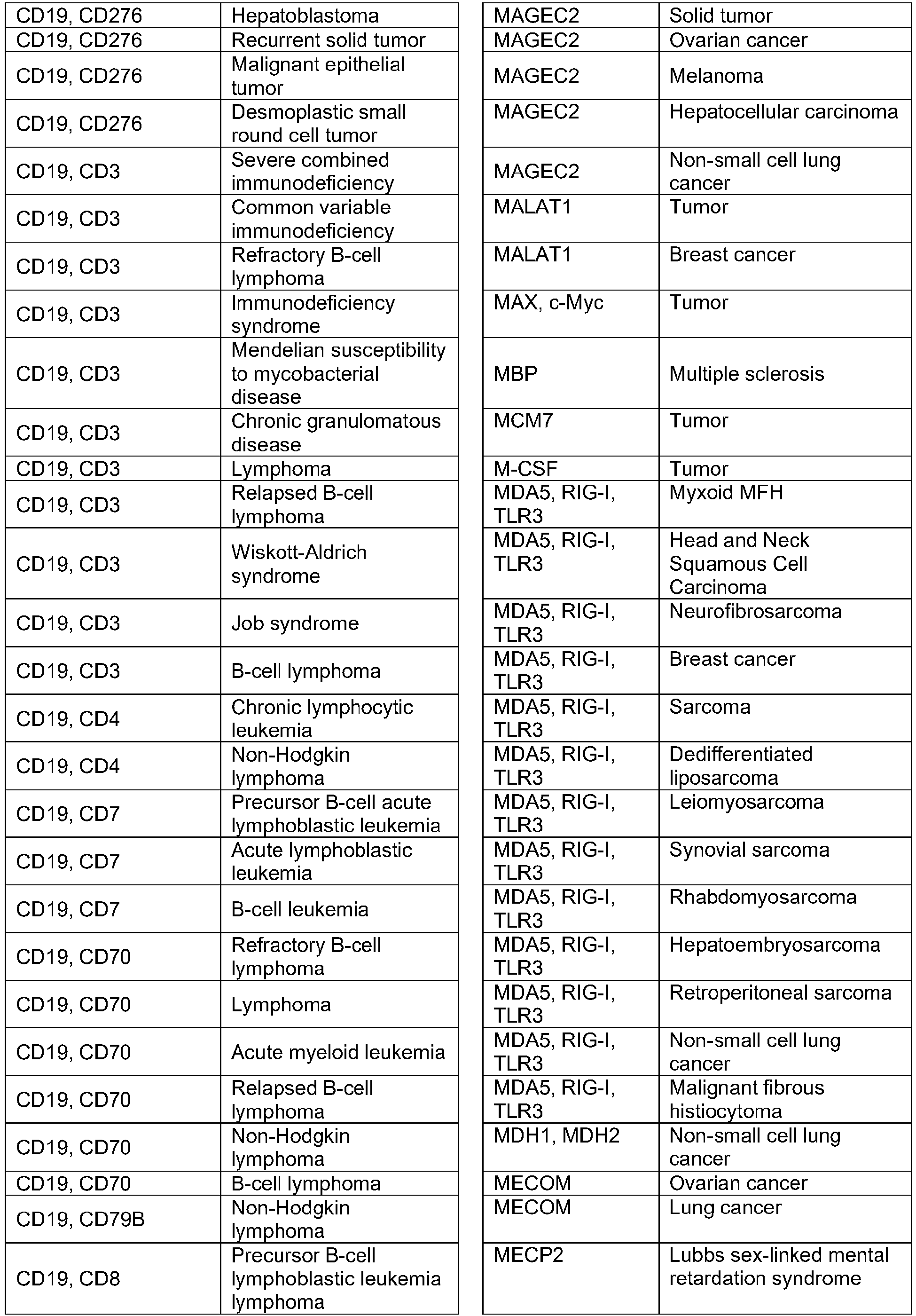

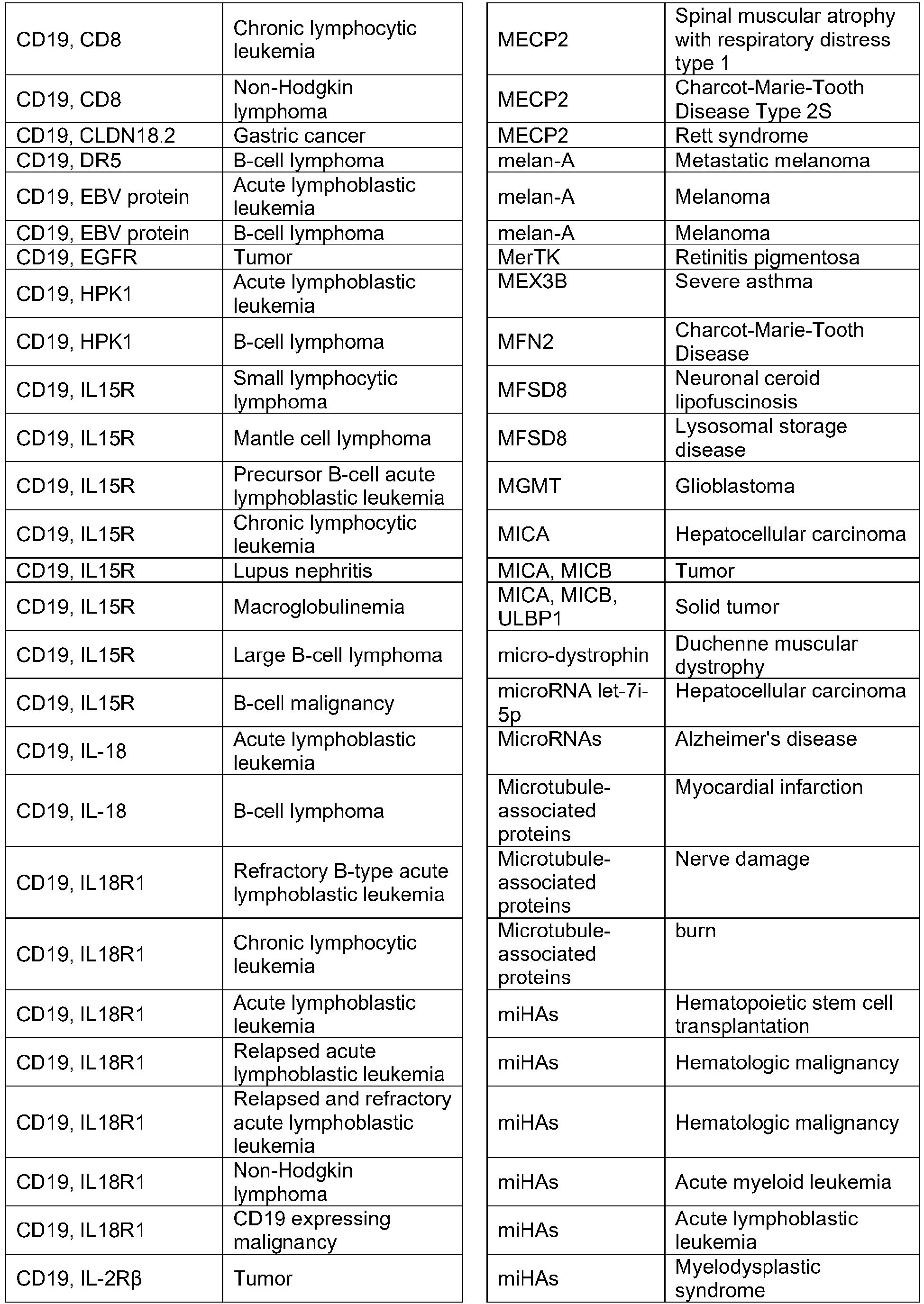

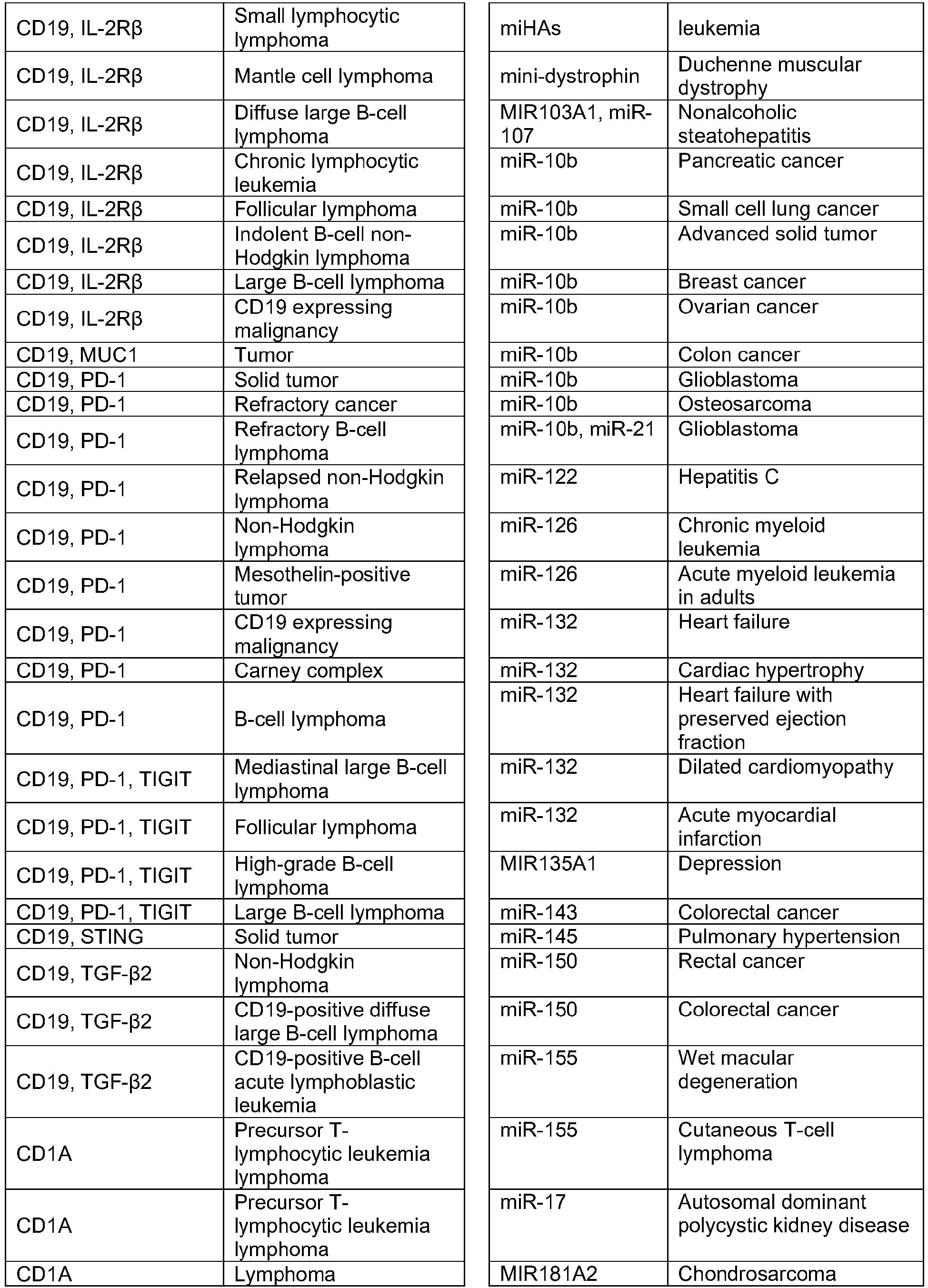

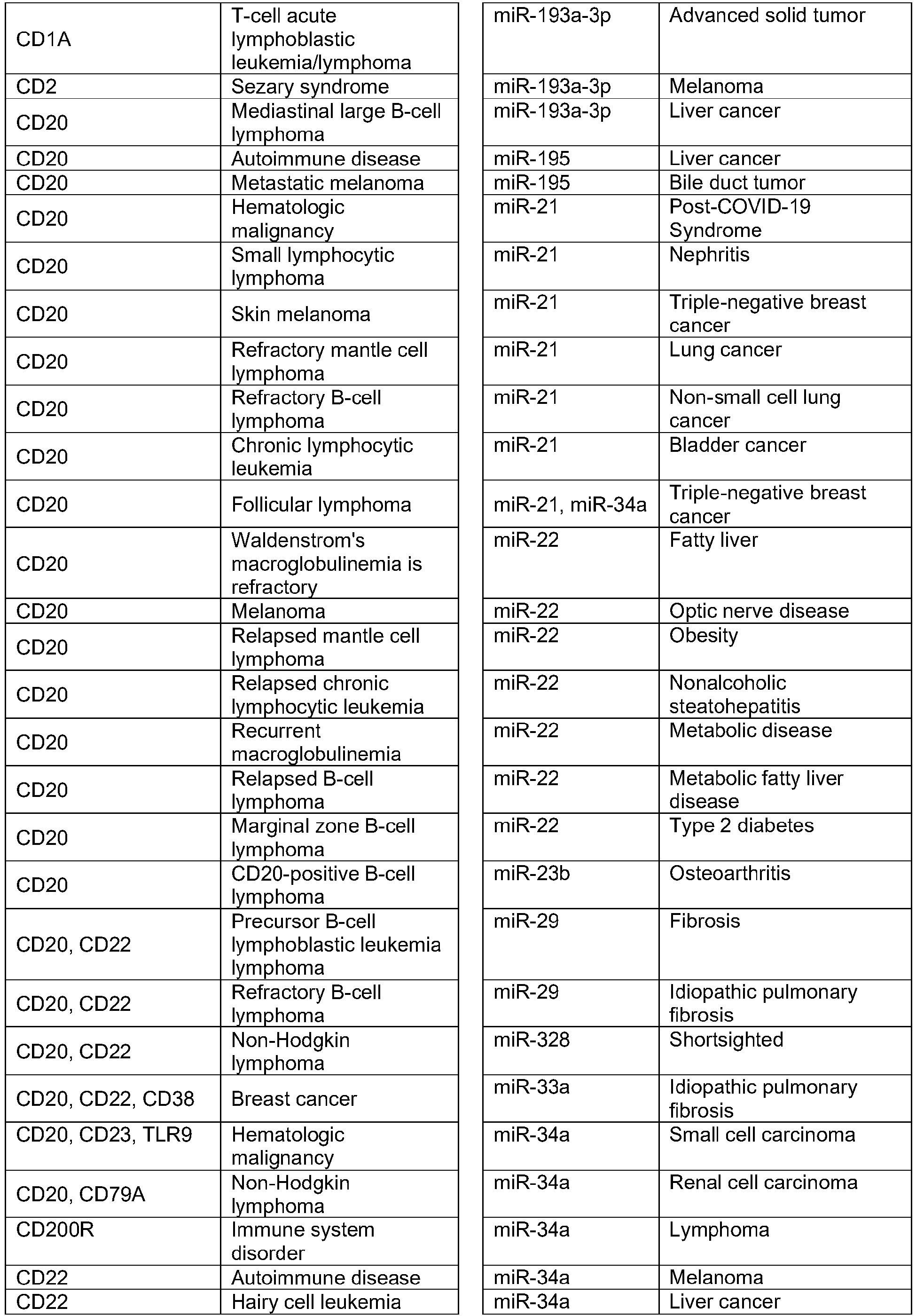

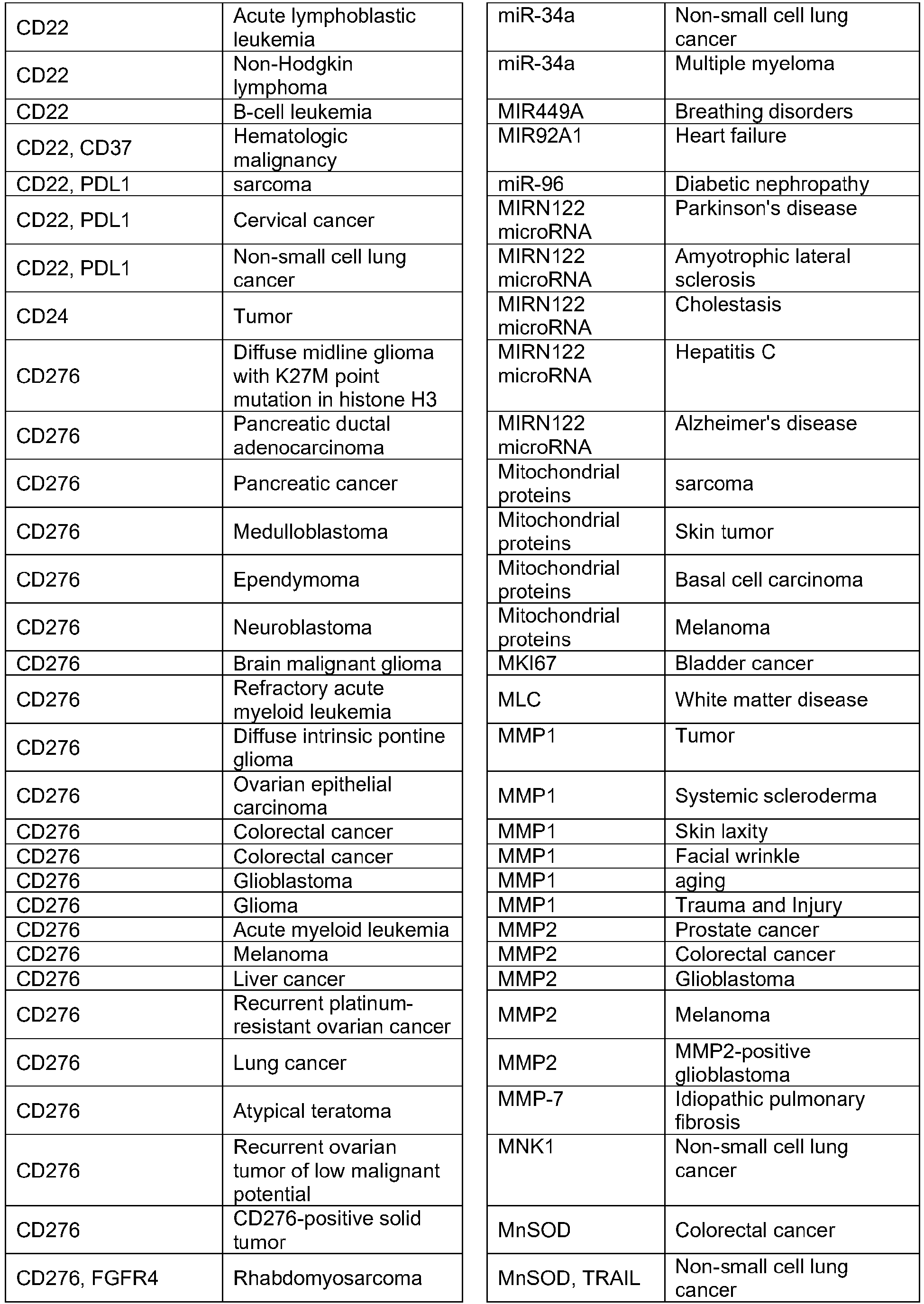

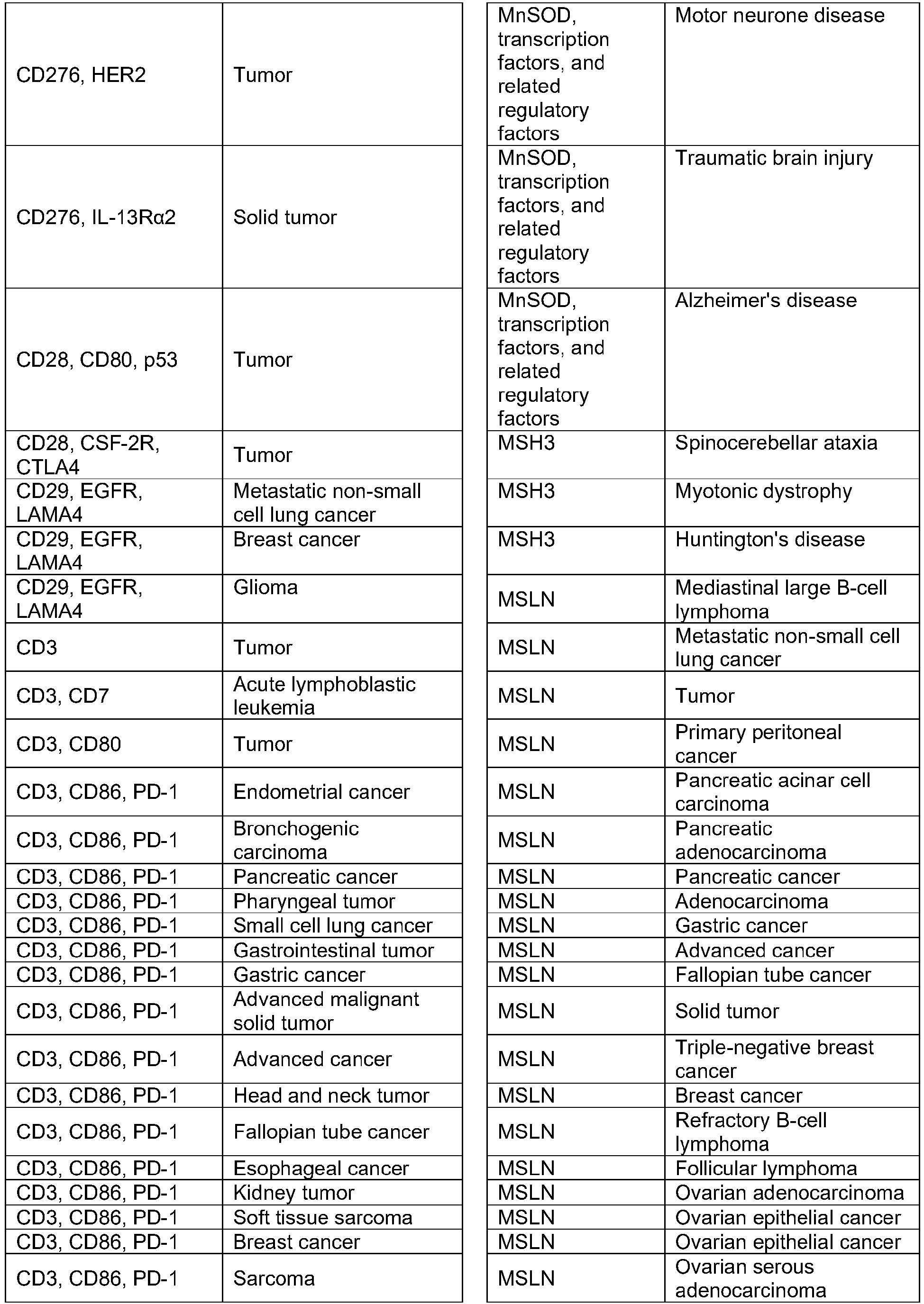

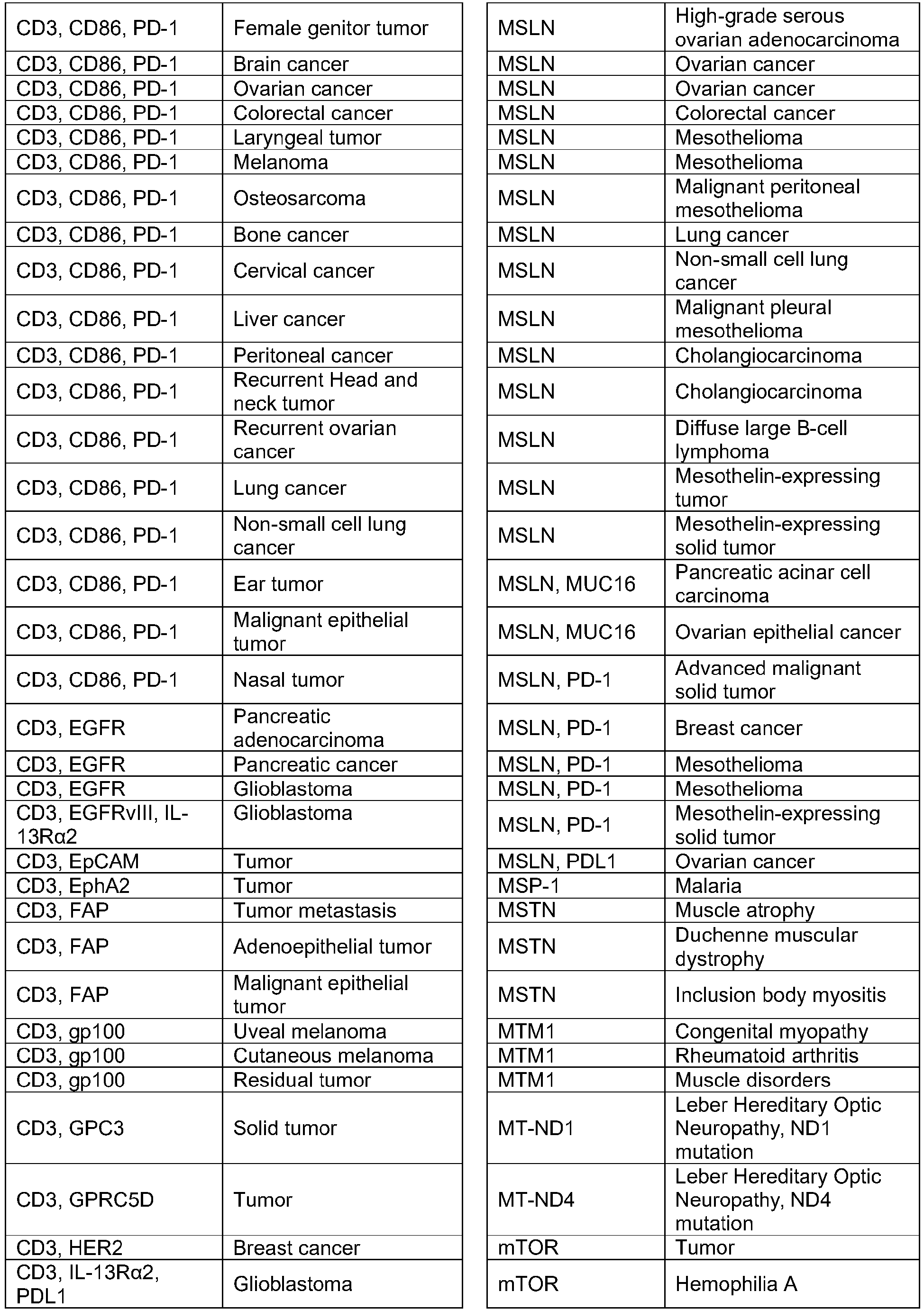

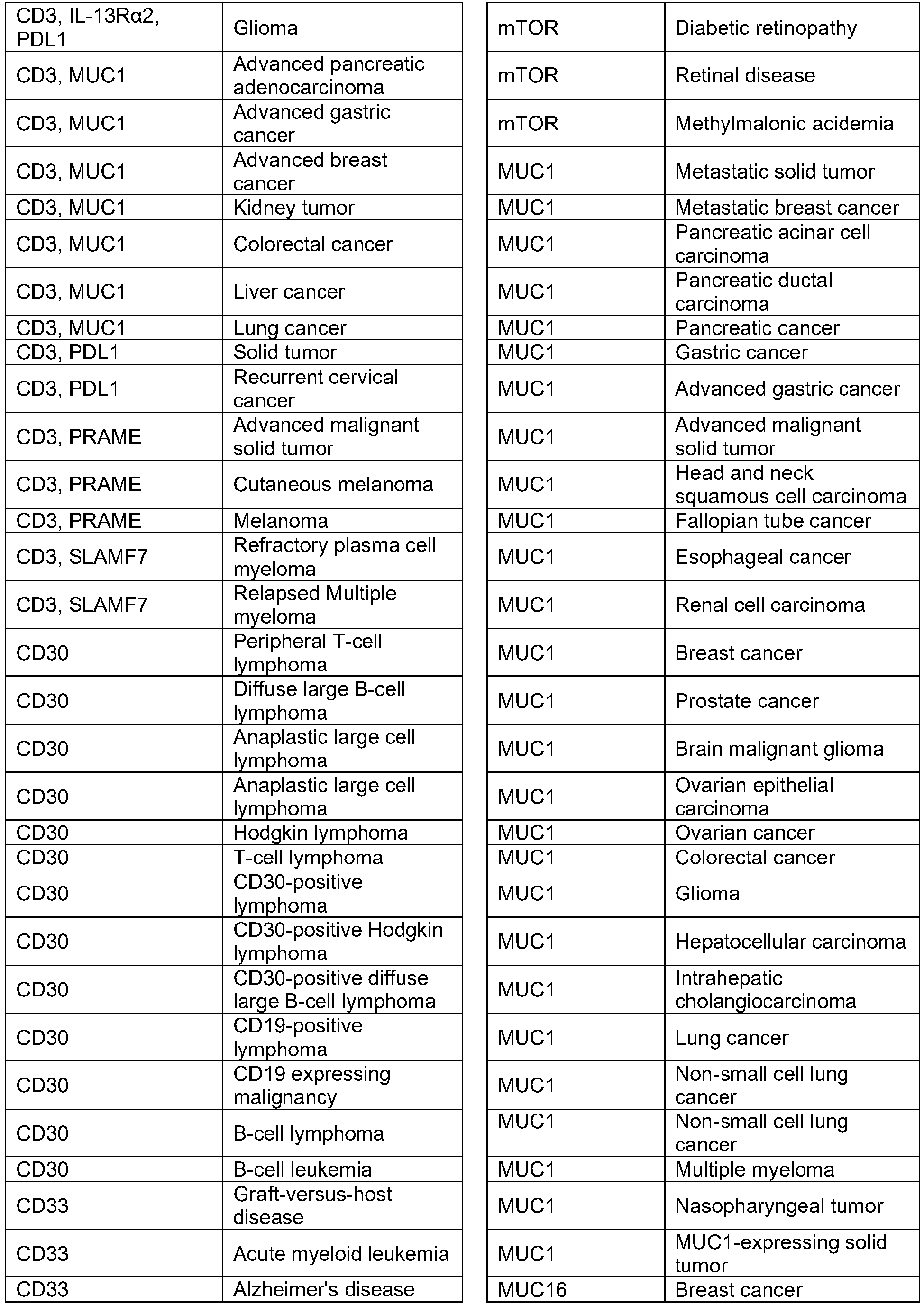

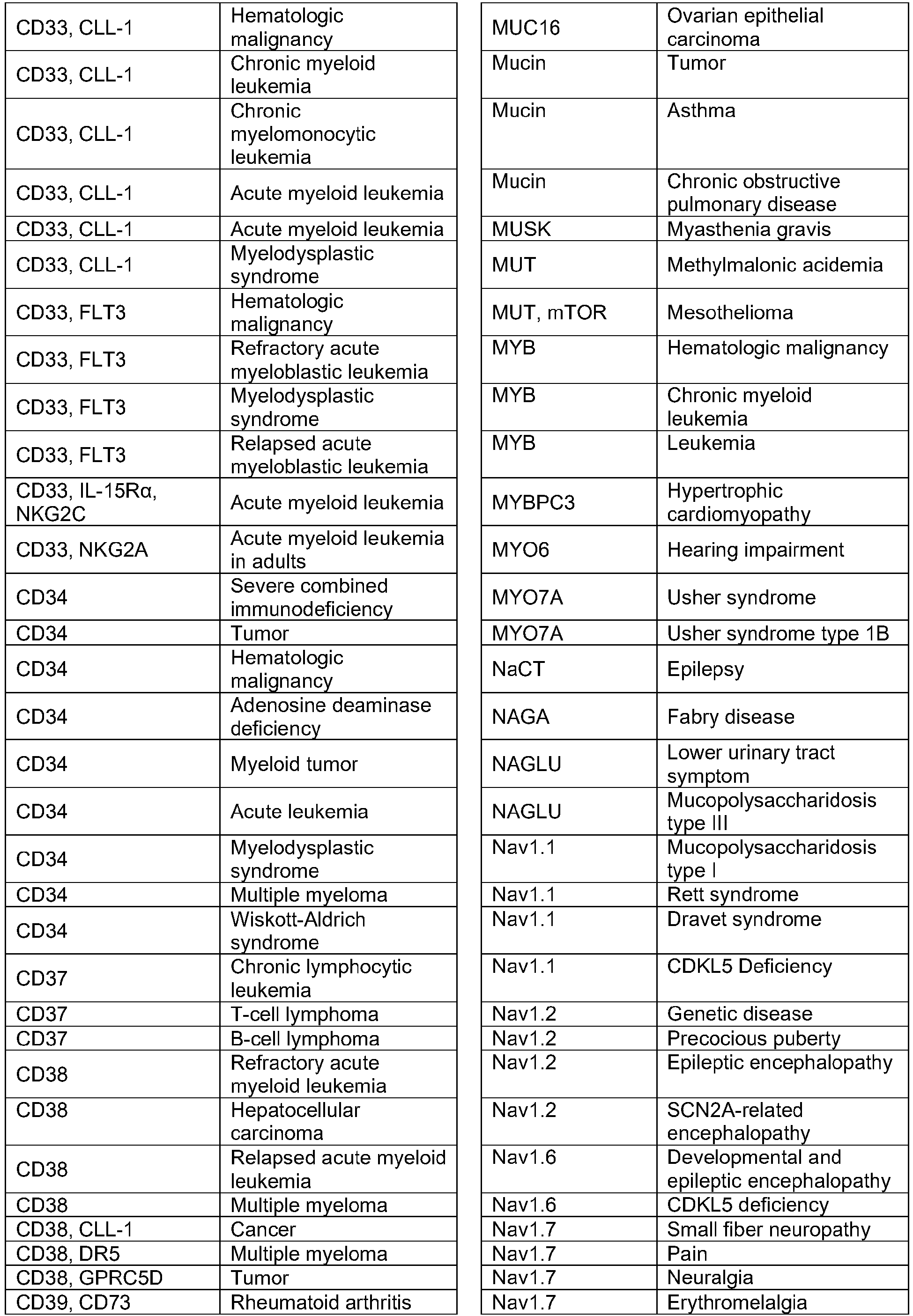

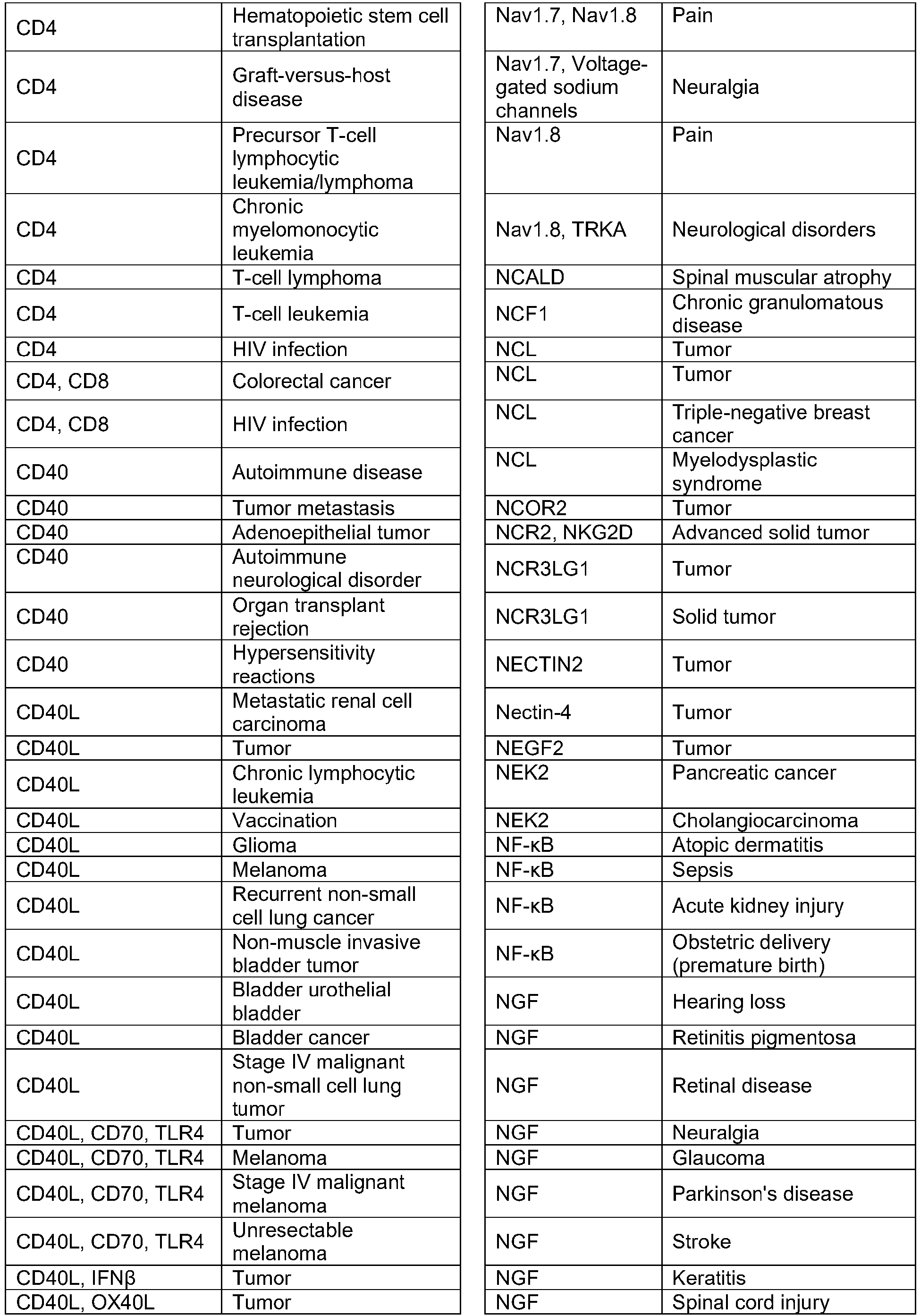

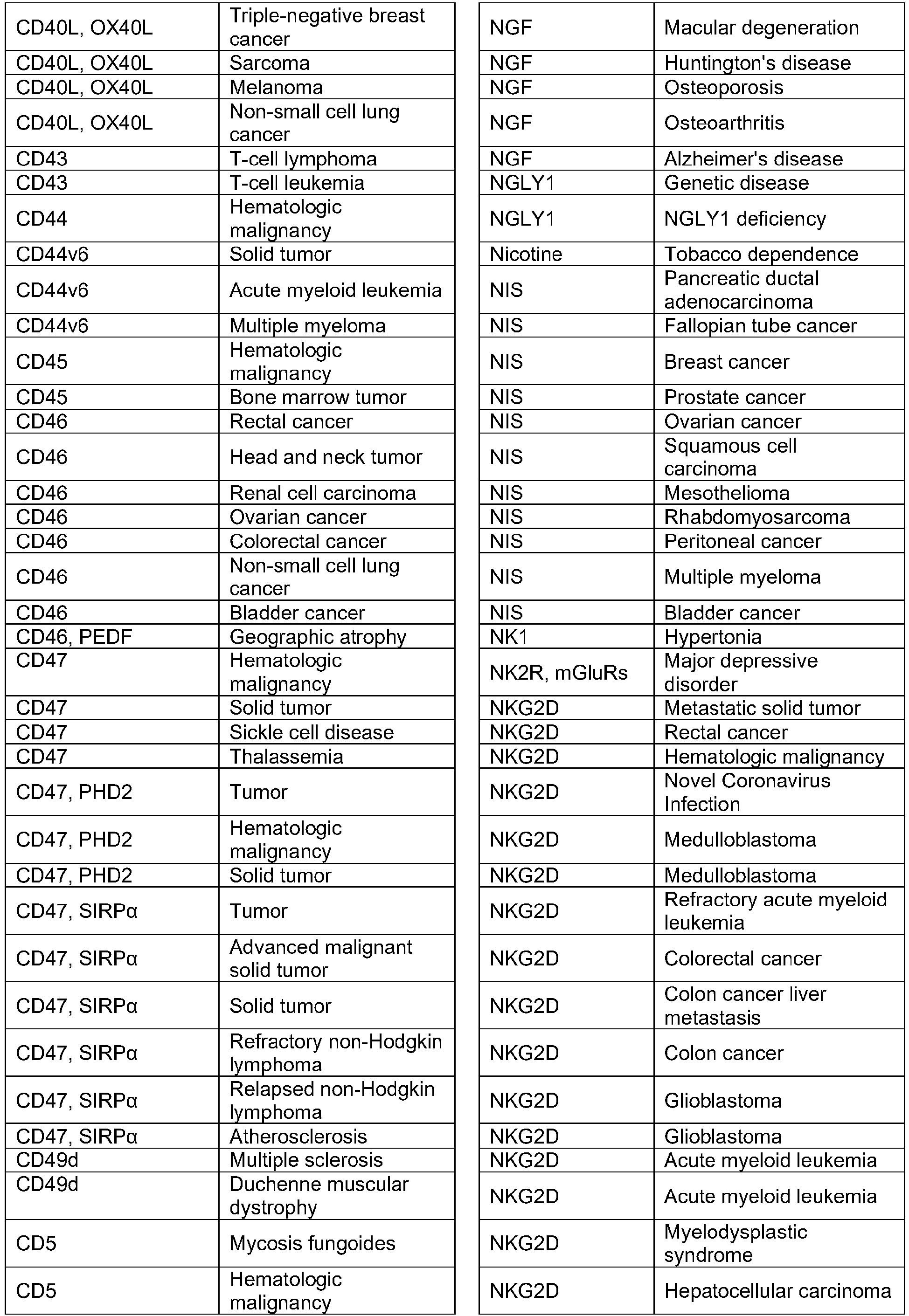

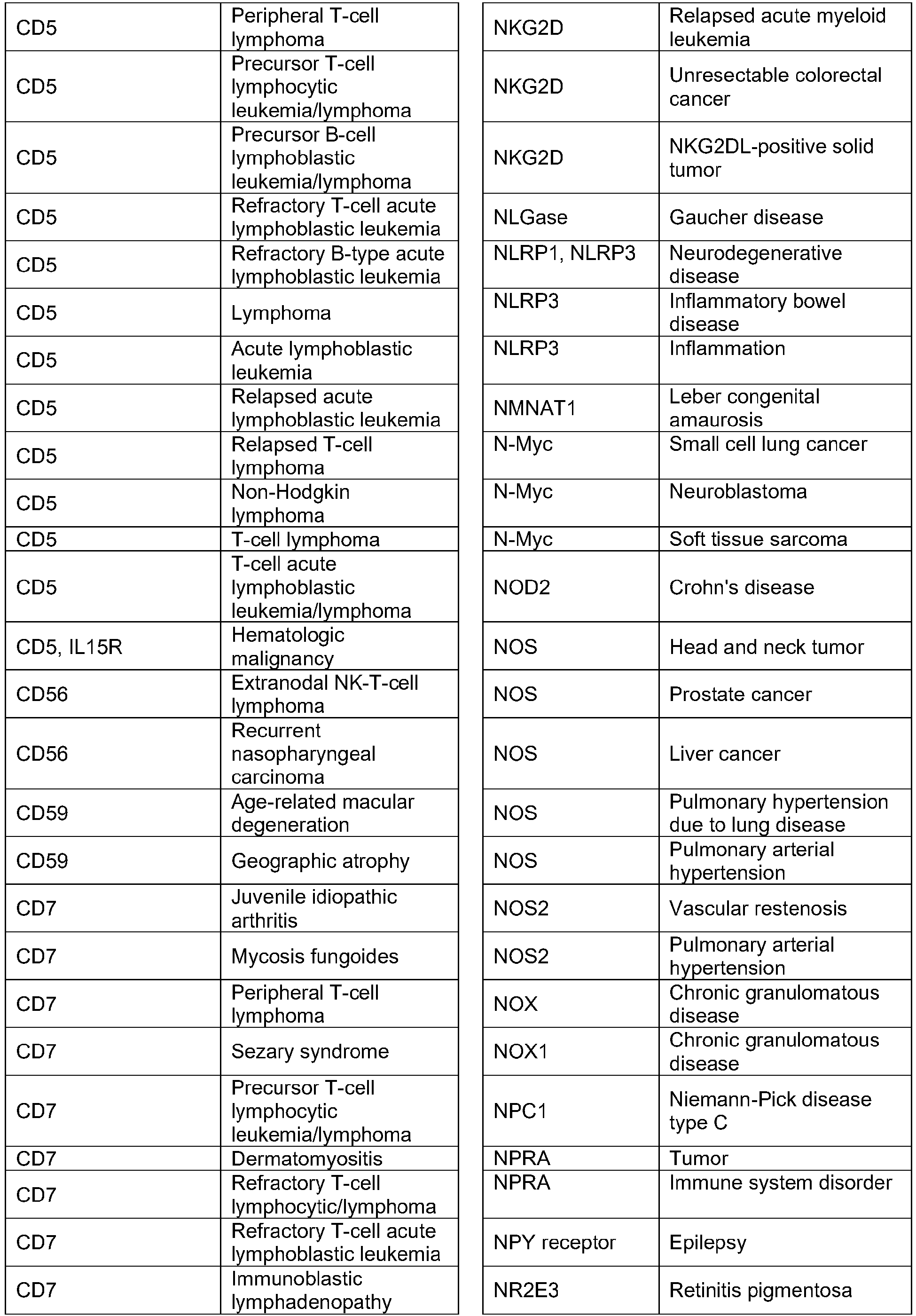

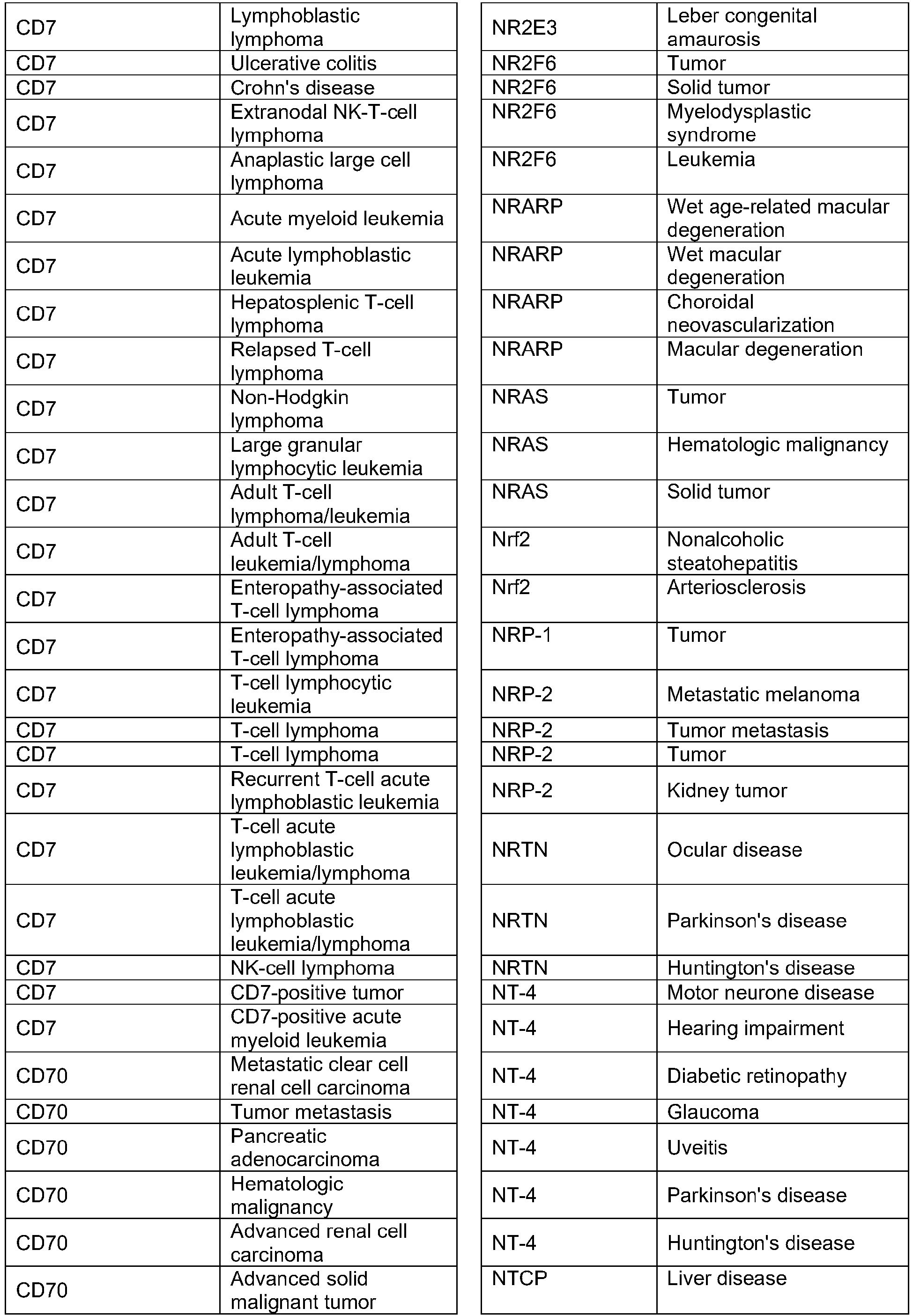

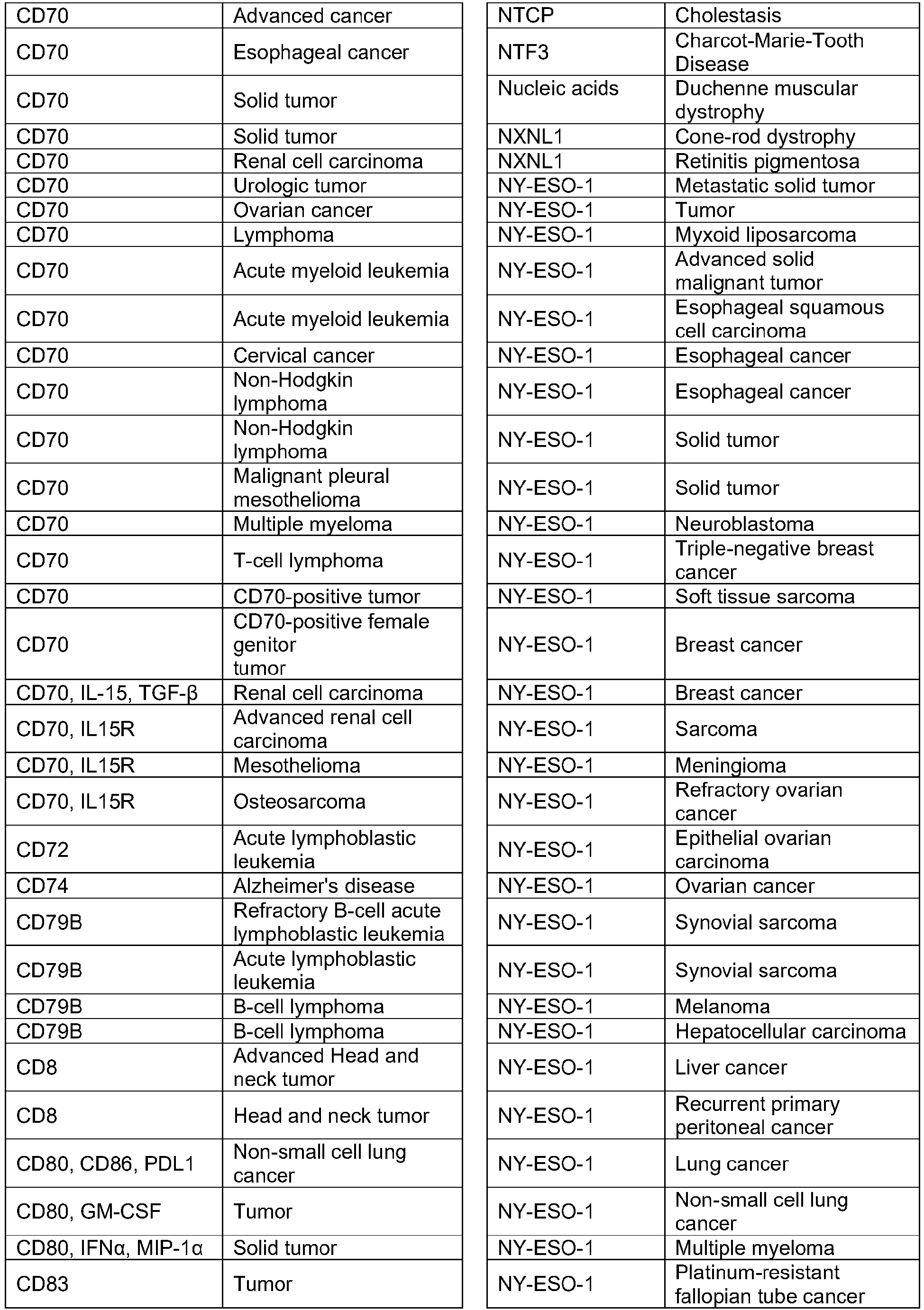

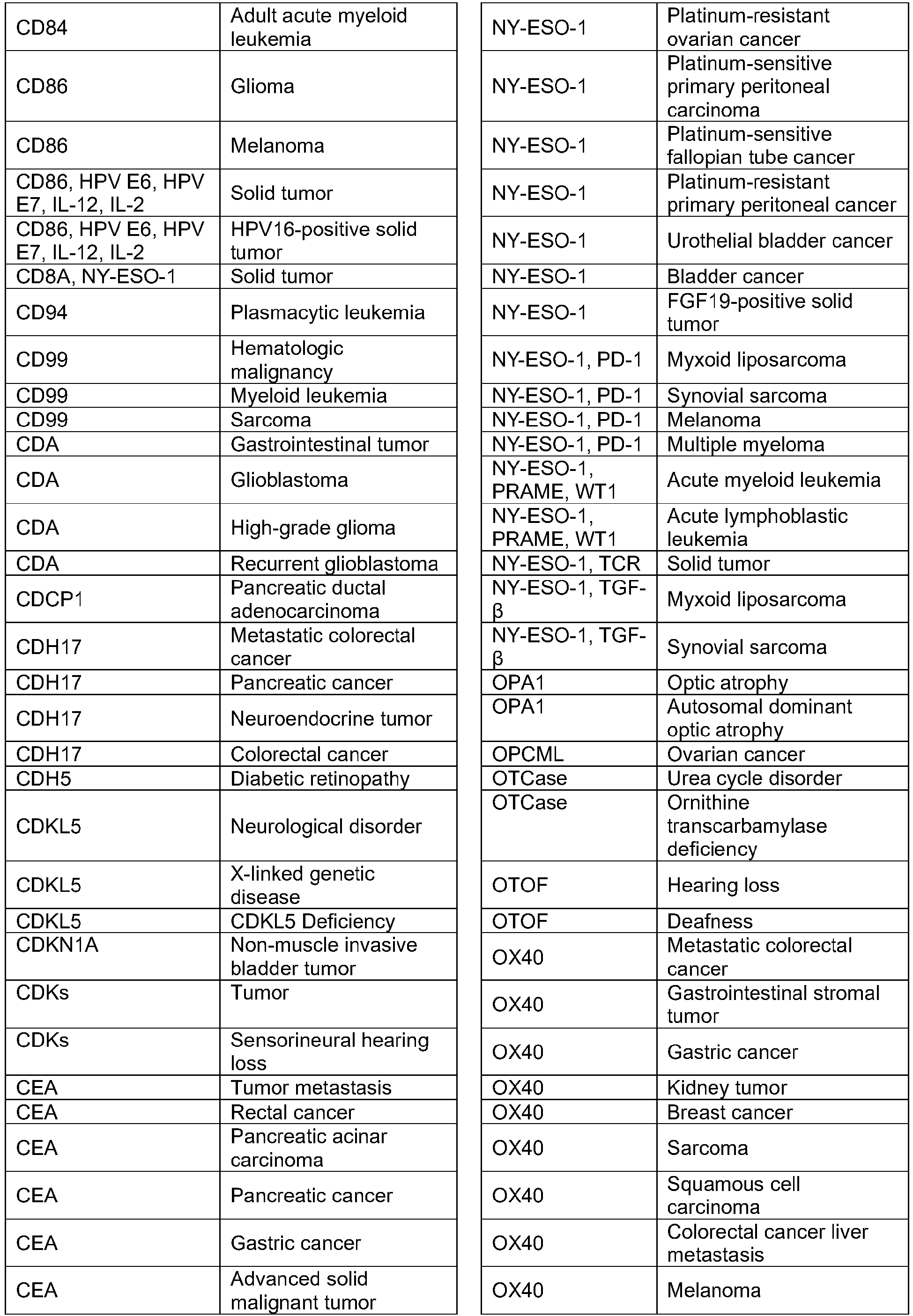

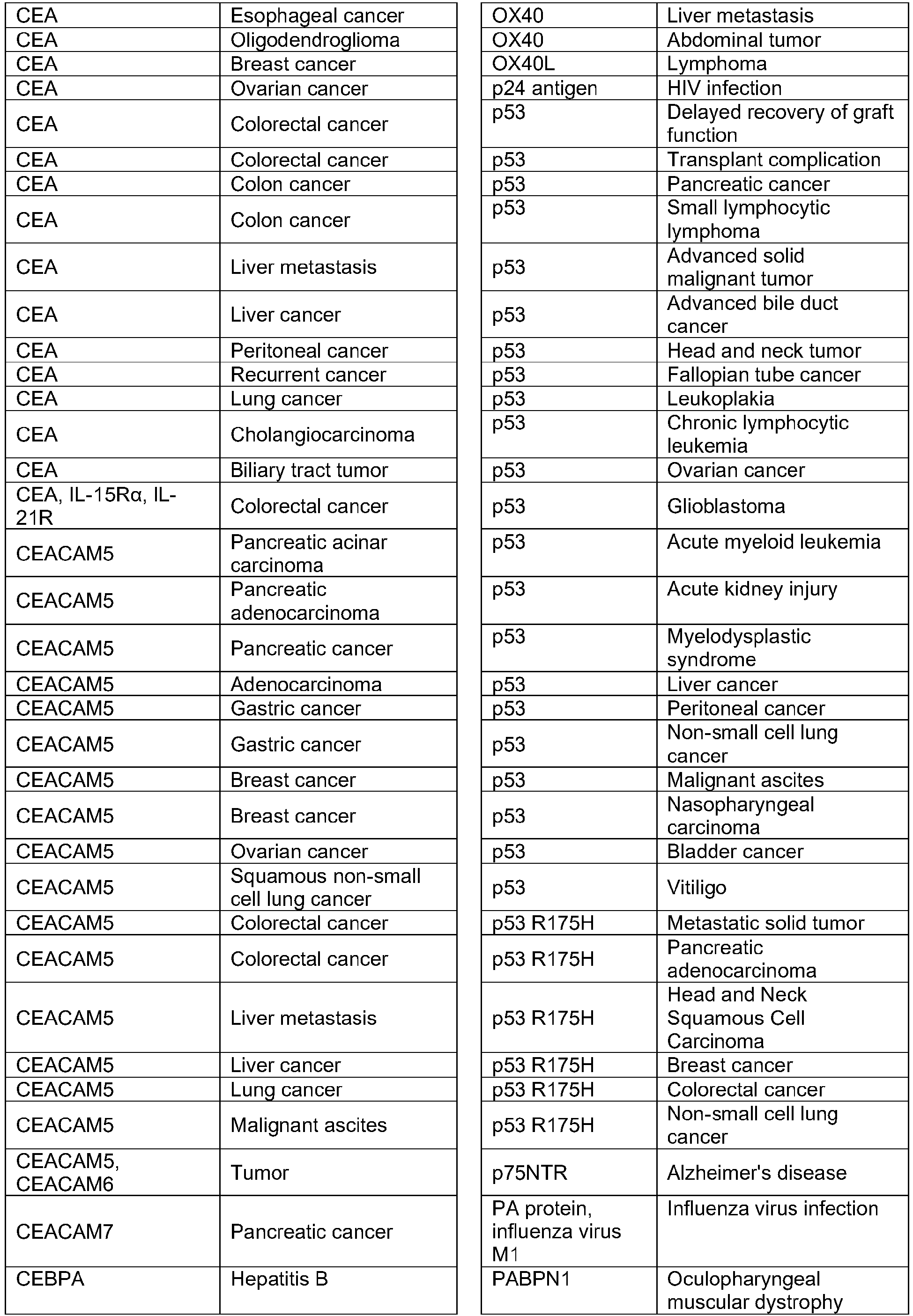

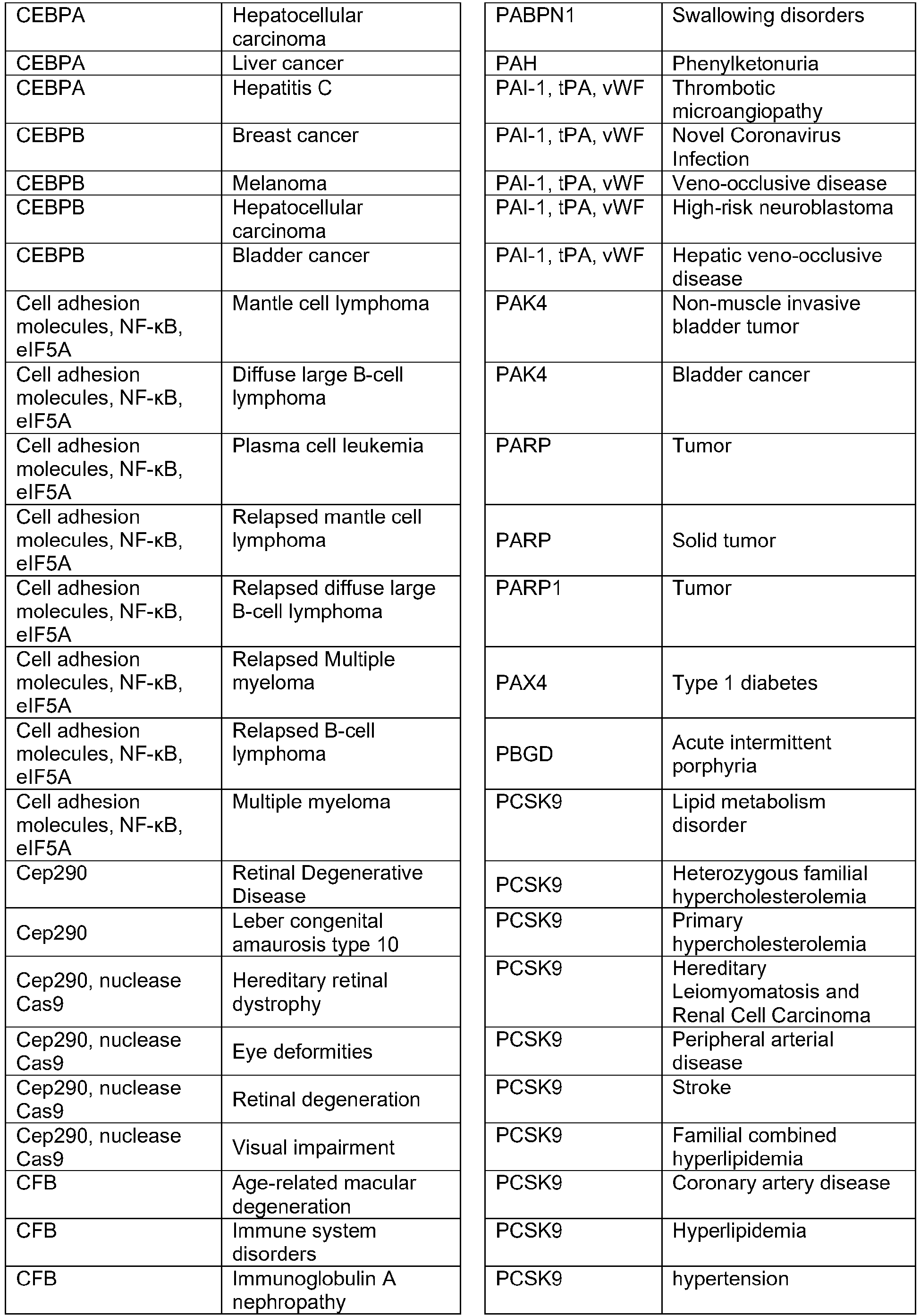

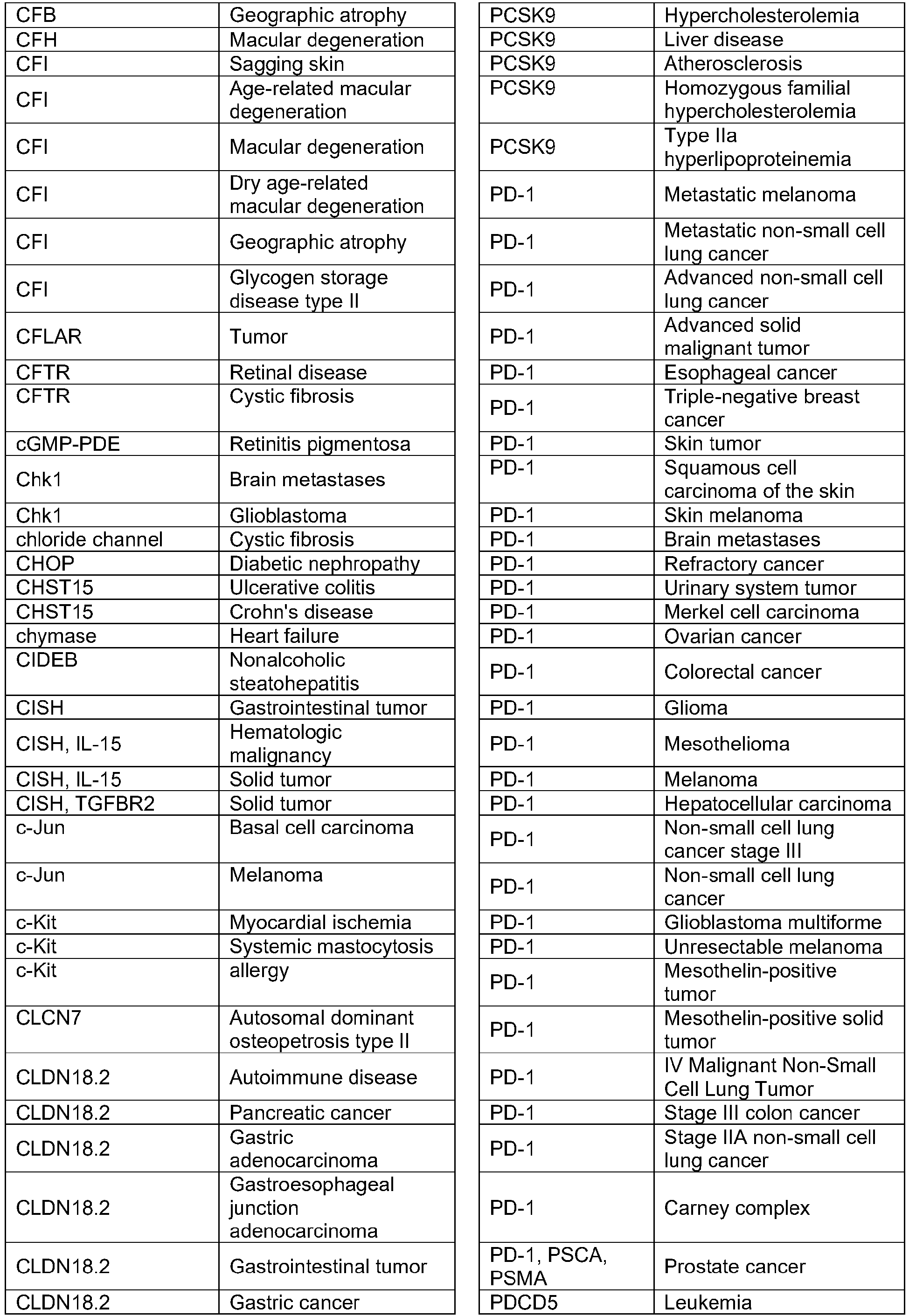

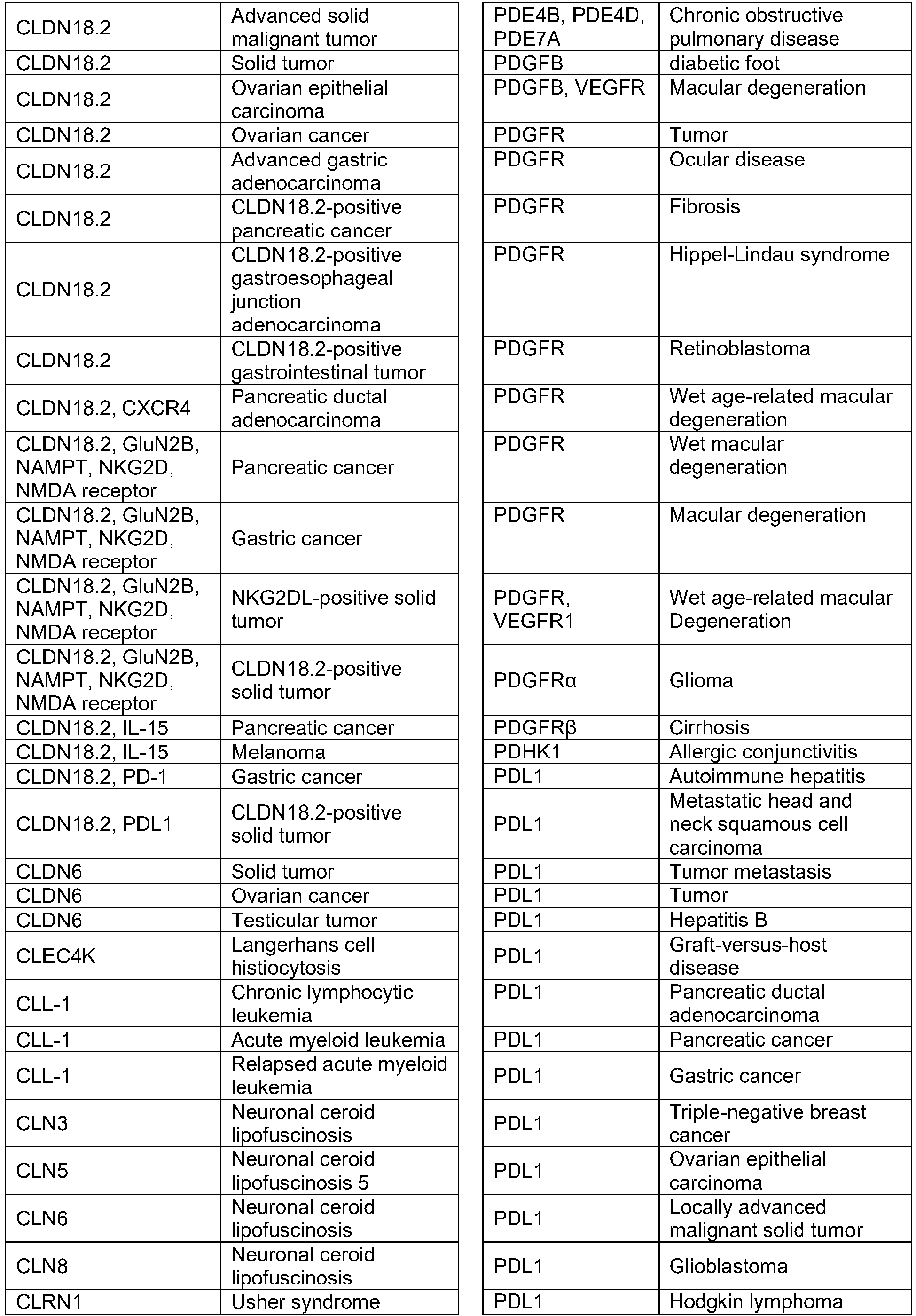

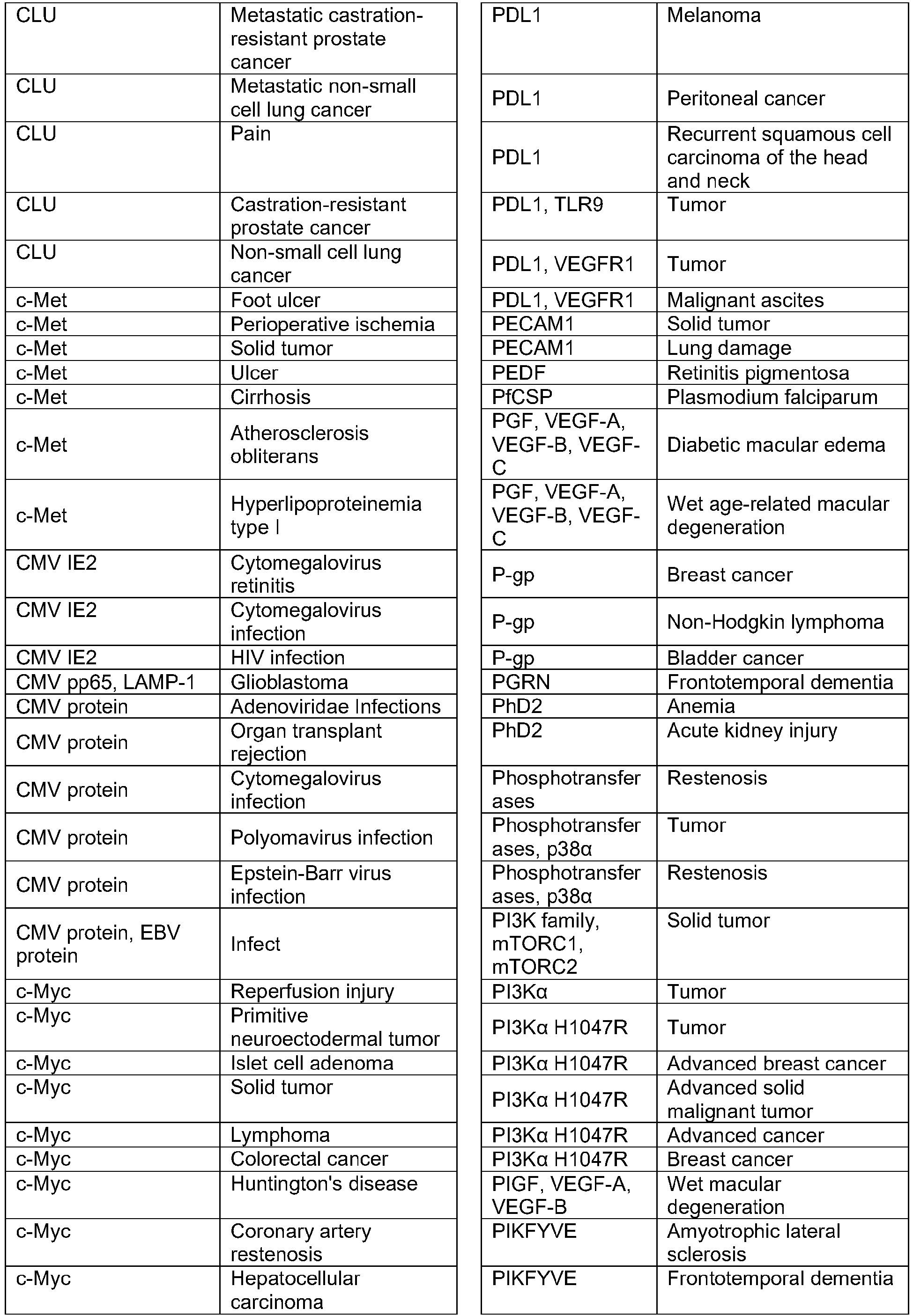

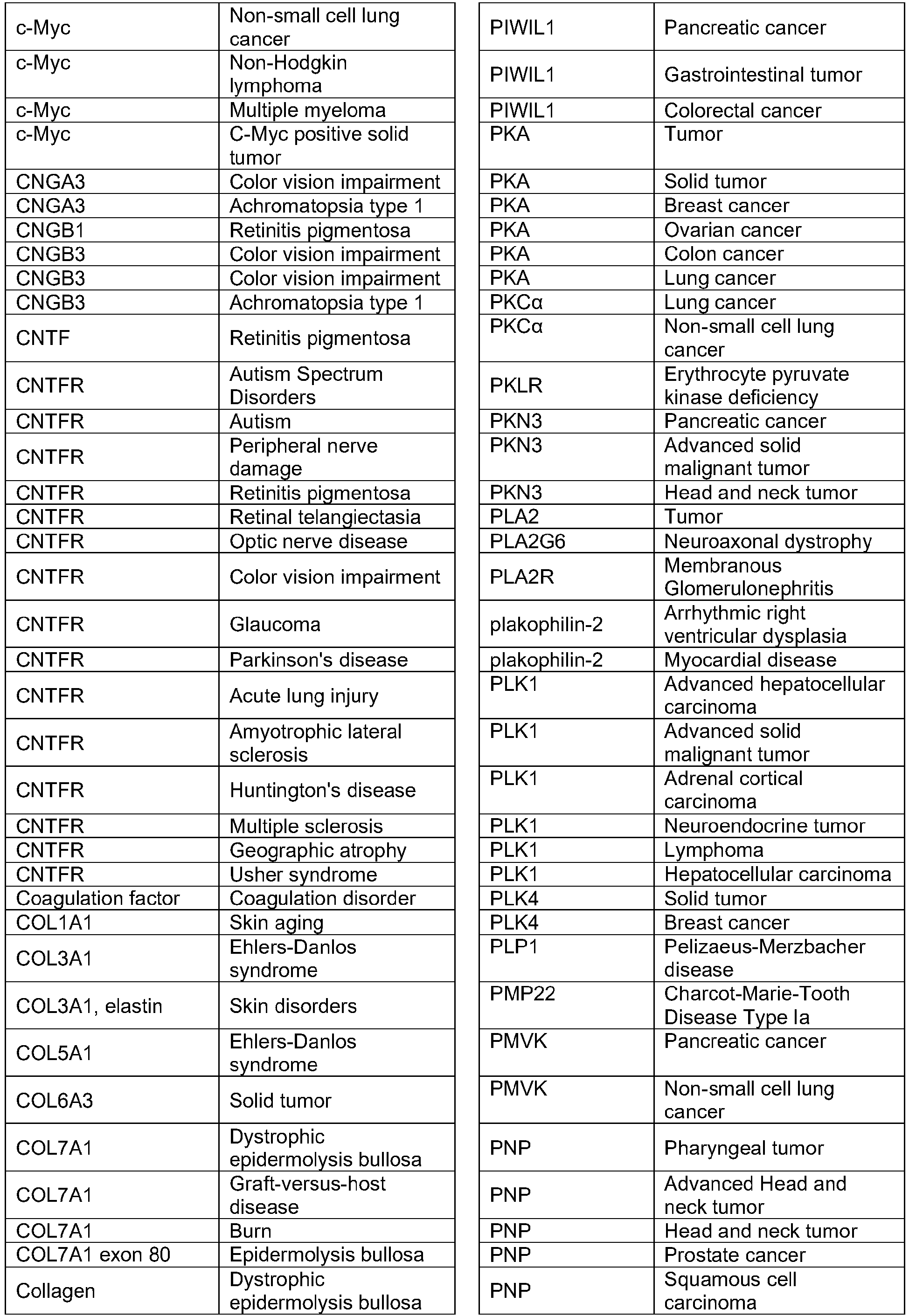

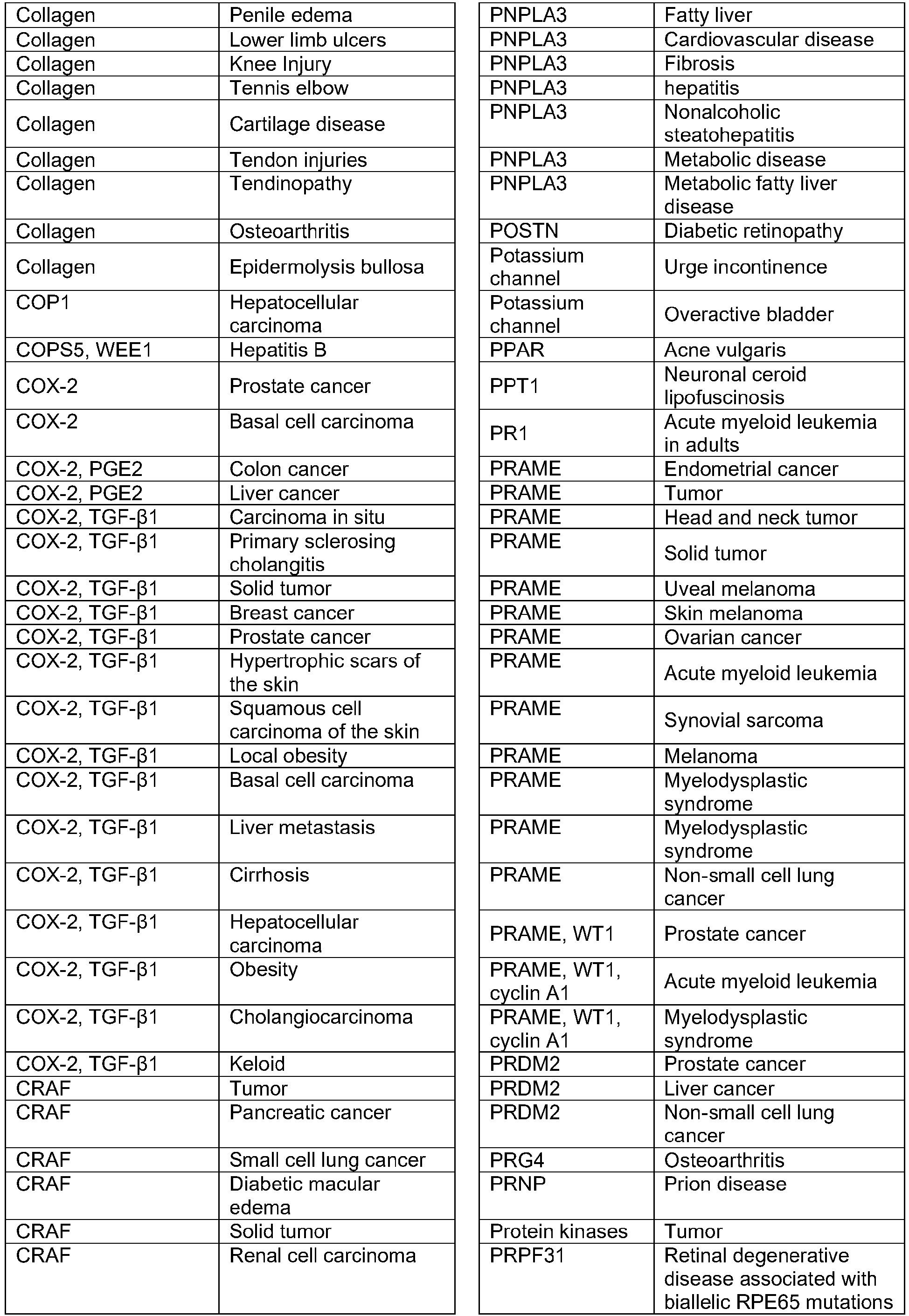

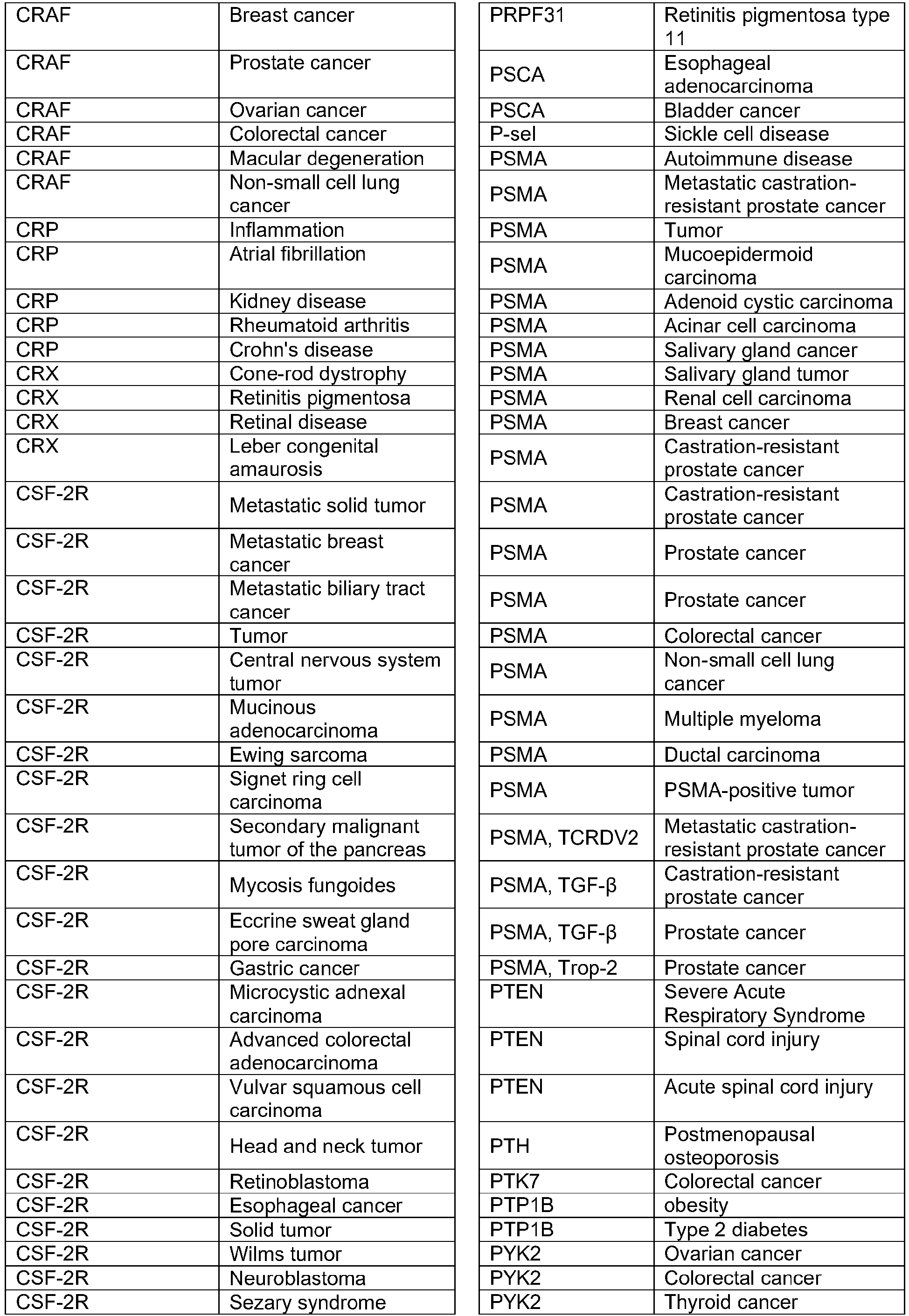

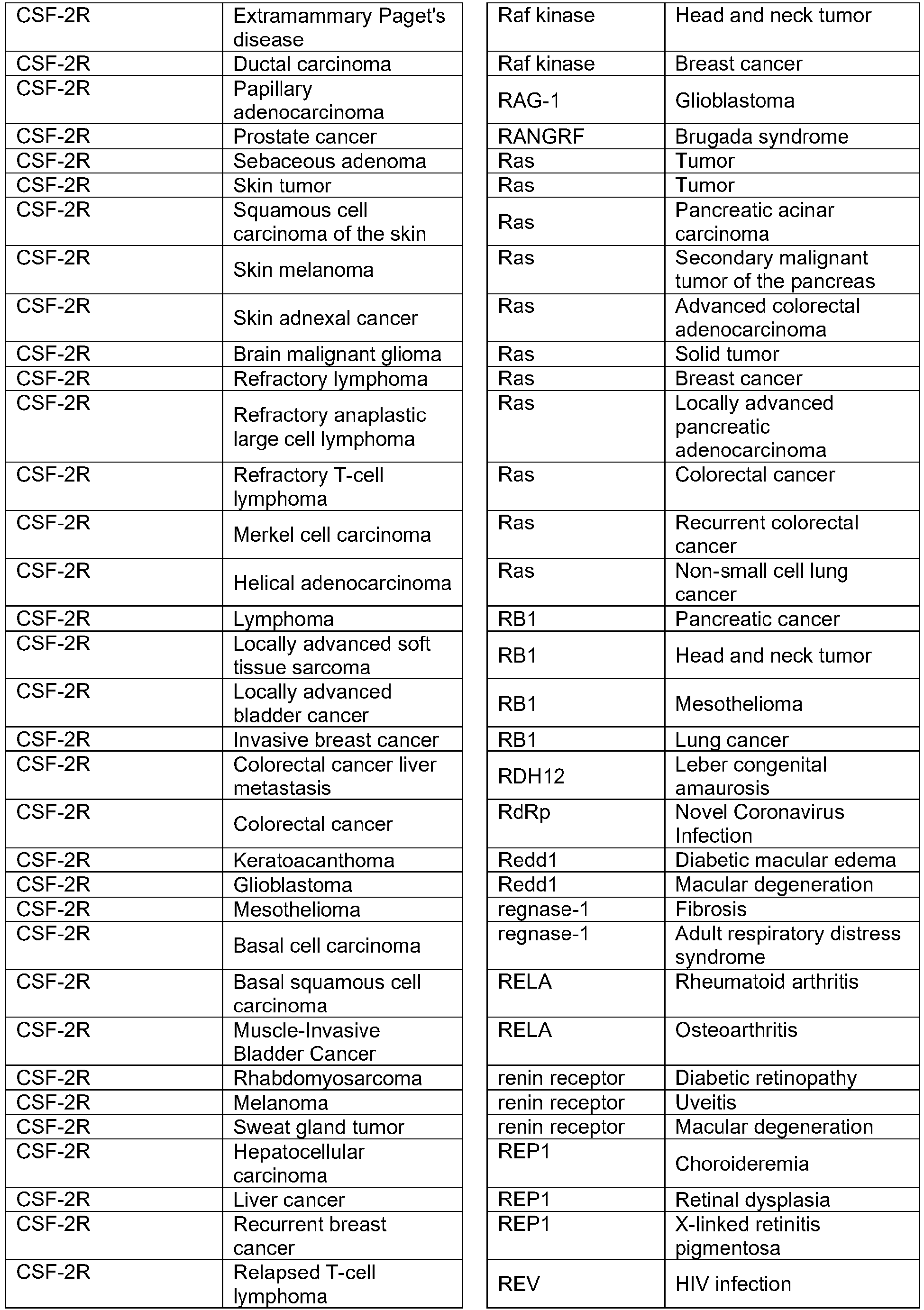

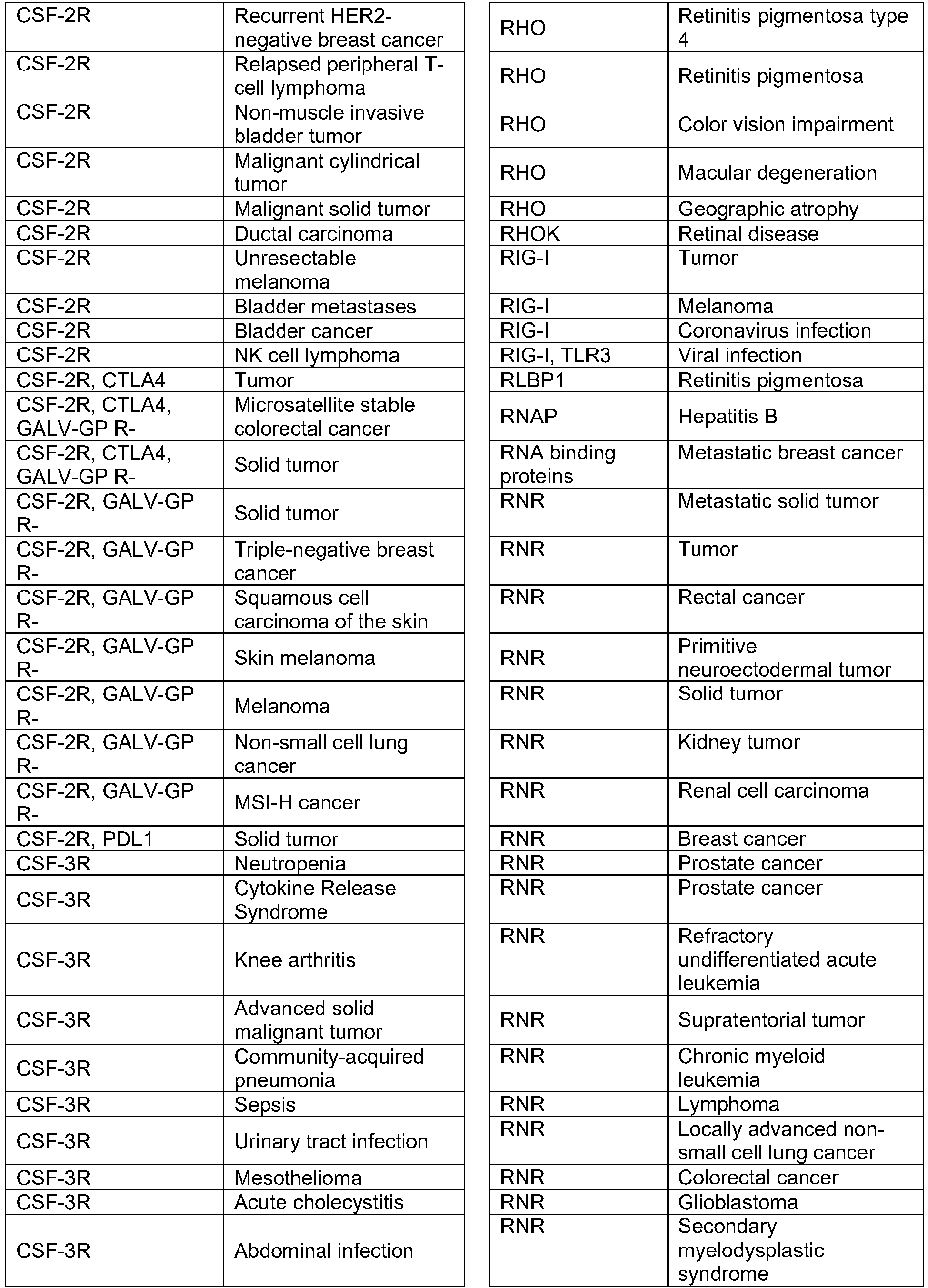

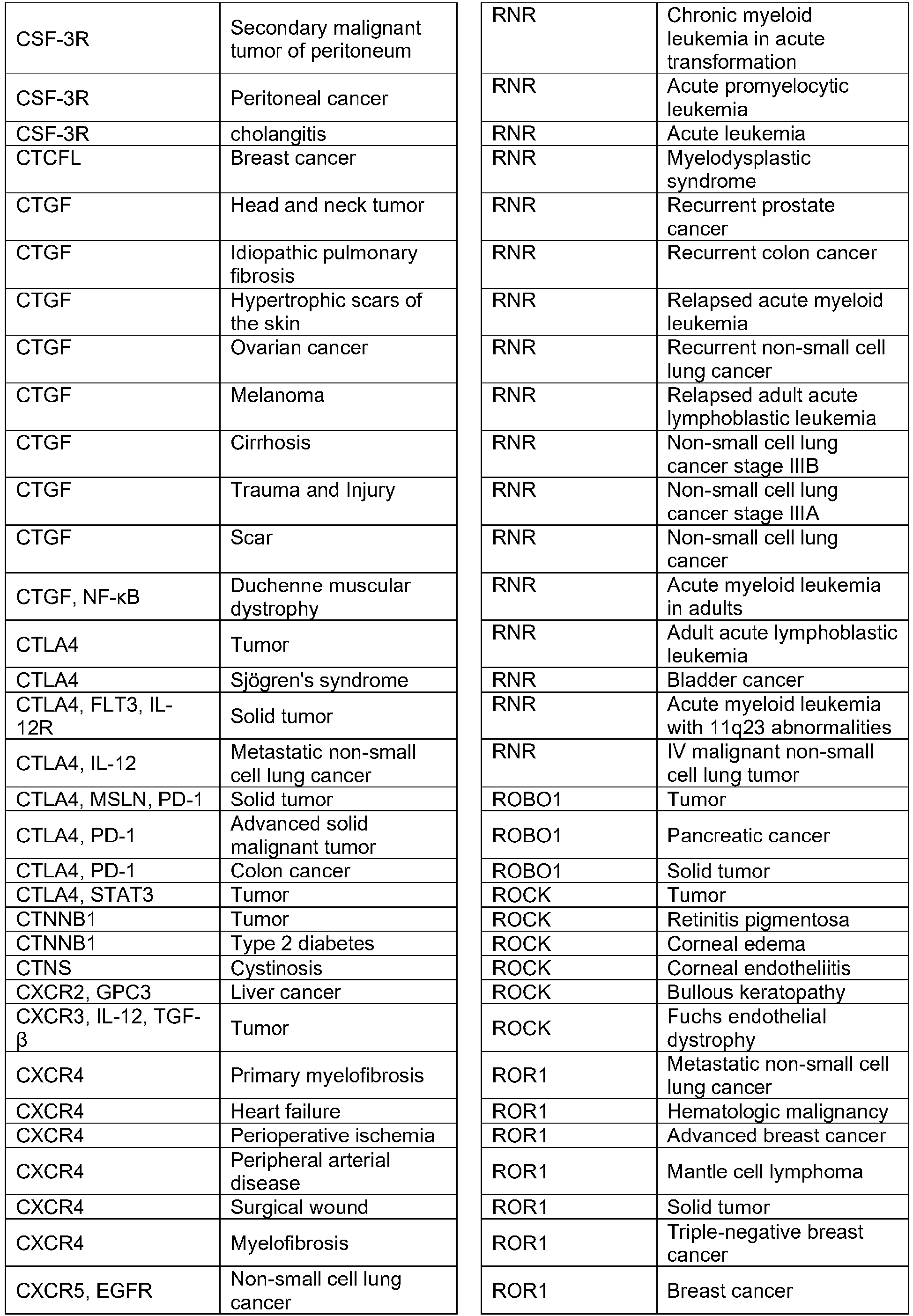

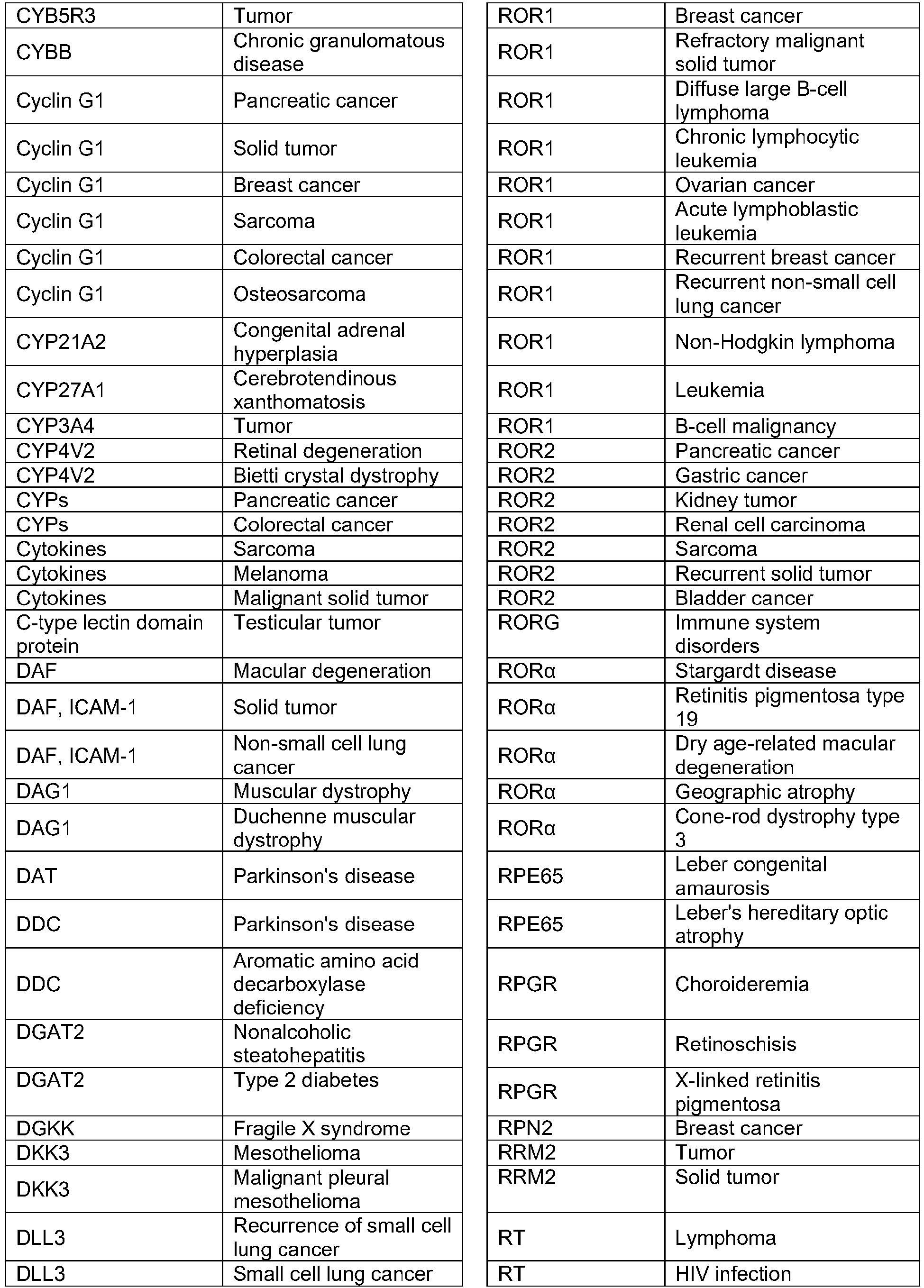

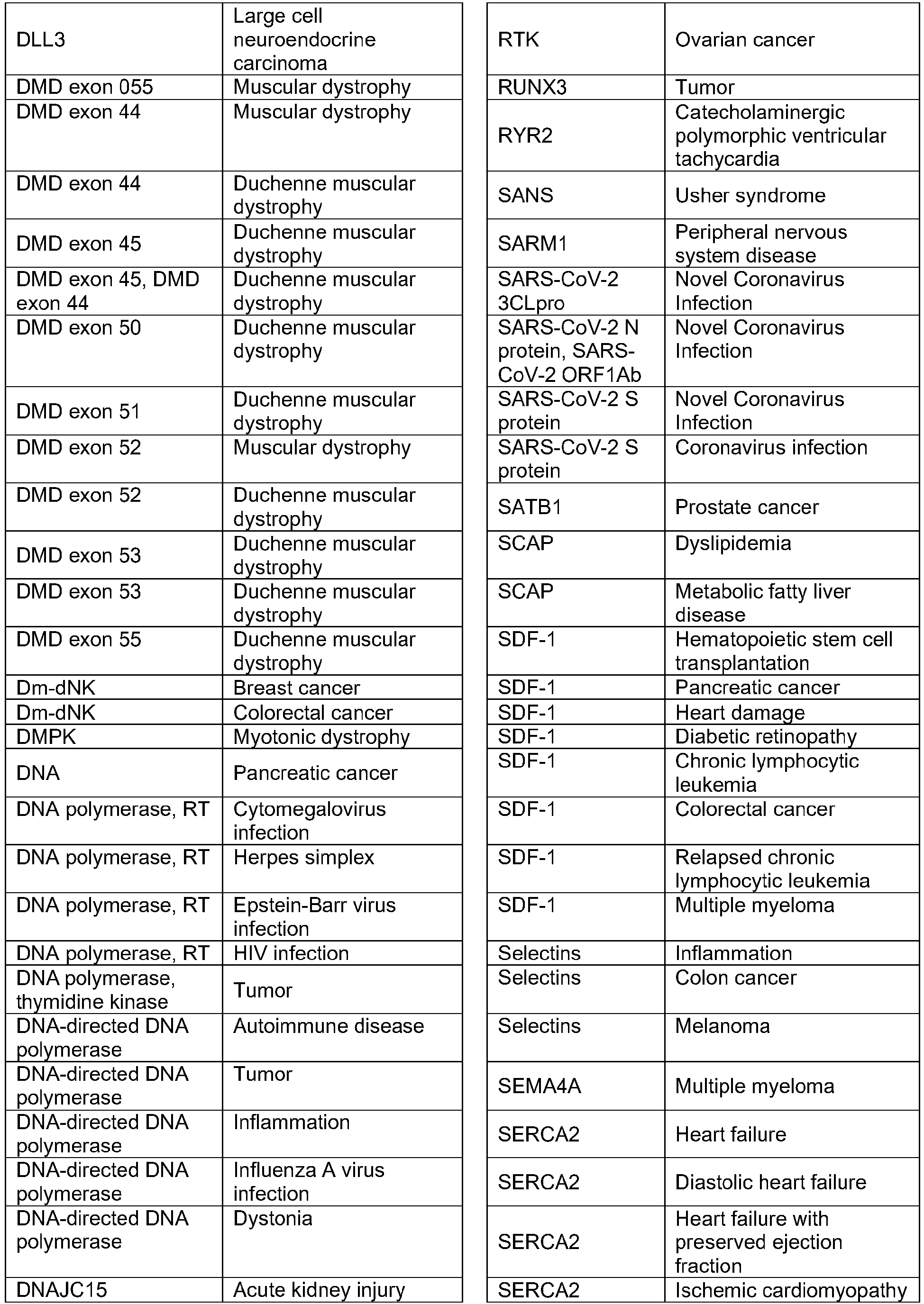

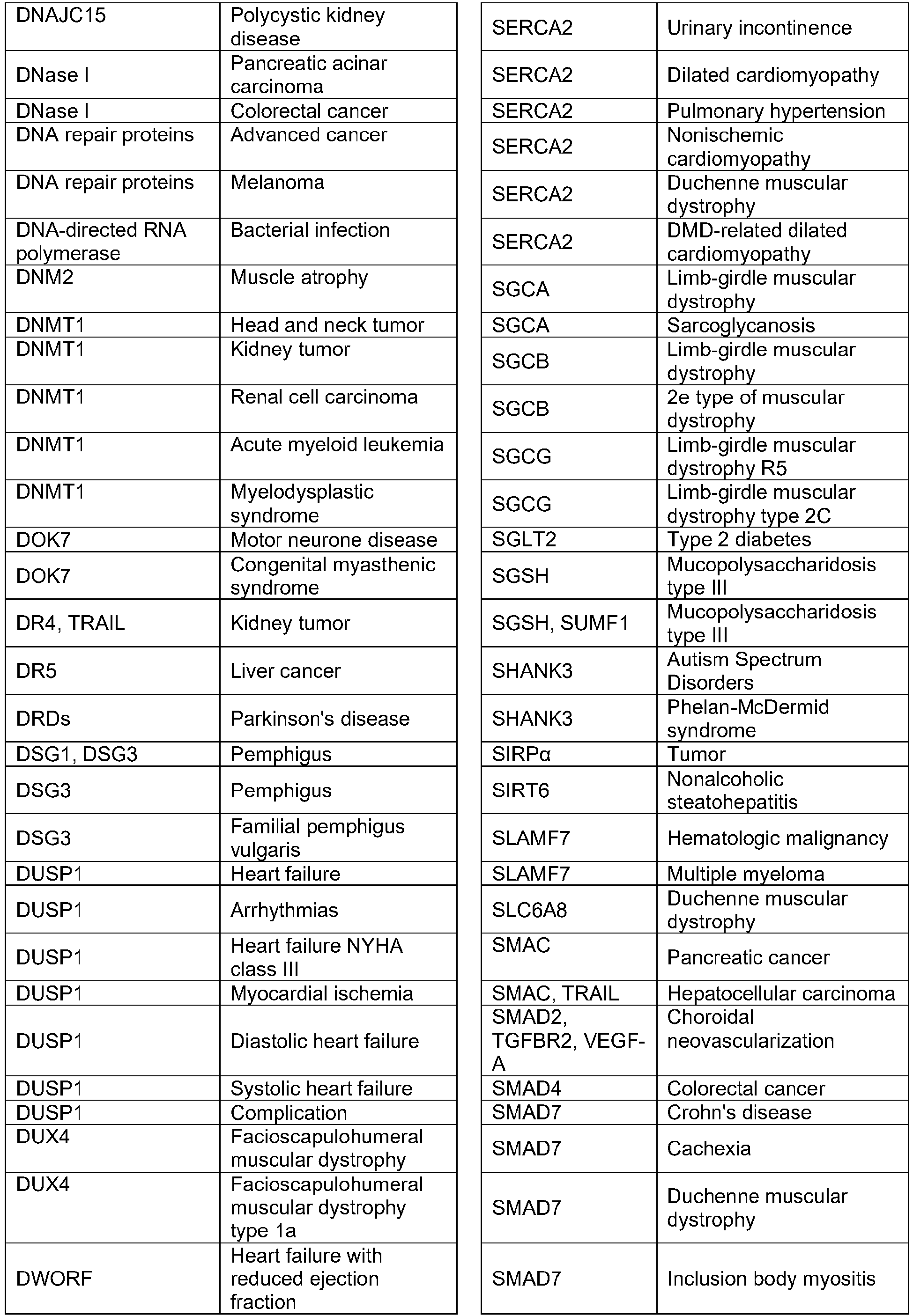

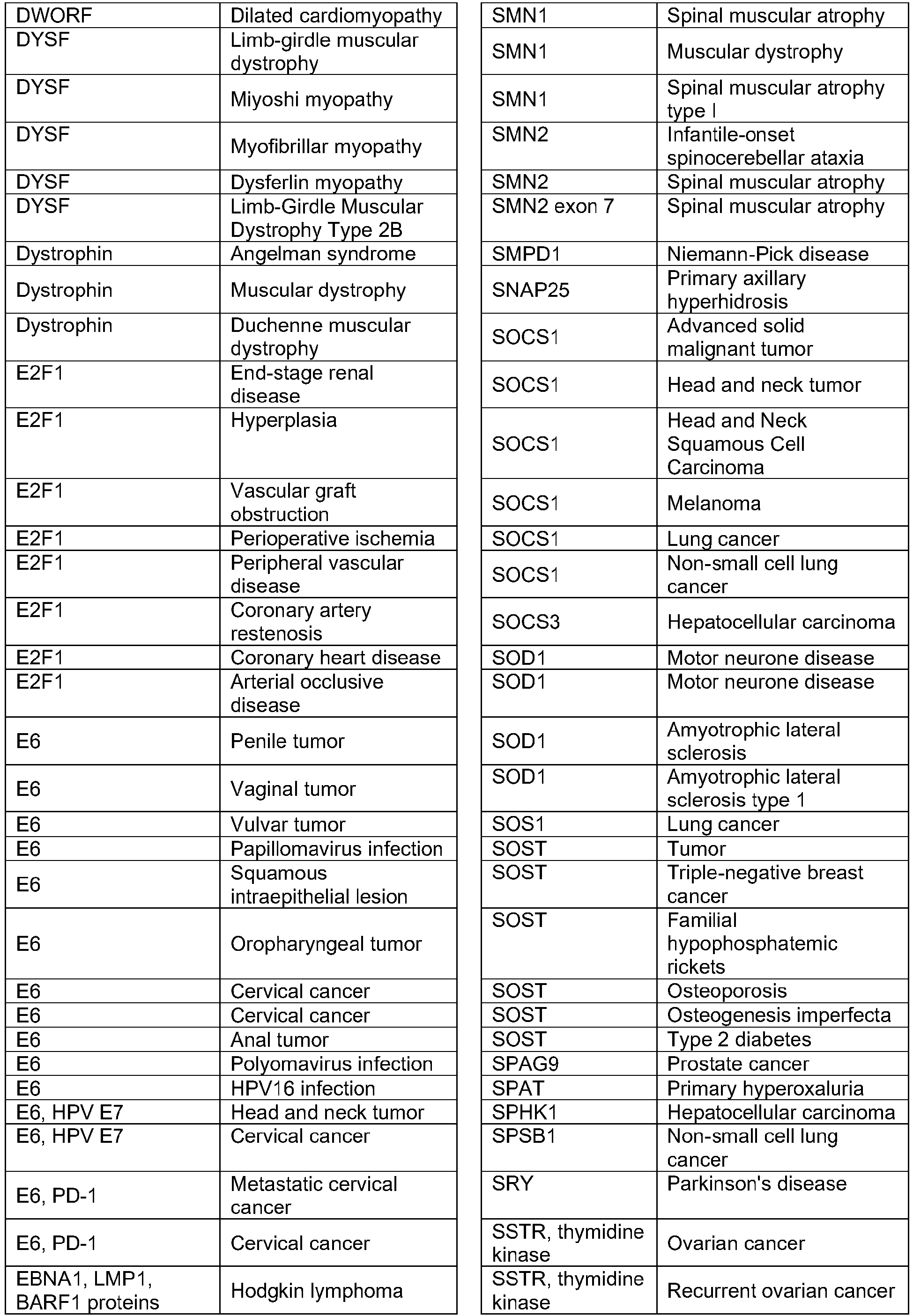

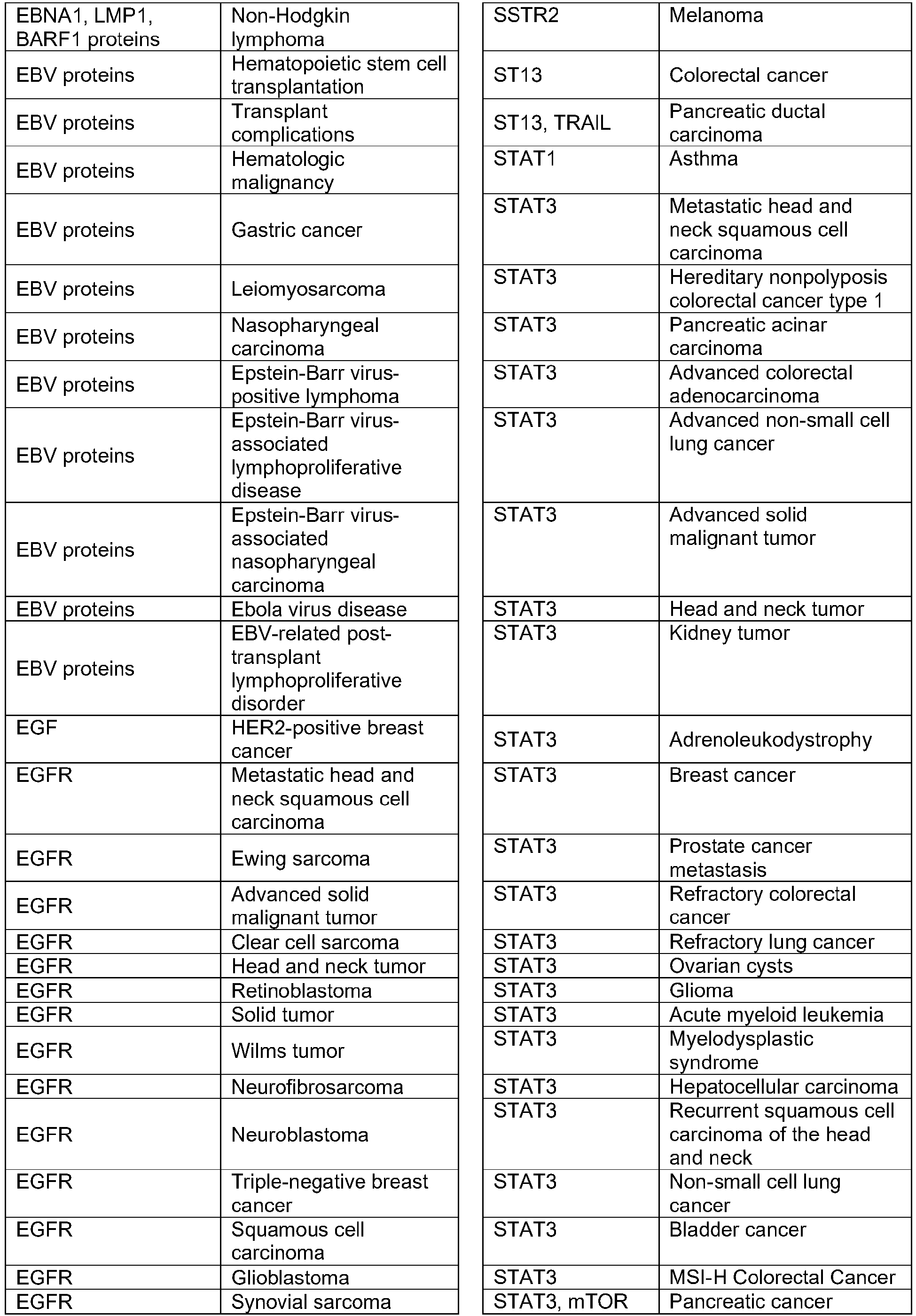

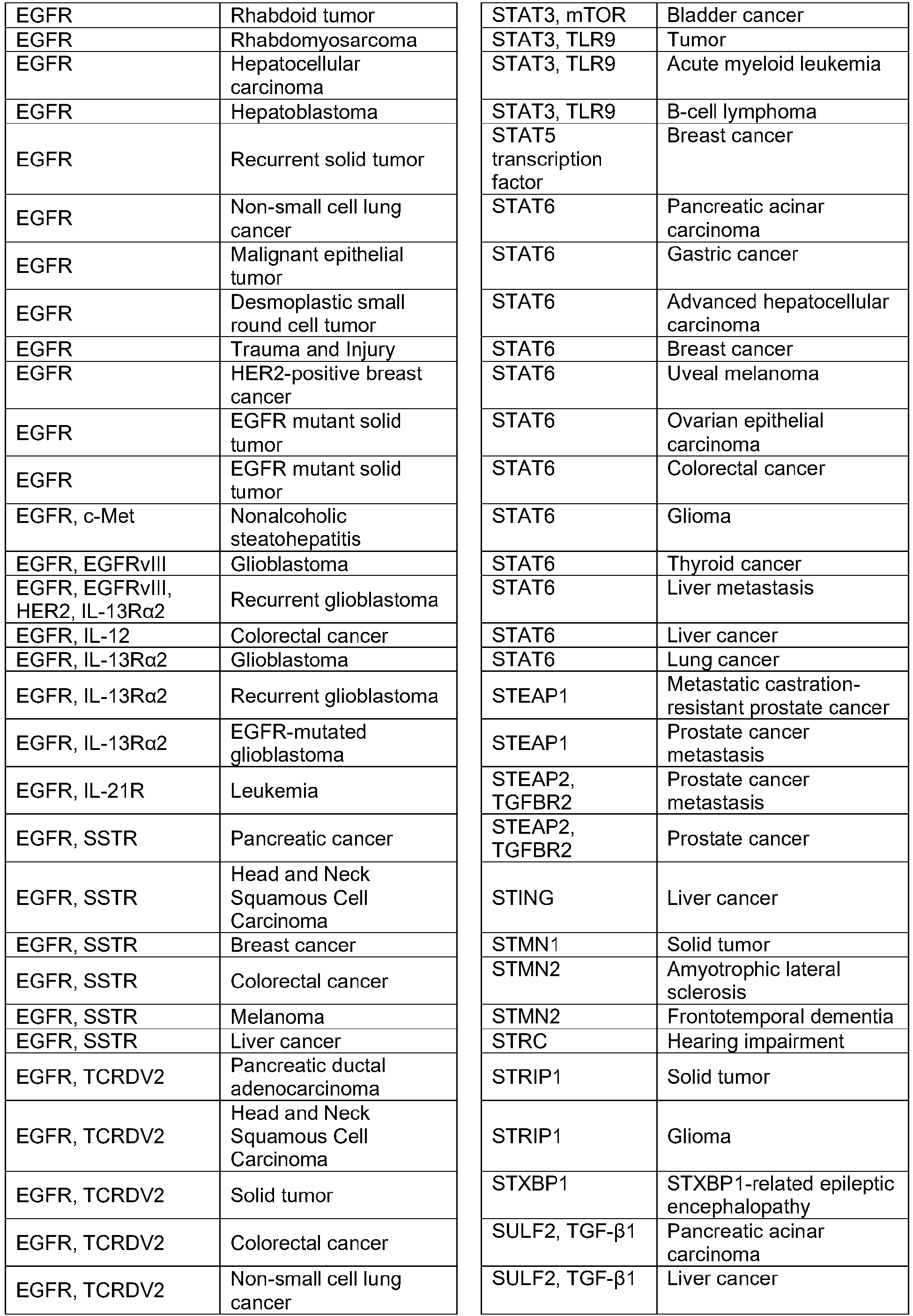

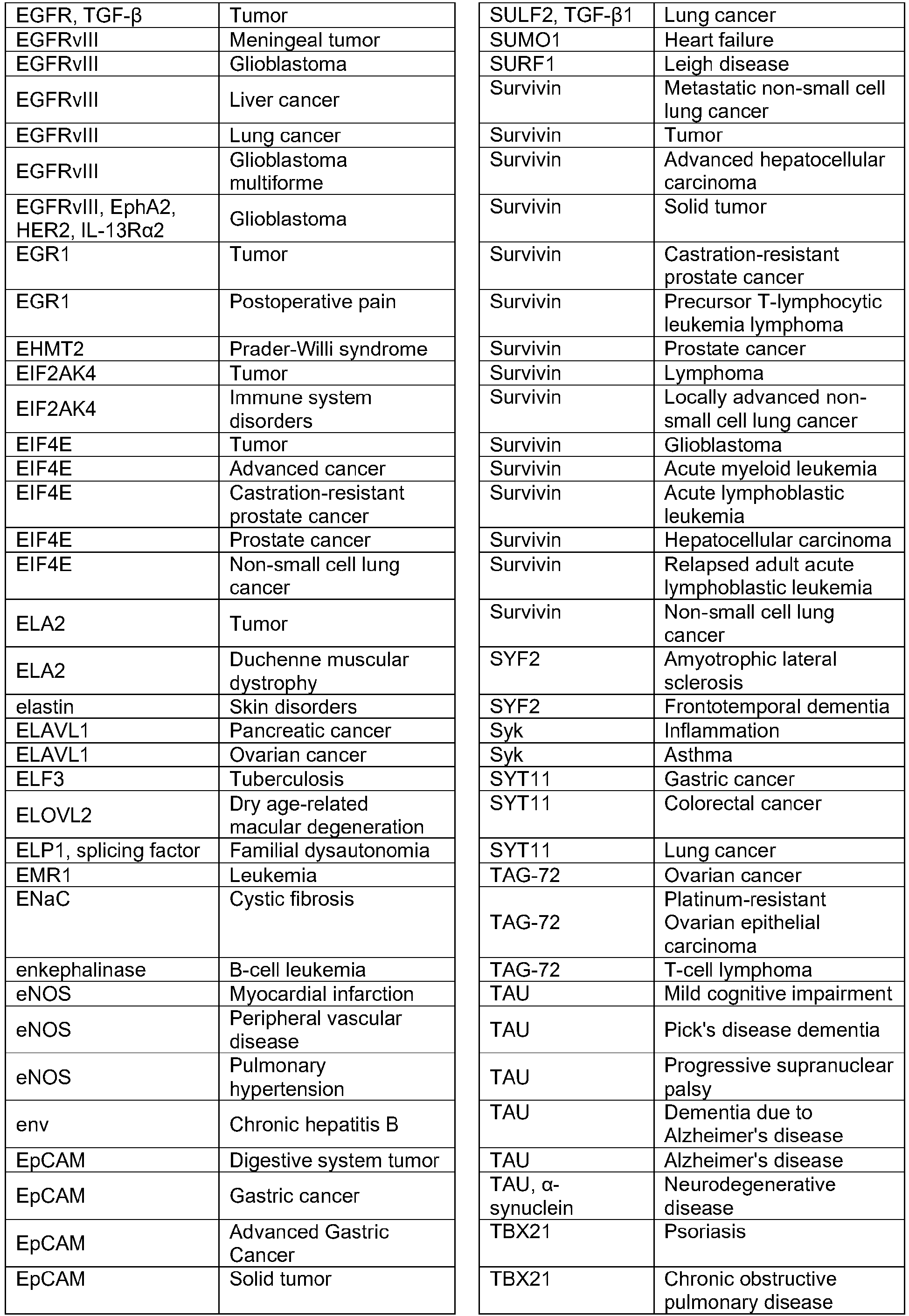

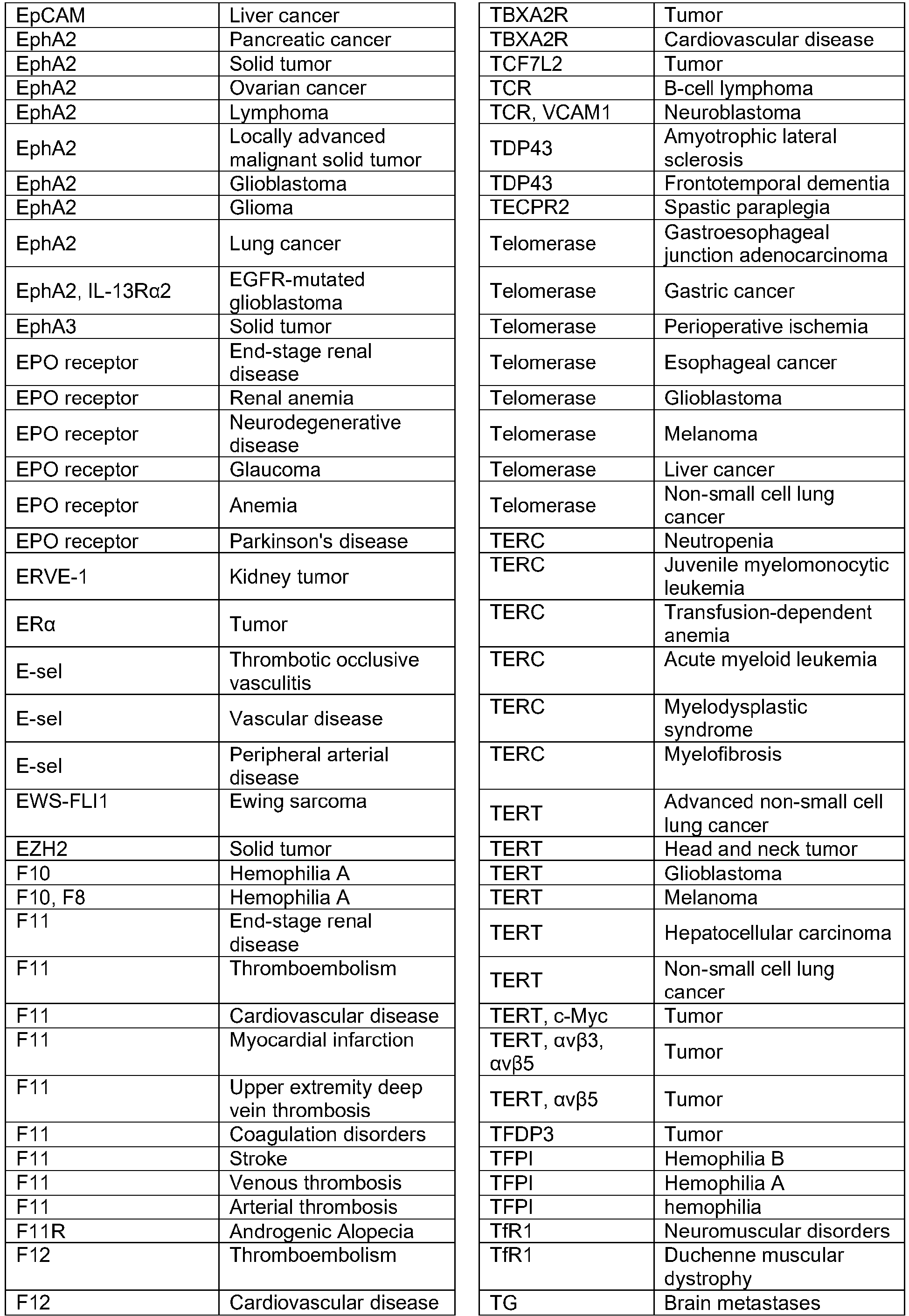

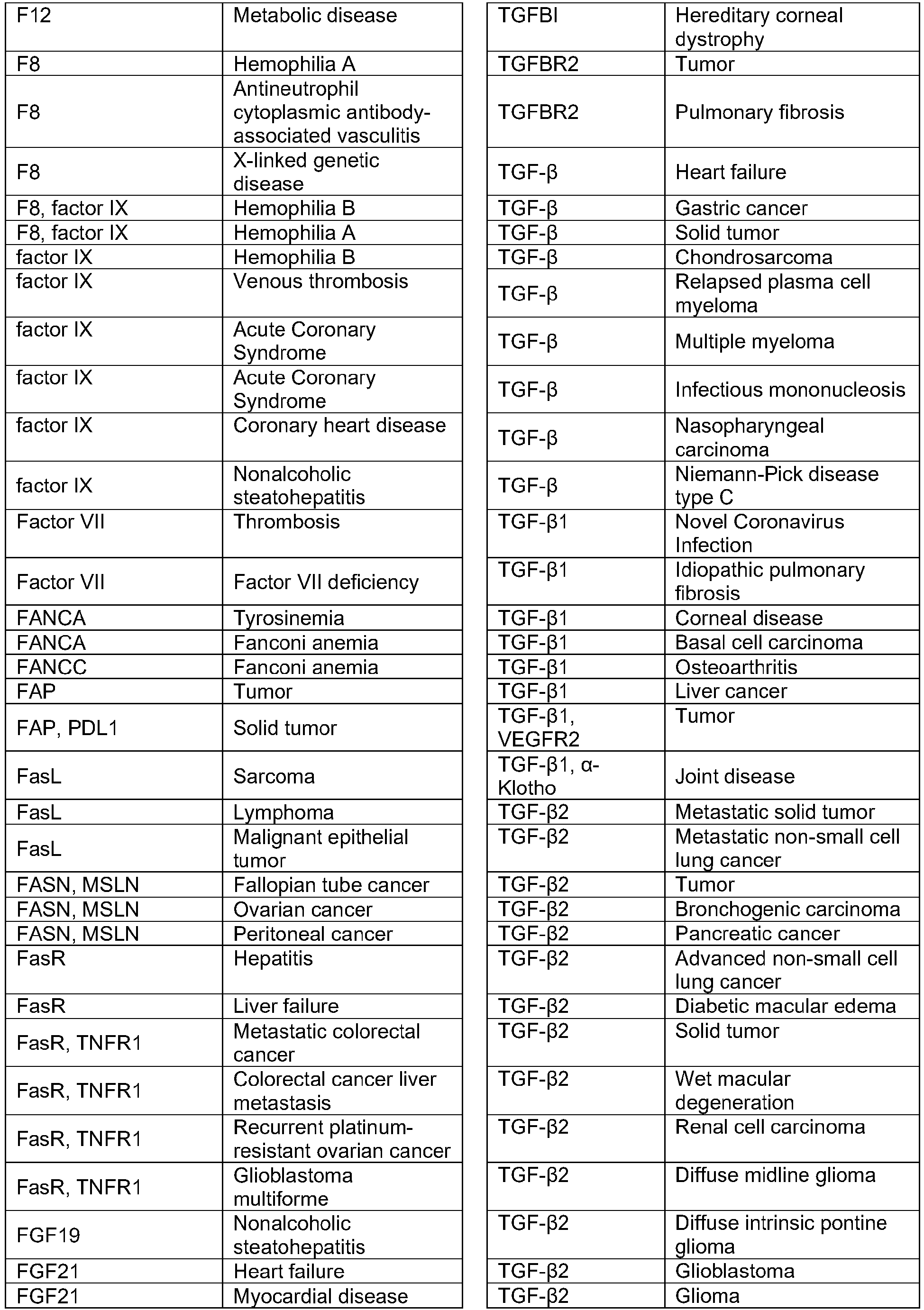

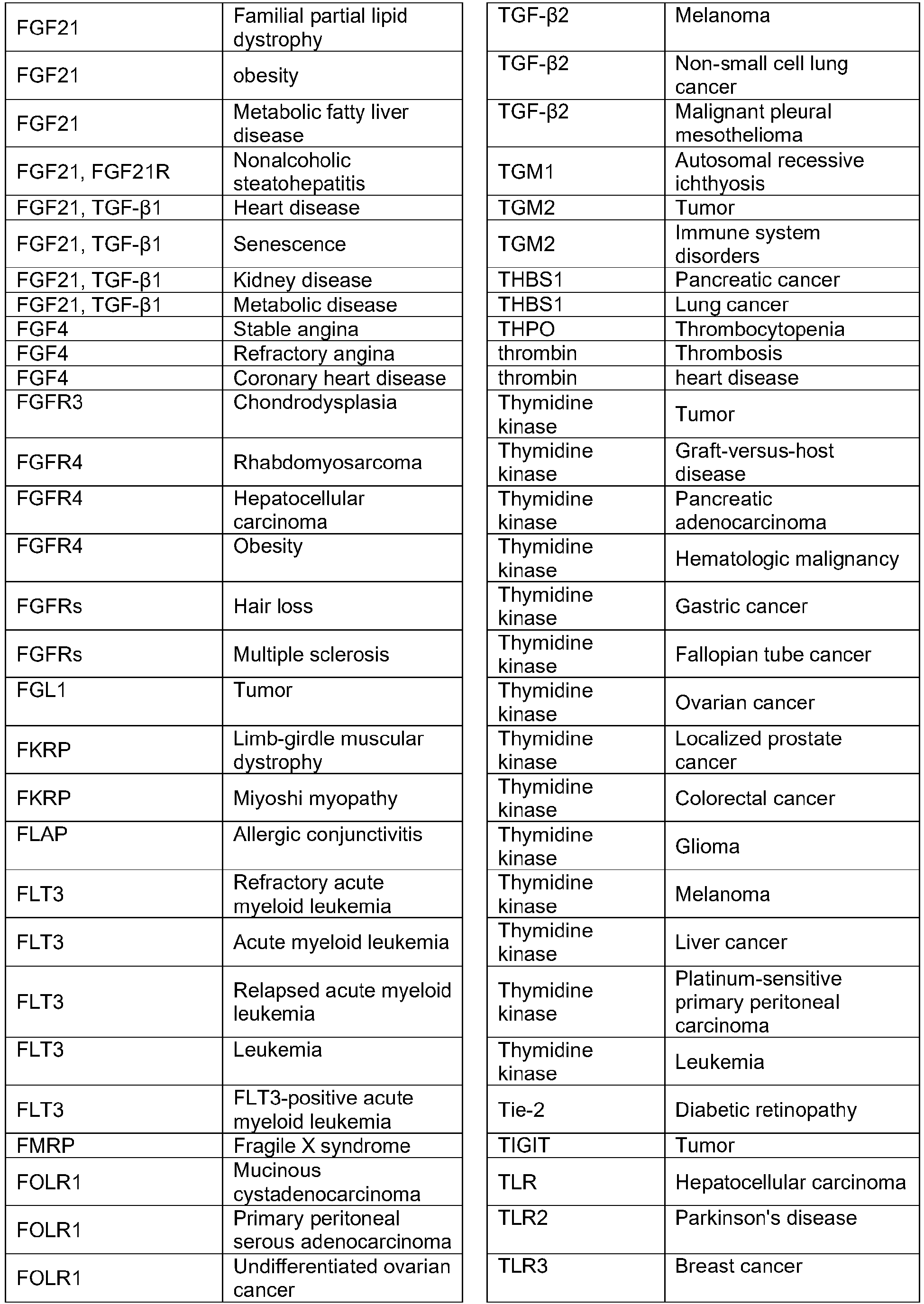

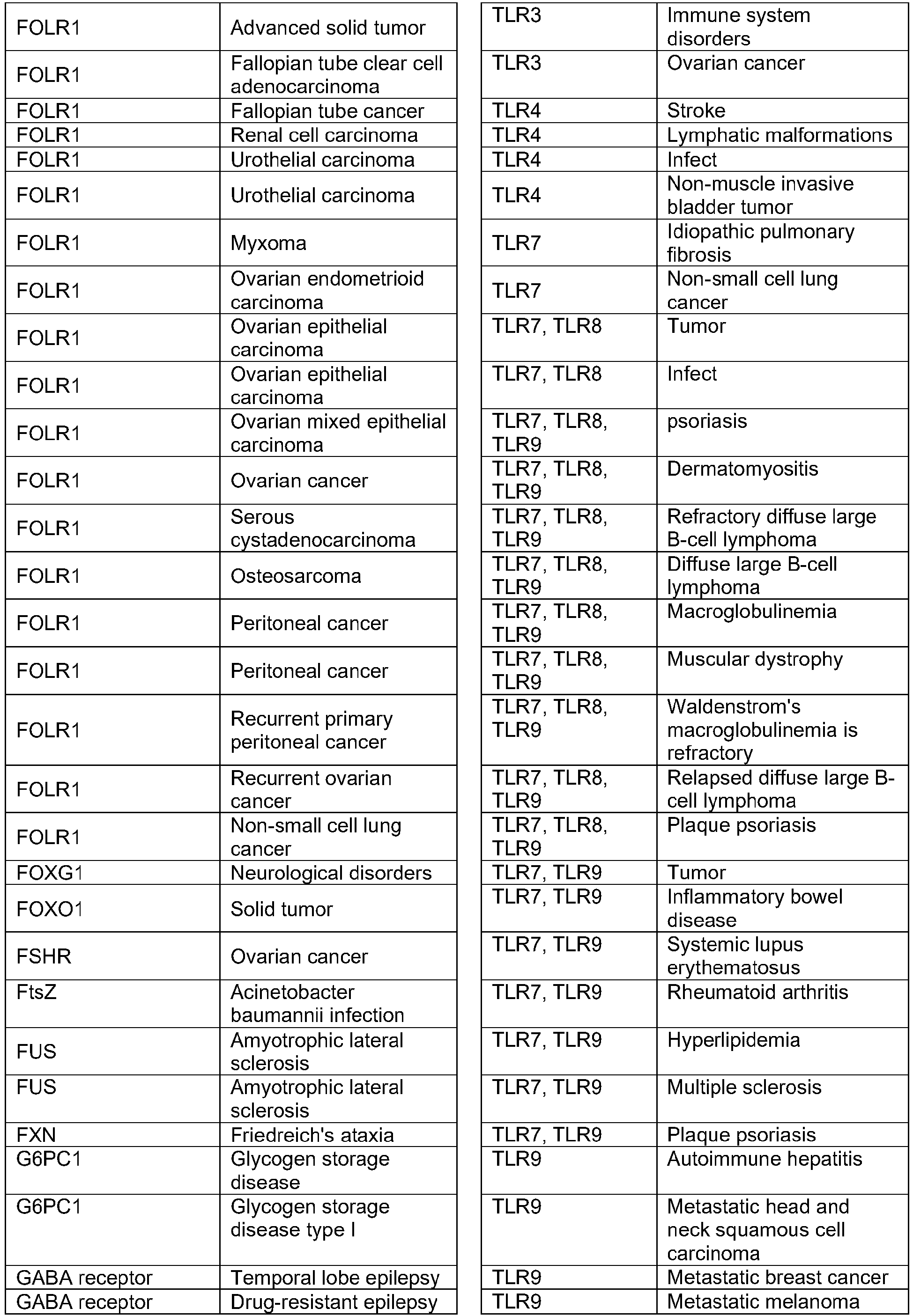

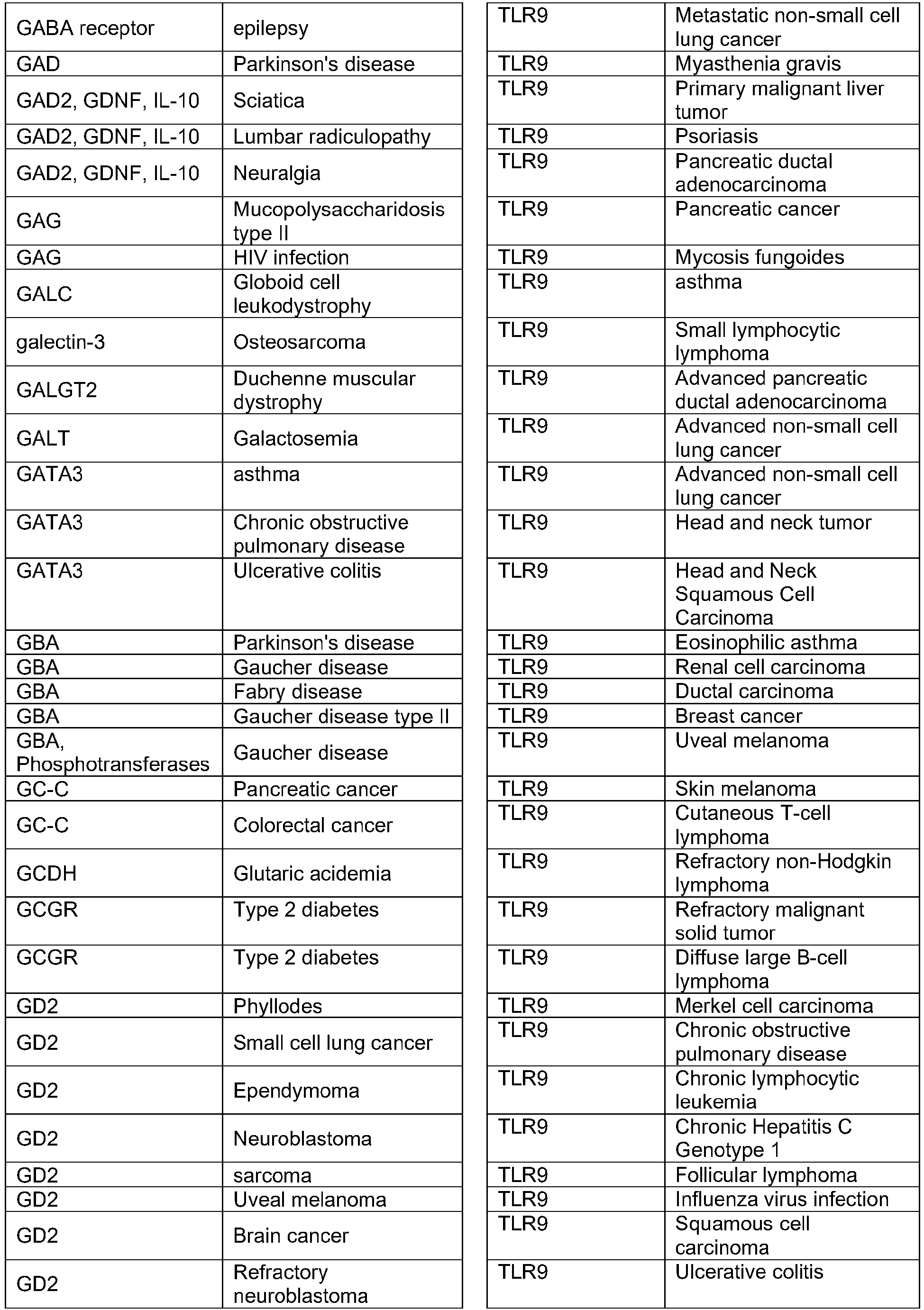

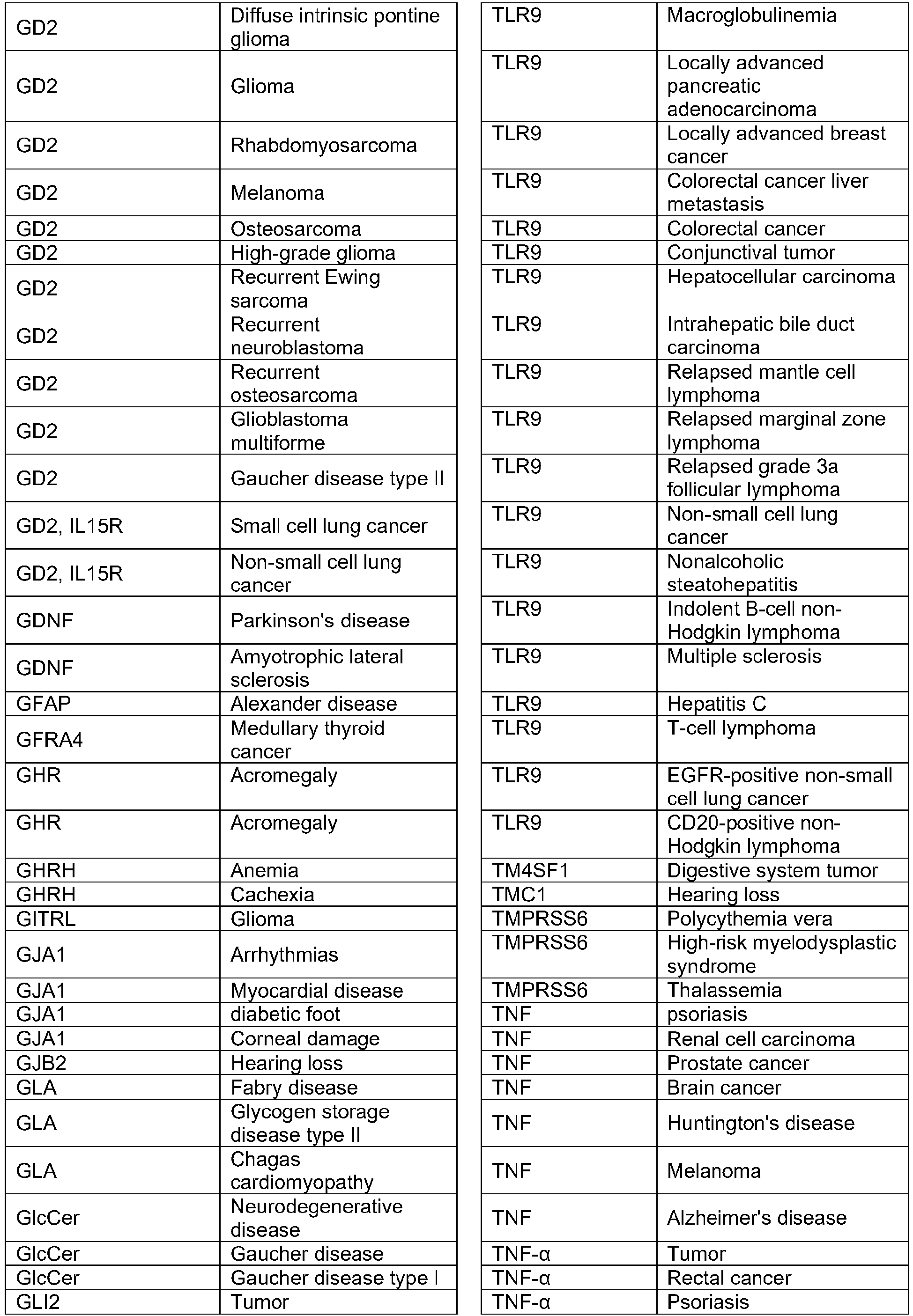

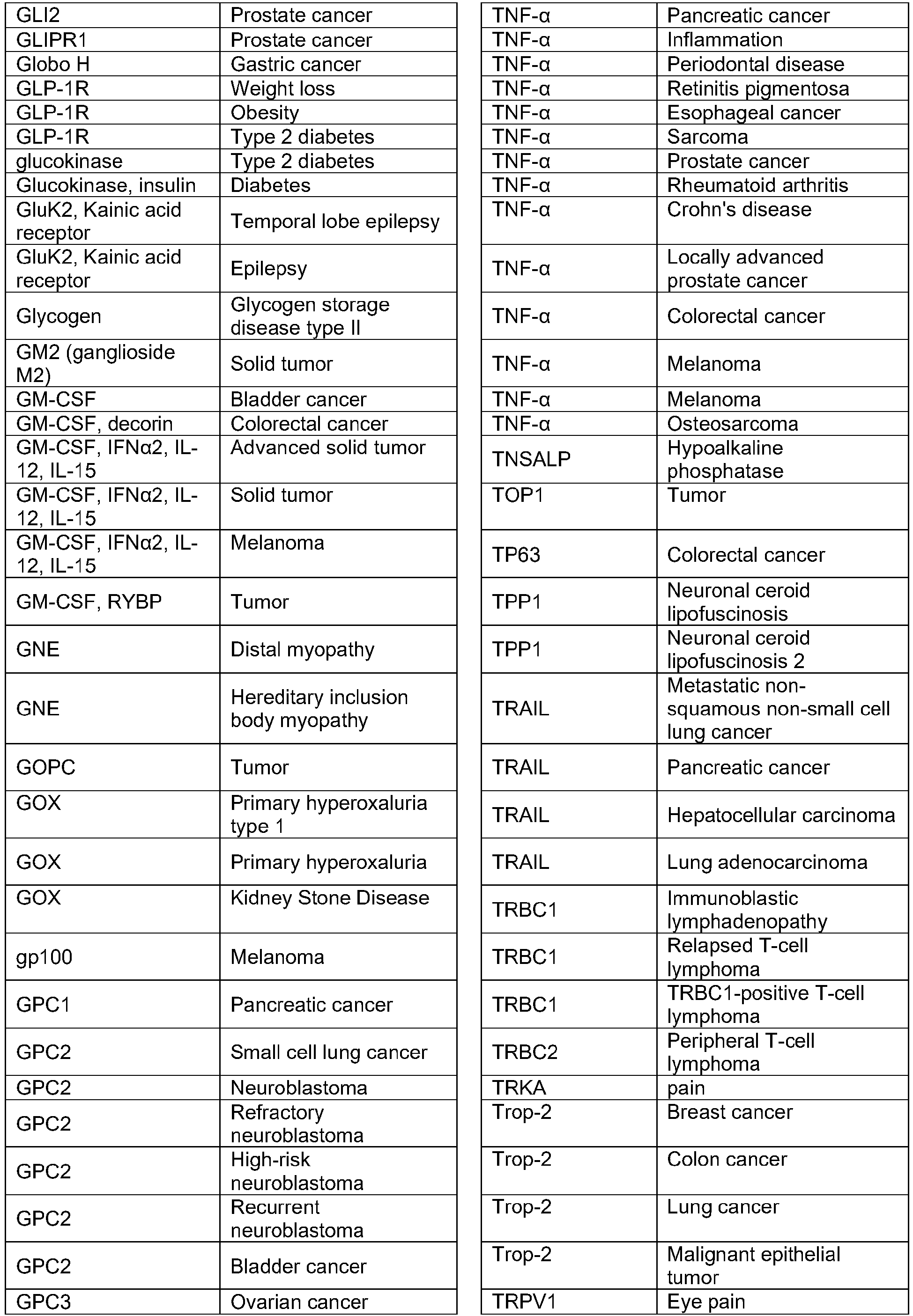

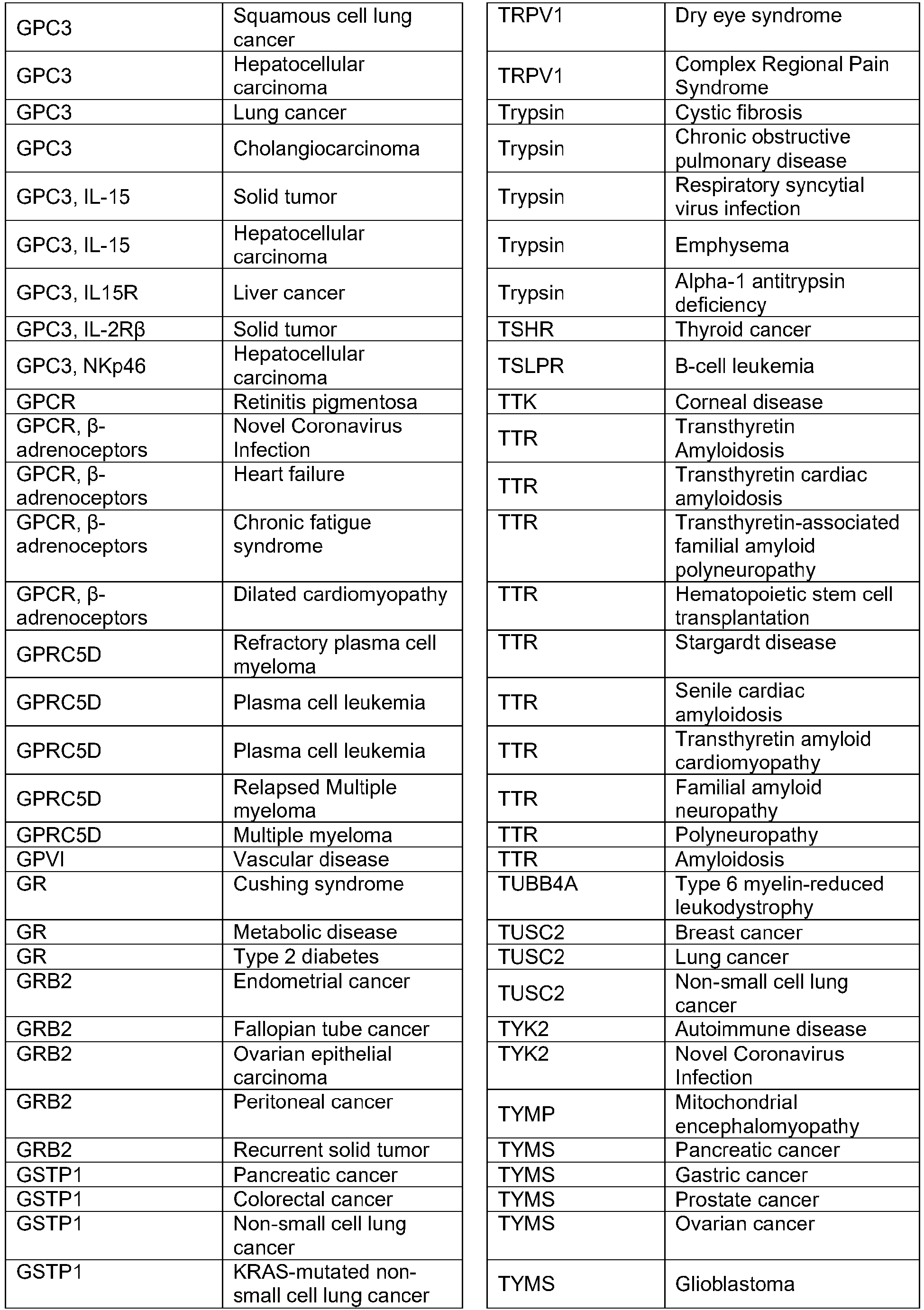

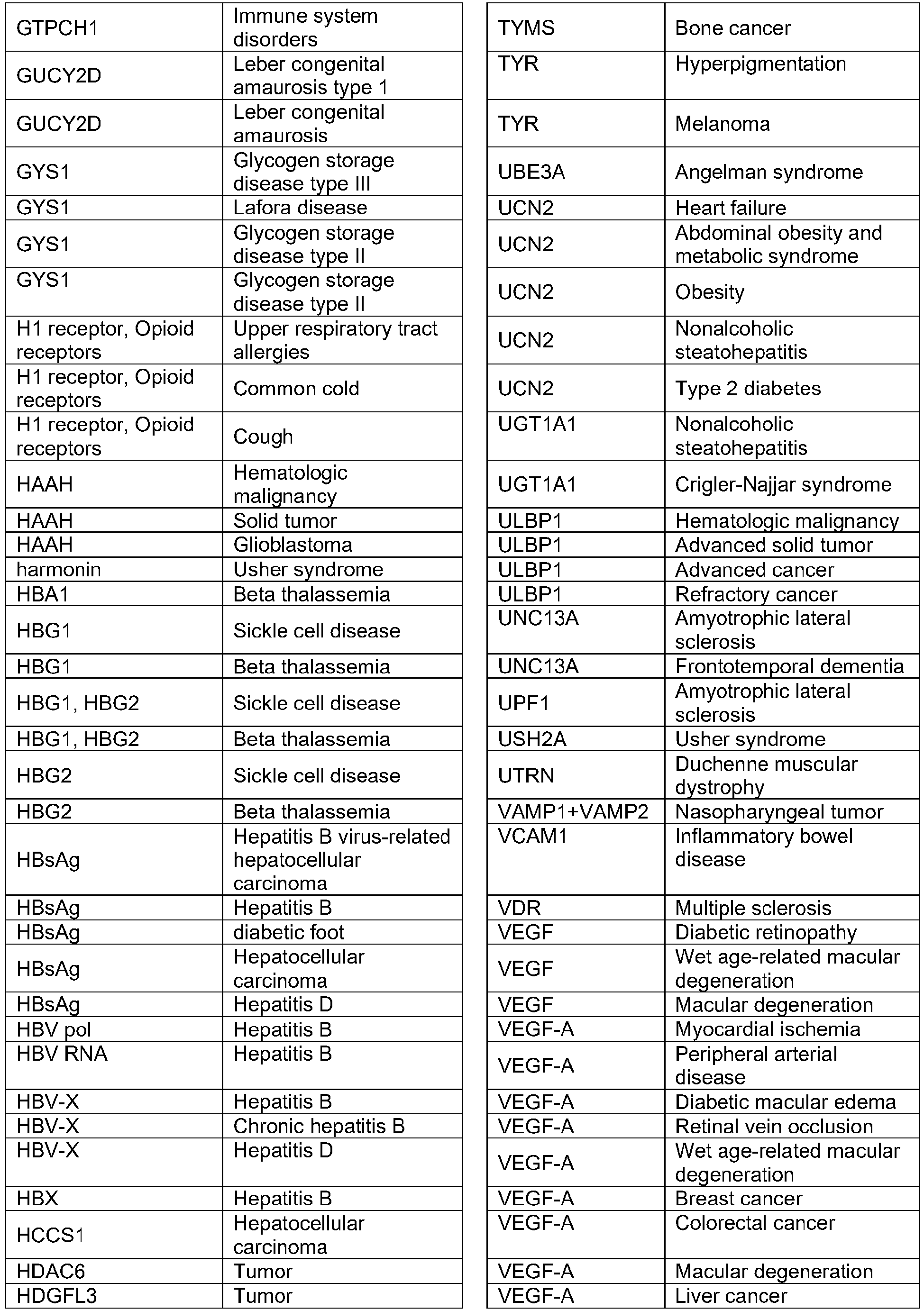

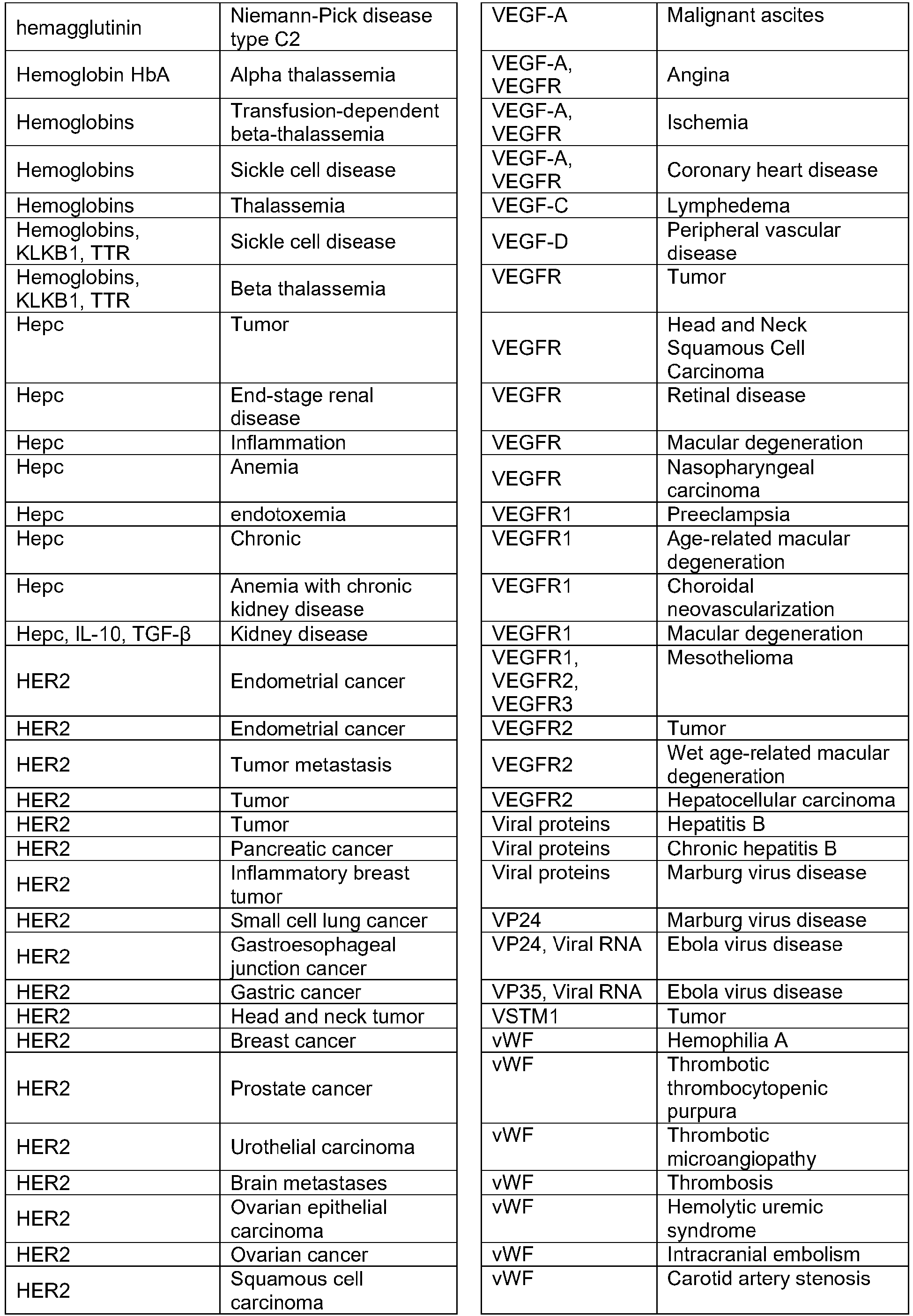

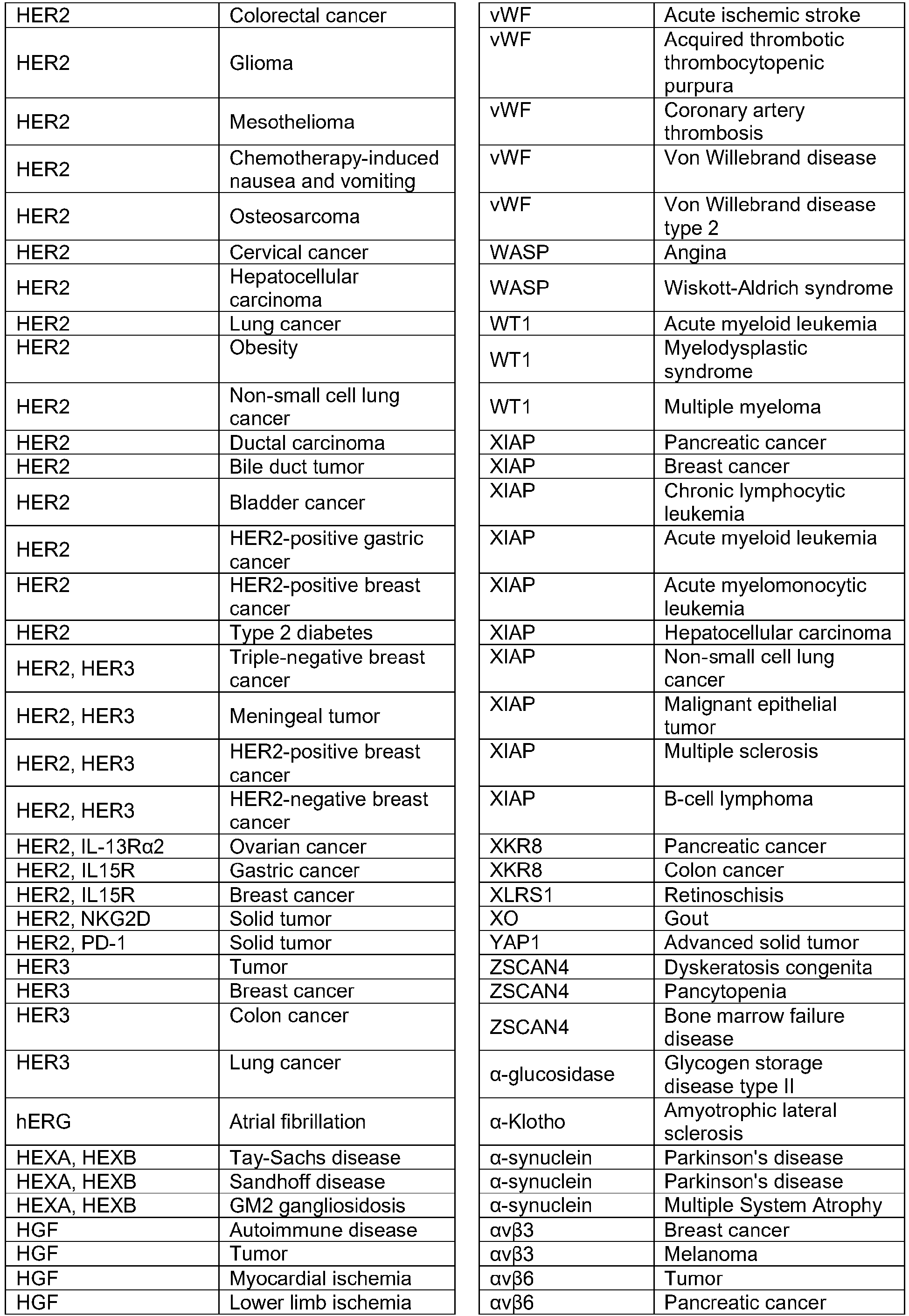

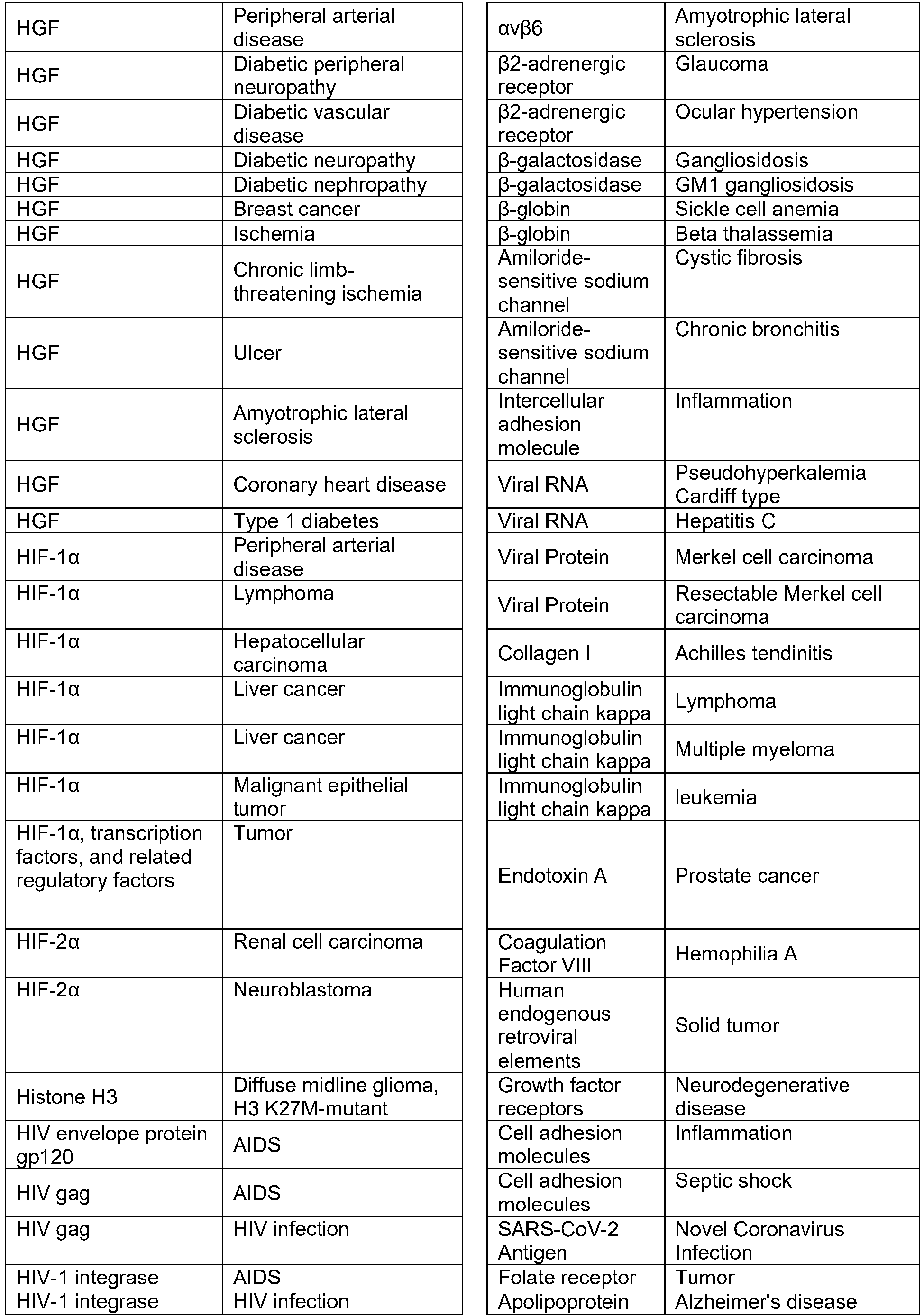

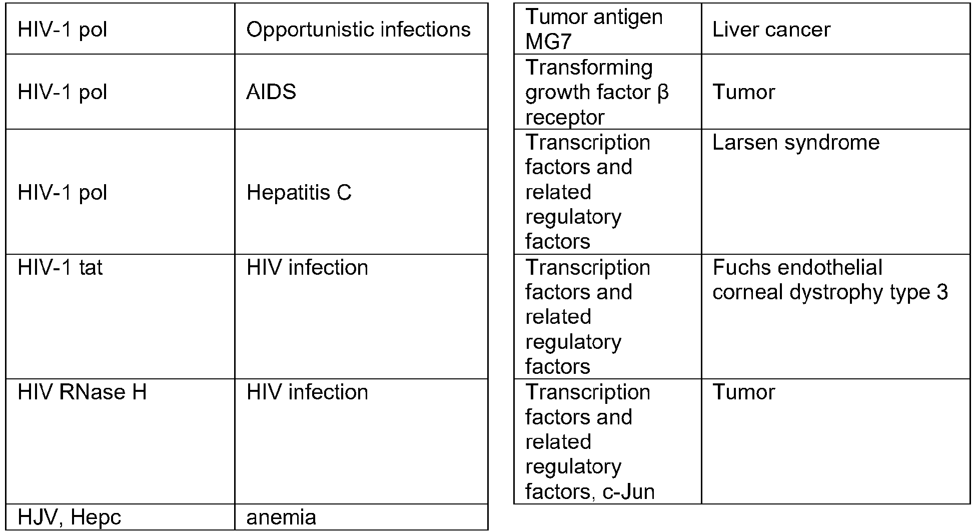

## Claims

1. A deaminase, wherein the deaminase comprises an amino acid sequence having at least 50% sequence identity to a sequence set forth in any one of SEQ ID NO: 1-6, 39-164, and 297-312;
optionally, the deaminase deaminates at least one nucleotide in a polynucleotide;
optionally, the deaminase deaminates a base in a nucleic acid sequence together with a nucleic acid-binding polypeptide;
optionally, the deaminase deaminates a base in a target polynucleotide together with a nucleic acid-binding polypeptide and a guide RNA (gRNA);
optionally, the deaminase comprises a homologous or heterologous domain; further, the deaminase comprises a nucleic acid-binding polypeptide, a cellular localization signal, and/or a UGI domain.

2. The deaminase according to claim 1, wherein the deaminase comprises an amino acid sequence set forth in any one of SEQ ID NO: 1-6, 39-164, and 297-312; optionally, the amino acid sequence of the deaminase is not any one of SEQ ID NO: 1-5 and 54-60;
optionally, the deaminase comprises an amino acid sequence having at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the sequence set forth in any one of SEQ ID NO: 3, 5, 6, 160, 163, 164, 297, 299, and 301;
optionally, the deaminase comprises a mutation at a position corresponding to amino acid residue 122, 191, 114, 2, 176, 68, and/or 89 of a reference sequence set forth in SEQ ID NO: 301.

3. A fusion protein, wherein the fusion protein comprises the deaminase according to claim 1 or 2.

4. The fusion protein according to claim 3, wherein the fusion protein comprises: a) a nucleic acid-binding polypeptide that binds to a target polynucleotide; and b) the deaminase according to claim 1 or 2;
optionally, the nucleic acid-binding polypeptide is a TALEN nuclease, a zinc finger nuclease (ZFN), a CRISPR-Cas nuclease, or a meganuclease;
optionally, the nucleic acid-binding polypeptide is an RNA-guided nuclease; optionally, the nucleic acid-binding polypeptide is a Cas enzyme (CRISPR-Cas nuclease);
optionally, the nucleic acid-binding polypeptide is selected from Cas9, Cas12, Cas13, TnpB, IscB, IsrB, a Fancor nuclease, or a fragment of any one of the Cas9, the Cas12, the Cas13, the TnpB, the IscB, the IsrB, and the Fancor nuclease, the fragment including but not limited to a nucleic acid binding domain fragment;
further optionally, the fusion protein further comprises one or more Uracil Glycosylase Inhibitors (UGIs); and/or the fusion protein comprises one or more Nuclear Localization Signals (NLSs);
further optionally, the nucleic acid-binding polypeptide, the deaminase, the one or more UGIs, and the one or more NLSs are linked directly or via linkers.

5. An isolated nucleic acid, wherein the nucleic acid encodes the deaminase according to claim 1 or 2 or the fusion protein according to claim 3 or 4.

6. An expression vector, wherein the expression vector comprises the nucleic acid according to claim 5.

7. A pharmaceutical composition, wherein the pharmaceutical composition comprises the deaminase according to claim 1 or 2, the fusion protein according to claim 3 or 4, the nucleic acid according to claim 5, or the expression vector according to claim 6, and a pharmaceutically acceptable delivery carrier.

8. An expression system, wherein the expression system comprises the expression vector according to claim 6, the pharmaceutical composition according to claim 7, or the nucleic acid according to claim 5 integrated in a genome.

9. A system for modifying a target polynucleotide, wherein the system comprises:
a) one or more guide RNAs (gRNAs) or one or more nucleic acids encoding the one or more gRNAs, the one or more gRNAs being capable of hybridizing with the target polynucleotide;
the system further comprises any one or more of the following (a)-(f):
b) the deaminase according to claim 1 or 2, or a nucleic acid encoding the deaminase; and a nucleic acid-binding polypeptide, or a nucleic acid encoding the nucleic acid-binding polypeptide;
c) the fusion protein according to claim 3 or 4, or a nucleic acid encoding the fusion protein;
d) the expression vector according to claim 6;
e) the pharmaceutical composition according to claim 7;
f) the expression system according to claim 8.

10. A method for modifying a target polynucleotide, wherein the method comprises contacting the target polynucleotide with the deaminase according to claim 1 or 2, the fusion protein according to claim 3 or 4, the nucleic acid according to claim 5, the expression vector according to claim 6, the pharmaceutical composition according to claim 7, the expression system according to claim 8, or the system for modifying the target polynucleotide according to claim 9.
